(19) Europäisches Patentamt / European Patent Office / Office européen des brevets

(11) **EP 3 943 505 A1**

(12) **EUROPEAN PATENT APPLICATION**

(43) Date of publication:
**26.01.2022 Bulletin 2022/04**

(21) Application number: **20305843.3**

(22) Date of filing: **22.07.2020**

(51) International Patent Classification (IPC):
**C07K 14/435** *(2006.01)*    **A61K 38/17** *(2006.01)*

(52) Cooperative Patent Classification (CPC):
**C07K 14/70503; A61K 38/08; A61P 3/10;**
**C07K 14/705;** A61K 38/00

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **ENCEFA**
**94100 Saint-Maur-des-Fossés (FR)**

(72) Inventors:
• **BRESSAC, Laurence**
  **94100 Saint-Maur-des-Fossés (FR)**
• **GUERREIRO DA SILVA, Serge**
  **94140 Alfortville (FR)**
• **TOULORGE, Damien**
  **75013 Paris (FR)**

(74) Representative: **Icosa**
**83 avenue Denfert-Rochereau**
**75014 Paris (FR)**

(54) **CD38-BINDING CD31 PEPTIDES AND USES THEREOF**

(57)    The present invention relates to peptides derived from CD31, specifically binding to CD38; and their use in the prevention and/or treatment of diseases.

FIG. 1

EP 3 943 505 A1

SEQUENCE LISTING

<110> ENCEFA
BRESSAC Laurence
GUERREIRO DA SILVA Serge
TOULORGE Damien

<120> CD38-BINDING CD31 PEPTIDES AND USES THEREOF

<130> CV - 1596/EP

<160> 18

<170> BiSSAP 1.3.6

<210> 1
<211> 738
<212> PRT
<213> Homo sapiens

<220>
<221> SIGNAL
<222> 1..26
<223> Signal peptide

<220>
<223> Human CD31

<220>
<221> DOMAIN
<222> 28..601
<223> Extracellular domain

<220>
<221> DOMAIN
<222> 35..121
<223> Ig-like 1

<220>
<221> DOMAIN
<222> 145..233
<223> Ig-like 2

<220>
<221> DOMAIN
<222> 236..315
<223> Ig-like 3

<220>
<221> DOMAIN
<222> 328..401
<223> Ig-like 4

<220>
<221> DOMAIN
<222> 424..493
<223> Ig-like 5

<220>
<221> DOMAIN
<222> 499..591
<223> Ig-like 6

```
<220>
<221> DOMAIN
<222> 602..620
<223> Transmembrane domain

<220>
<221> DOMAIN
<222> 621..738
<223> Cytoplasmic domain

<300>
<308> NCBI: NP_000433
<309> June 09, 2020
<313> 1-738

<400> 1
Met Gln Pro Arg Trp Ala Gln Gly Ala Thr Met Trp Leu Gly Val Leu
1               5                   10                  15
Leu Thr Leu Leu Leu Cys Ser Ser Leu Glu Gly Gln Glu Asn Ser Phe
                20                  25                  30
Thr Ile Asn Ser Val Asp Met Lys Ser Leu Pro Asp Trp Thr Val Gln
        35                  40                  45
Asn Gly Lys Asn Leu Thr Leu Gln Cys Phe Ala Asp Val Ser Thr Thr
    50                  55                  60
Ser His Val Lys Pro Gln His Gln Met Leu Phe Tyr Lys Asp Asp Val
65                  70                  75                  80
Leu Phe Tyr Asn Ile Ser Ser Met Lys Ser Thr Glu Ser Tyr Phe Ile
                85                  90                  95
Pro Glu Val Arg Ile Tyr Asp Ser Gly Thr Tyr Lys Cys Thr Val Ile
            100                 105                 110
Val Asn Asn Lys Glu Lys Thr Thr Ala Glu Tyr Gln Val Leu Val Glu
        115                 120                 125
Gly Val Pro Ser Pro Arg Val Thr Leu Asp Lys Lys Glu Ala Ile Gln
    130                 135                 140
Gly Gly Ile Val Arg Val Asn Cys Ser Val Pro Glu Glu Lys Ala Pro
145                 150                 155                 160
Ile His Phe Thr Ile Glu Lys Leu Glu Leu Asn Glu Lys Met Val Lys
                165                 170                 175
Leu Lys Arg Glu Lys Asn Ser Arg Asp Gln Asn Phe Val Ile Leu Glu
            180                 185                 190
Phe Pro Val Glu Glu Gln Asp Arg Val Leu Ser Phe Arg Cys Gln Ala
        195                 200                 205
Arg Ile Ile Ser Gly Ile His Met Gln Thr Ser Glu Ser Thr Lys Ser
    210                 215                 220
Glu Leu Val Thr Val Thr Glu Ser Phe Ser Thr Pro Lys Phe His Ile
225                 230                 235                 240
Ser Pro Thr Gly Met Ile Met Glu Gly Ala Gln Leu His Ile Lys Cys
                245                 250                 255
Thr Ile Gln Val Thr His Leu Ala Gln Glu Phe Pro Glu Ile Ile Ile
            260                 265                 270
Gln Lys Asp Lys Ala Ile Val Ala His Asn Arg His Gly Asn Lys Ala
        275                 280                 285
Val Tyr Ser Val Met Ala Met Val Glu His Ser Gly Asn Tyr Thr Cys
    290                 295                 300
Lys Val Glu Ser Ser Arg Ile Ser Lys Val Ser Ser Ile Val Val Asn
305                 310                 315                 320
Ile Thr Glu Leu Phe Ser Lys Pro Glu Leu Glu Ser Ser Phe Thr His
                325                 330                 335
Leu Asp Gln Gly Glu Arg Leu Asn Leu Ser Cys Ser Ile Pro Gly Ala
            340                 345                 350
Pro Pro Ala Asn Phe Thr Ile Gln Lys Glu Asp Thr Ile Val Ser Gln
        355                 360                 365
```

```
Thr Gln Asp Phe Thr Lys Ile Ala Ser Lys Ser Asp Ser Gly Thr Tyr
    370             375             380
Ile Cys Thr Ala Gly Ile Asp Lys Val Val Lys Lys Ser Asn Thr Val
385             390             395             400
Gln Ile Val Val Cys Glu Met Leu Ser Gln Pro Arg Ile Ser Tyr Asp
                405             410             415
Ala Gln Phe Glu Val Ile Lys Gly Gln Thr Ile Glu Val Arg Cys Glu
            420             425             430
Ser Ile Ser Gly Thr Leu Pro Ile Ser Tyr Gln Leu Leu Lys Thr Ser
            435             440             445
Lys Val Leu Glu Asn Ser Thr Lys Asn Ser Asn Asp Pro Ala Val Phe
    450             455             460
Lys Asp Asn Pro Thr Glu Asp Val Glu Tyr Gln Cys Val Ala Asp Asn
465             470             475             480
Cys His Ser His Ala Lys Met Leu Ser Glu Val Leu Arg Val Lys Val
                485             490             495
Ile Ala Pro Val Asp Glu Val Gln Ile Ser Ile Leu Ser Ser Lys Val
            500             505             510
Val Glu Ser Gly Glu Asp Ile Val Leu Gln Cys Ala Val Asn Glu Gly
            515             520             525
Ser Gly Pro Ile Thr Tyr Lys Phe Tyr Arg Glu Lys Glu Gly Lys Pro
    530             535             540
Phe Tyr Gln Met Thr Ser Asn Ala Thr Gln Ala Phe Trp Thr Lys Gln
545             550             555             560
Lys Ala Ser Lys Glu Gln Glu Gly Glu Tyr Tyr Cys Thr Ala Phe Asn
            565             570             575
Arg Ala Asn His Ala Ser Ser Val Pro Arg Ser Lys Ile Leu Thr Val
            580             585             590
Arg Val Ile Leu Ala Pro Trp Lys Lys Gly Leu Ile Ala Val Val Ile
    595             600             605
Ile Gly Val Ile Ile Ala Leu Leu Ile Ile Ala Ala Lys Cys Tyr Phe
    610             615             620
Leu Arg Lys Ala Lys Ala Lys Gln Met Pro Val Glu Met Ser Arg Pro
625             630             635             640
Ala Val Pro Leu Leu Asn Ser Asn Asn Glu Lys Met Ser Asp Pro Asn
            645             650             655
Met Glu Ala Asn Ser His Tyr Gly His Asn Asp Asp Val Arg Asn His
            660             665             670
Ala Met Lys Pro Ile Asn Asp Asn Lys Glu Pro Leu Asn Ser Asp Val
    675             680             685
Gln Tyr Thr Glu Val Gln Val Ser Ser Ala Glu Ser His Lys Asp Leu
    690             695             700
Gly Lys Lys Asp Thr Glu Thr Val Tyr Ser Glu Val Arg Lys Ala Val
705             710             715             720
Pro Asp Ala Val Glu Ser Arg Tyr Ser Arg Thr Glu Gly Ser Leu Asp
            725             730             735
Gly Thr
```

```
<210> 2
<211> 459
<212> PRT
<213> Artificial Sequence


<220>
<223> Human sCD31

<400> 2
Asn Ser Val Asp Met Lys Ser Leu Pro Asp Trp Thr Val Gln Asn Gly
1               5               10              15
Lys Asn Leu Thr Leu Gln Cys Phe Ala Asp Val Ser Thr Thr Ser His
            20              25              30
```

```
Val Lys Pro Gln His Gln Met Leu Phe Tyr Lys Asp Asp Val Leu Phe
        35                      40                  45
Tyr Asn Ile Ser Ser Met Lys Ser Thr Glu Ser Tyr Phe Ile Pro Glu
    50                      55                  60
Val Arg Ile Tyr Asp Ser Gly Thr Tyr Lys Cys Thr Val Ile Val Asn
65                  70                  75                      80
Asn Lys Glu Lys Thr Ala Glu Tyr Gln Val Leu Val Glu Gly Val
                85                  90                      95
Pro Ser Pro Arg Val Thr Leu Asp Lys Lys Glu Ala Ile Gln Gly Gly
            100                 105                 110
Ile Val Arg Val Asn Cys Ser Val Pro Glu Glu Lys Ala Pro Ile His
        115                 120                 125
Phe Thr Ile Glu Lys Leu Glu Leu Asn Glu Lys Met Val Lys Leu Lys
    130                 135                 140
Arg Glu Lys Asn Ser Arg Asp Gln Asn Phe Val Ile Leu Glu Phe Pro
145                 150                 155                 160
Val Glu Glu Gln Asp Arg Val Leu Ser Phe Arg Cys Gln Ala Arg Ile
                165                 170                 175
Ile Ser Gly Ile His Met Gln Thr Ser Glu Ser Thr Lys Ser Glu Leu
            180                 185                 190
Val Thr Val Thr Glu Ser Phe Ser Thr Pro Lys Phe His Ile Ser Pro
        195                 200                 205
Thr Gly Met Ile Met Glu Gly Ala Gln Leu His Ile Lys Cys Thr Ile
    210                 215                 220
Gln Val Thr His Leu Ala Gln Glu Phe Pro Glu Ile Ile Ile Gln Lys
225                 230                 235                 240
Asp Lys Ala Ile Val Ala His Asn Arg His Gly Asn Lys Ala Val Tyr
                245                 250                 255
Ser Val Met Ala Met Val Glu His Ser Gly Asn Tyr Thr Cys Lys Val
            260                 265                 270
Glu Ser Ser Arg Ile Ser Lys Val Ser Ser Ile Val Val Asn Ile Thr
        275                 280                 285
Glu Leu Phe Ser Lys Pro Glu Leu Glu Ser Ser Phe Thr His Leu Asp
    290                 295                 300
Gln Gly Glu Arg Leu Asn Leu Ser Cys Ser Ile Pro Gly Ala Pro Pro
305                 310                 315                 320
Ala Asn Phe Thr Ile Gln Lys Glu Asp Thr Ile Val Ser Gln Thr Gln
                325                 330                 335
Asp Phe Thr Lys Ile Ala Ser Lys Ser Asp Ser Gly Thr Tyr Ile Cys
            340                 345                 350
Thr Ala Gly Ile Asp Lys Val Val Lys Lys Ser Asn Thr Val Gln Ile
        355                 360                 365
Val Val Cys Glu Met Leu Ser Gln Pro Arg Ile Ser Tyr Asp Ala Gln
    370                 375                 380
Phe Glu Val Ile Lys Gly Gln Thr Ile Glu Val Arg Cys Glu Ser Ile
385                 390                 395                 400
Ser Gly Thr Leu Pro Ile Ser Tyr Gln Leu Leu Lys Thr Ser Lys Val
            405                 410                 415
Leu Glu Asn Ser Thr Lys Asn Ser Asn Asp Pro Ala Val Phe Lys Asp
        420                 425                 430
Asn Pro Thr Glu Asp Val Glu Tyr Gln Cys Val Ala Asp Asn Cys His
    435                 440                 445
Ser His Ala Lys Met Leu Ser Glu Val Leu Arg
    450                 455
```

<210> 3
<211> 300
<212> PRT
<213> Homo sapiens


<220>
<223> Human CD38

```
<300>
<308> NCBI: NP_001766
<309> May 24, 2020

<400> 3
Met Ala Asn Cys Glu Phe Ser Pro Val Ser Gly Asp Lys Pro Cys Cys
1               5                   10                  15
Arg Leu Ser Arg Arg Ala Gln Leu Cys Leu Gly Val Ser Ile Leu Val
            20                  25                  30
Leu Ile Leu Val Val Val Leu Ala Val Val Val Pro Arg Trp Arg Gln
        35                  40                  45
Gln Trp Ser Gly Pro Gly Thr Thr Lys Arg Phe Pro Glu Thr Val Leu
    50                  55                  60
Ala Arg Cys Val Lys Tyr Thr Glu Ile His Pro Glu Met Arg His Val
65                  70                  75                  80
Asp Cys Gln Ser Val Trp Asp Ala Phe Lys Gly Ala Phe Ile Ser Lys
            85                  90                  95
His Pro Cys Asn Ile Thr Glu Glu Asp Tyr Gln Pro Leu Met Lys Leu
            100                 105                 110
Gly Thr Gln Thr Val Pro Cys Asn Lys Ile Leu Leu Trp Ser Arg Ile
        115                 120                 125
Lys Asp Leu Ala His Gln Phe Thr Gln Val Gln Arg Asp Met Phe Thr
    130                 135                 140
Leu Glu Asp Thr Leu Leu Gly Tyr Leu Ala Asp Asp Leu Thr Trp Cys
145                 150                 155                 160
Gly Glu Phe Asn Thr Ser Lys Ile Asn Tyr Gln Ser Cys Pro Asp Trp
            165                 170                 175
Arg Lys Asp Cys Ser Asn Asn Pro Val Ser Val Phe Trp Lys Thr Val
            180                 185                 190
Ser Arg Arg Phe Ala Glu Ala Ala Cys Asp Val Val His Val Met Leu
    195                 200                 205
Asn Gly Ser Arg Ser Lys Ile Phe Asp Lys Asn Ser Thr Phe Gly Ser
    210                 215                 220
Val Glu Val His Asn Leu Gln Pro Glu Lys Val Gln Thr Leu Glu Ala
225                 230                 235                 240
Trp Val Ile His Gly Gly Arg Glu Asp Ser Arg Asp Leu Cys Gln Asp
            245                 250                 255
Pro Thr Ile Lys Glu Leu Glu Ser Ile Ile Ser Lys Arg Asn Ile Gln
            260                 265                 270
Phe Ser Cys Lys Asn Ile Tyr Arg Pro Asp Lys Phe Leu Gln Cys Val
    275                 280                 285
Lys Asn Pro Glu Asp Ser Ser Cys Thr Ser Glu Ile
    290                 295                 300

<210> 4
<211> 7
<212> PRT
<213> Artificial Sequence

<220>
<223> Human sCD31 peptide

<400> 4
Ser Thr Glu Ser Tyr Phe Ile
1               5

<210> 5
<211> 9
<212> PRT
<213> Artificial Sequence
```

<220>
<223> Human sCD31 peptide

<400> 5
Cys Ser Thr Glu Ser Tyr Phe Ile Cys
1               5

<210> 6
<211> 15
<212> PRT
<213> Artificial Sequence

<220>
<223> Human sCD31 peptide

<400> 6
Asn Ser Arg Asp Gln Asn Phe Val Ile Leu Glu Phe Pro Val Glu
1               5                   10                  15

<210> 7
<211> 7
<212> PRT
<213> Artificial Sequence

<220>
<223> Human sCD31 peptide

<400> 7
Asn Phe Val Ile Leu Glu Phe
1               5

<210> 8
<211> 9
<212> PRT
<213> Artificial Sequence

<220>
<223> Human sCD31 peptide

<400> 8
Cys Asn Phe Val Ile Leu Glu Phe Cys
1               5

<210> 9
<211> 17
<212> PRT
<213> Artificial Sequence

<220>
<223> Human sCD31 peptide

<400> 9
Cys Arg Asp Gln Asn Phe Val Ile Leu Glu Phe Pro Val Glu Glu Gln
1               5                   10                  15
Cys

<210> 10

<211> 22
<212> PRT
<213> Artificial Sequence

<220>
<223> Human sCD31 peptide

<400> 10
Cys Met Val Lys Leu Lys Arg Glu Lys Asn Pro Gly Asp Gln Asn Phe
1               5                   10                  15
Val Ile Leu Glu Phe Cys
            20

<210> 11
<211> 9
<212> PRT
<213> Artificial Sequence

<220>
<223> Human sCD31 peptide

<400> 11
Cys Val Glu Glu Gln Asp Arg Val Cys
1               5

<210> 12
<211> 11
<212> PRT
<213> Artificial Sequence

<220>
<223> Human sCD31 peptide

<400> 12
Cys Ile Val Val Asn Ile Thr Glu Leu Phe Cys
1               5                   10

<210> 13
<211> 22
<212> PRT
<213> Artificial Sequence

<220>
<223> Human sCD31 peptide

<400> 13
Cys Val Glu Ser Ser Arg Ile Ser Lys Val Pro Gly Ile Val Val Asn
1               5                   10                  15
Ile Thr Glu Leu Phe Cys
            20

<210> 14
<211> 22
<212> PRT
<213> Artificial Sequence

<220>
<223> Human sCD31 peptide

56

```
<400> 14
Cys Pro Pro Ala Asn Phe Thr Ile Gln Lys Pro Gly Thr Ile Val Ser
1               5                   10                  15
Gln Thr Gln Asp Phe Cys
                20


<210> 15
<211> 8
<212> PRT
<213> Artificial Sequence


<220>
<223> Human sCD31 peptide


<400> 15
Cys Glu Asp Val Glu Tyr Gln Cys
1               5


<210> 16
<211> 22
<212> PRT
<213> Artificial Sequence


<220>
<223> Human CD31 peptide


<400> 16
Cys Ser Glu Val Leu Arg Val Lys Val Ile Pro Gly Val Asp Glu Val
1               5                   10                  15
Gln Ile Ser Ile Leu Cys
                20


<210> 17
<211> 8
<212> PRT
<213> Artificial Sequence


<220>
<223> Human CD31 peptide


<400> 17
Arg Val Ile Leu Ala Pro Trp Lys
1               5


<210> 18
<211> 585
<212> PRT
<213> Mus musculus


<220>
<223> Murine sCD31


<400> 18
Met Leu Leu Ala Leu Gly Leu Thr Leu Val Leu Tyr Ala Ser Leu Gln
1               5                   10                  15
Ala Glu Glu Asn Ser Phe Thr Ile Asn Ser Ile His Met Glu Ser Leu
                20                  25                  30
Pro Ser Trp Glu Val Met Asn Gly Gln Gln Leu Thr Leu Glu Cys Leu
```

```
                    35                      40                      45
        Val Asp Ile Ser Thr Thr Ser Lys Ser Arg Ser Gln His Arg Val Leu
            50                      55                      60
        Phe Tyr Lys Asp Asp Ala Met Val Tyr Asn Val Thr Ser Arg Glu His
            65                      70                      75                      80
        Thr Glu Ser Tyr Val Ile Pro Gln Ala Arg Val Phe His Ser Gly Lys
                        85                      90                      95
        Tyr Lys Cys Thr Val Met Leu Asn Asn Lys Glu Lys Thr Thr Ile Glu
                    100                     105                     110
        Tyr Glu Val Lys Val His Gly Val Ser Lys Pro Lys Val Thr Leu Asp
                    115                     120                     125
        Lys Lys Glu Val Thr Glu Gly Gly Val Val Thr Val Asn Cys Ser Leu
            130                     135                     140
        Gln Glu Glu Lys Pro Pro Ile Phe Phe Lys Ile Glu Lys Leu Glu Val
        145                     150                     155                     160
        Gly Thr Lys Phe Val Lys Arg Arg Ile Asp Lys Thr Ser Asn Glu Asn
                    165                     170                     175
        Phe Val Leu Met Glu Phe Pro Ile Glu Ala Gln Asp His Val Leu Val
                    180                     185                     190
        Phe Arg Cys Gln Ala Gly Ile Leu Ser Gly Phe Lys Leu Gln Glu Ser
                    195                     200                     205
        Glu Pro Ile Arg Ser Glu Tyr Val Thr Val Gln Glu Ser Phe Ser Thr
            210                     215                     220
        Pro Lys Phe Glu Ile Lys Pro Pro Gly Met Ile Ile Glu Gly Asp Gln
        225                     230                     235                     240
        Leu His Ile Arg Cys Ile Val Gln Val Thr His Leu Val Gln Glu Phe
                    245                     250                     255
        Thr Glu Ile Ile Ile Gln Lys Asp Lys Ala Ile Val Ala Thr Ser Lys
                    260                     265                     270
        Gln Ser Ser Glu Ala Val Tyr Ser Val Met Ala Met Val Glu Tyr Ser
            275                     280                     285
        Gly His Tyr Thr Cys Lys Val Glu Ser Asn Arg Ile Ser Lys Ala Ser
            290                     295                     300
        Ser Ile Met Val Asn Ile Thr Glu Leu Phe Pro Lys Pro Lys Leu Glu
        305                     310                     315                     320
        Phe Ser Ser Ser Arg Leu Asp Gln Gly Glu Leu Leu Asp Leu Ser Cys
                    325                     330                     335
        Ser Val Ser Gly Thr Pro Val Ala Asn Phe Thr Ile Gln Lys Glu Glu
                    340                     345                     350
        Thr Val Leu Ser Gln Tyr Gln Asn Phe Ser Lys Ile Ala Glu Glu Ser
            355                     360                     365
        Asp Ser Gly Glu Tyr Ser Cys Thr Ala Gly Ile Gly Lys Val Val Lys
            370                     375                     380
        Arg Ser Gly Leu Val Pro Ile Gln Val Cys Glu Met Leu Ser Lys Pro
        385                     390                     395                     400
        Ser Ile Phe His Asp Ala Lys Ser Glu Ile Ile Lys Gly His Ala Ile
                    405                     410                     415
        Gly Ile Ser Cys Gln Ser Glu Asn Gly Thr Ala Pro Ile Thr Tyr His
                    420                     425                     430
        Leu Met Lys Ala Lys Ser Asp Phe Gln Thr Leu Glu Val Thr Ser Asn
            435                     440                     445
        Asp Pro Ala Thr Phe Thr Asp Lys Pro Thr Arg Asp Met Glu Tyr Gln
            450                     455                     460
        Cys Arg Ala Asp Asn Cys His Ser His Pro Ala Val Phe Ser Glu Ile
        465                     470                     475                     480
        Leu Arg Val Arg Val Ile Ala Pro Val Asp Glu Val Val Ile Ser Ile
                    485                     490                     495
        Leu Ser Ser Asn Glu Val Gln Ser Gly Ser Glu Met Val Leu Arg Cys
                    500                     505                     510
        Ser Val Lys Glu Gly Thr Ser Pro Ile Thr Phe Gln Phe Tyr Lys Glu
            515                     520                     525
        Lys Glu Asp Arg Pro Phe His Gln Ala Val Val Asn Asp Thr Gln Ala
            530                     535                     540
```

58

```
Phe Trp His Asn Lys Gln Ala Ser Lys Lys Gln Glu Gly Gln Tyr Tyr
545             550             555             560
Cys Thr Ala Ser Asn Arg Ala Ser Ser Met Arg Thr Ser Pro Arg Ser
                565             570             575
Ser Thr Leu Ala Val Arg Val Phe Leu
            580             585
```

**Description**

**FIELD OF INVENTION**

The present invention relates to peptides derived from CD31, specifically binding to CD38; and their use in the prevention and/or treatment of diseases.

**BACKGROUND OF INVENTION**

CD38 is a 45 kDa transmembrane protein that is ubiquitously expressed (Malavasi et al., 2008. Physiol Rev. 88(3):841-86). CD38 is mostly known for its enzymatic function that in part consists in degrading Nicotinamide Adenine Dinucleotide (NAD; Chini, 2009. Curr Pharm Des. 15(1):57-63), a metabolite involved in more than 400 redox reactions (Verdin, 2015. Science. 350(6265):1208-13). However, CD38 also possesses a receptor function whose physiological role remains elusive, that was serendipitously discovered in 1990 (Funaro et al., 1990. J Immunol. 145(8):2390-6) thanks to the use of antibodies that were later qualified as "agonistic". The endogenous ligand activating CD38 receptor function, CD31, was only identified in 1998 (Deaglio et al., 1998. J Immunol. 160(1):395-402).

CD31, also known as Platelet endothelial cell adhesion molecule-1 (PECAM-1), is a 130 kDa type I transmembrane glycoprotein that consists of six extracellular immunoglobulins (Ig)-like homology domains, a 19-residue transmembrane domain, and a 118-residue cytoplasmic tail (Newman and Newman, 2003. Arterioscler Thromb Vasc Biol. 23:953-964). As such, CD31 belongs to the Ig superfamily of cell adhesion molecules. CD31 is able to bind to itself through homophilic interaction involving Ig-like domain 1; as well to two other ligands, $\alpha V\beta 3$ integrin and CD38 (Newman, 1997. J Clin Invest. 99(1):3-8; Kalinowska & Losy, 2006. Eur J Neurol. 13(12):1284-90).

CD31 expression is mainly observed in endothelial cells, where it is considered as a constitutive marker (Kalinowska & Losy, 2006. Eur J Neurol. 13(12):1284-90), but also in most non-erythroid cells of the hematopoietic lineage including platelets, monocytes, neutrophils, T and B cell subsets (Wang et al., 2003. Am J Physiol Heart Circ Physiol. 284(3):H1008-17). While CD31 homophilic interaction is a major constituent of the endothelial cell intercellular junction, it is also involved in the process of leukocyte-endothelial transmigration (diapedesis), allowing the penetration of leucocytes into tissue during inflammation (Ilan & Madri, 2003. Curr Opin Cell Biol. 15(5):515-24).

CD31 is found under both a membrane form and a soluble form, following metalloproteinase-dependent cleavage (Ilan et al., 2001. FASEB J. 15(2):362-72). Increased circulating levels of soluble CD31 (sCD31) were observed in some inflammatory diseases, including atherosclerosis and sepsis (Feng et al., 2016. Eur Rev Med Pharmacol Sci. 20(19):4082-4088; Kjaergaard et al., 2016. APMIS. 124(10):846-55), which might reflect the fact that sCD31 is involved in reducing further leukocyte transmigration in a negative feedback loop pattern (Muller et al., 1993. J Exp Med. 178(2):449-60).

As previously mentioned, the physiological role of the activation of the CD38 receptor function by CD31 remains elusive (Malavasi et al., 2008. Physiol Rev. 88(3):841-86). Here, the Inventors have described that activating this receptor function of CD38 using the extracellular domain of CD31 (sCD31) was able to protect neurons from cell death *in vitro* as well as *in vivo,* which was unexpected. The neuroprotective effect of sCD31 was antagonized in the presence of the neutralizing clone Moon-1 anti-CD31 antibody, as well as by antagonistic anti-CD38 antibodies (clone OKT10 or AT13/5), demonstrating that the neuroprotective effect of sCD31 is mediated through interaction with its ligand CD38. Moreover, the neuroprotective effect of sCD31 was recapitulated by agonist anti-CD38 antibodies that were previously shown to mimic the interaction between sCD31 and CD38 (Deaglio et al., 1998. J Immunol. 160(1):395-402). Using a previously described agonist anti-CD38 antibody (clone HB7) or proprietary agonist anti-CD38 antibodies generated by phage display screening, the Inventors also observed that activation of CD38 receptor function had anti-inflammatory properties *in vitro* using human PBMCs as well as *in vivo* in a mouse model of ulcerative colitis. Altogether, these results demonstrate that engaging CD38 receptor function is of high therapeutic value.

How CD31 activates CD38 receptor function is still unknown. The only published data, dating back over 20 years ago, suggest that a fragment of CD31 consisting of the three first Ig-like domains of CD31 was still able to bind CD38 (Horenstein et al., 1998. Biochem J. 330(3):1129-35). For that reason, agonist anti-CD38 antibodies are still the main option used to activate CD38 receptor function.

Here, the Inventors identified CD31 peptide fragments specifically binding to CD38, that activate CD38 receptor function. Based on the data disclosed herein, these CD31 peptide fragment represent promising hits for the treatment of, *inter alia,* inflammatory diseases, autoimmune diseases, metabolic and endocrine diseases, and neuroinflammatory diseases.

**SUMMARY**

The present invention relates to an isolated peptide which specifically binds to human CD38 having SEQ ID NO: 3 and which comprises at most 25 contiguous amino acid residues from human CD31 having SEQ ID NO: 2 or a variant thereof.

In one embodiment, said isolated peptide is any one of the peptides of Table 1.

In one embodiment, said isolated peptide is a 2-D structured peptide, preferably said isolated peptide is cyclized and/or beta-turned.

In one embodiment, the 2-D structured peptide is any one of the peptides of Table 1, wherein:

a)

(i) a cysteine residue is inserted at both N-terminal and C-terminal extremities; and
(ii) any cysteine residue within the isolated peptide sequence is either protected or mutated to another amino acid residue, preferably to serine; and/or

b) a Pro-Gly or Asp-Gly dipeptide is inserted at a position which is central in said isolated peptide.

In one embodiment, the isolated peptide is selected from the group comprising SEQ ID NOs: 4 to 15, preferably SEQ ID NOs: 4 to 13, more preferably SEQ ID NOs: 6 to 10.

In one embodiment, the isolated peptide is fused to a payload being a therapeutic or diagnostic payload and/or a carrier payload.

The present invention further relates to a nucleic acid encoding the isolated peptide according to the present invention.

The present invention further relates to an expression vector comprising the nucleic acid according to the present invention.

The present invention further relates to a composition comprising at least one isolated peptide according to the present invention.

The present invention further relates to the isolated peptide according to the present invention, for use in preventing and/or treating a disease selected from inflammatory diseases; autoimmune diseases; metabolic and endocrine diseases; neurodegenerative diseases; neuroinflammatory diseases; ocular diseases; age-related diseases; and cancer and metastasis; in a subject in need thereof.

In one embodiment, said disease is selected from multiple sclerosis; rheumatoid arthritis; systemic lupus erythematosus; diabetes; obesity; non-alcoholic steatohepatitis; amyotrophic lateral sclerosis; Parkinson's disease and related disorders; Alzheimer's disease and related disorders; Huntington disease; age-related macular degeneration; and glaucoma.

The present invention further relates to the isolated peptide according to the present invention, for use in increasing the level of at least one anti-inflammatory cytokine in a subject in need thereof, in particular in the blood of said subject.

In one embodiment, said anti-inflammatory cytokine is interleukin-10 (IL-10).

The present invention further relates to the isolated peptide according to the present invention, for use in lowering glucose levels in a subject in need thereof, in particular in the blood of said subject.

## DEFINITIONS

Unless defined otherwise, all technical and scientific terms used herein have the same meaning as commonly understood to one skilled in the relevant art. For convenience, the meaning of certain terms and phrases employed in the specification, examples and claims are provided.

### Binding site, binding pocket

Refers to a specific arrangement of amino acids located on a protein (such as, *e.g.,* on CD38) to which a compound (such as, *e.g.,* the peptides according to the present invention) bind. Binding sites often consist of a chemically-active surface grouping of amino acids, and have specific 3-D structural characteristics as well as specific charge characteristics. Similarly to epitopes, binding sites can be linear or conformational, *i.e.,* they can involve sequences of amino acids which are not necessarily contiguous in the primary structure of the protein.

### CD31

Refers to the 130 kDa type I transmembrane glycoprotein, also known as Platelet endothelial cell adhesion molecule-1 (PECAM-1), PECA1, GPIIA', EndoCAM or CD31/EndoCAMas, and described in Newman & Newman (**2003.**

*Arterioscler Thromb Vasc Biol.* **23**:953-964). In the present invention, the term **"CD31"** refers more particularly to CD31 from a mammal species, even more particularly to human CD31. Preferably, the term **"human CD31"** refers to the protein of amino acid sequence SEQ ID NO: 1, referenced by the NP_000433 NCBI accession number (version 4 of June 09, 2020). The numbering of amino acids of human CD31 as described herein corresponds to the numbering of amino acids of the human CD31 sequence set forth in SEQ ID NO: 1, and referenced by the NP_000433 NCBI accession number. **Human CD31** is composed of several domains:

- a signal peptide, from residues 1 to 27 of SEQ ID NO: 1;
- an extracellular domain, from residues 28 to 601 of SEQ ID NO: 1 (herein referred to as **"CD31extra"),** itself composed of:

  ○ a first Ig-like C2-type domain (herein referred to as **"CD31Ig-like 1"**), from residues 35 to 121 of SEQ ID NO: 1,
  ○ a second Ig-like C2-type domain (herein referred to as **"CD31Ig-like 2"**), from residues 145 to 233 of SEQ ID NO: 1,
  ○ a third Ig-like C2-type domain (herein referred to as **"CD31Ig-like 3"**), from residues 236 to 315 of SEQ ID NO: 1,
  ○ a fourth Ig-like C2-type domain (herein referred to as **"CD31Ig-like 4"**), from residues 328 to 401 of SEQ ID NO: 1,
  ○ a fifth Ig-like C2-type domain (herein referred to as **"CD31Ig-like 5"**), from residues 424 to 493 of SEQ ID NO: 1,
  ○ a sixth Ig-like C2-type domain (herein referred to as **"CD31Ig-like 6"**), from residues 499 to 591 of SEQ ID NO: 1,

- a transmembrane domain, from residues 602 to 620 of SEQ ID NO: 1; and
- a cytoplasmic domain, from residues 621 to 738 of SEQ ID NO: 1.

Preferably, the term **"soluble CD31"** or **"sCD31"** refers to a soluble form of CD31 (*i.e.,* not membrane-bound). Goldberger et al. (1994. J Biol Chem. 269(25):17183-17191) hypothesized that sCD31 was encoded by an alternatively spliced mRNA species from which the exon encoding the transmembrane domain had been remove. More recently, it was shown that the extracellular domain of CD31 comprising the first five Ig-like C2-type domains **(CD31Ig-like 1** to **CD31Ig-like 5**) was cleaved and shed from the surface of human T-cells upon activation via their TCR (Fornasa et al., 2010. J Immunol. 184(10):5485-5492). Accordingly, human sCD31 refers to the protein of amino acid sequence SEQ ID NO: 2, corresponding to amino acid residues 35 to 493 of SEQ ID NO: 1.

## SEQ ID NO: 1

MQPRWAQGATMWLGVLLTLLLCSSLEGQENSFTINSVDMKSLPDWTVQNGKN

LTLQCFADVSTTSHVKPQHQMLFYKDDVLFYNISSMKSTESYFIPEVRIYDSGTY

KCTVIVNNKEKTTAEYQVLVEGVPSPRVTLDKKEAIQGGIVRVNCSVPEEKAPI

HFTIEKLELNEKMVKLREKNSRDQNFVILEFPVEEQDRVLSFRCQARIISGIHM

QTSESTKSELVTVTESFSTPKFHISPTGMIMEGAQLHIKCTIQVTHLAQEFPEIIIQ

KDKAIVAHNRHGNKAVYSVMAMVEHSGNYTCKVESSRISKVSSIVVNITELFS

KPELESSFTHLDQGERLNLSCSIPGAPPANFTIQKEDTIVSQTQDFTKIASKSDSG

TYICTAGIDKVVKKSNTVQIVVCEMLSQPRISYDAQFEVIKGQTIEVRCESISGTL

PISYQLLKTSKVLENSTKNSNDPAVFKDNPTEDVEYQCVADNCHSHAKMLSEV

LRVKVIAPVDEVQISILSSKVVESGEDIVLQCAVNEGSGPITYKFYREKEGKPFY

QMTSNATQAFWTKQKASKEQEGEYYCTAFNRANHASSVPRSKILTVRVILAPW

KKGLIAVVIIGVIIALLIIAAKCYFLRKAKAKQMPVEMSRPAVPLLNSNNEKMSD

PNMEANSHYGHNDDVRNHAMKPINDNKEPLNSDVQYTEVQVSSAESHKDLGK

KDTETVYSEVRKAVPDAVESRYSRTEGSLDGT

## SEQ ID NO: 2

NSVDMKSLPDWTVQNGKNLTLQCFADVSTTSHVKPQHQMLFYKDDVLFYNIS

SMKSTESYFIPEVRIYDSGTYKCTVIVNNKEKTTAEYQVLVEGVPSPRVTLDKK

EAIQGGIVRVNCSVPEEKAPIHFTIEKLELNEKMVKLKREKNSRDQNFVILEFPV

EEQDRVLSFRCQARIISGIHMQTSESTKSELVTVTESFSTPKFHISPTGMIMEGAQ

LHIKCTIQVTHLAQEFPEIIIQKDKAIVAHNRHGNKAVYSVMAMVEHSGNYTCK

VESSRISKVSSIVVNITELFSKPELESSFTHLDQGERLNLSCSIPGAPPANFTIQKED

TIVSQTQDFTKIASKSDSGTYICTAGIDKVVKKSNTVQIVVCEMLSQPRISYDAQ

FEVIKGQTIEVRCESISGTLPISYQLLKTSKVLENSTKNSNDPAVFKDNPTEDVEY

QCVADNCHSHAKMLSEVLR

### CD38

Refers to the 45kDa type II transmembrane glycoprotein also known as T10, cyclic ADP-ribose hydrolase 1, ADPRC1, as described in Malavasi et al. (2008. Physiological Review. 88:841-886). In the present invention, the term **"CD38"** refers more particularly to particularly from a mammal species, even more particularly to human CD38. Preferably, the term **"human CD38"** refers to the protein of amino acid sequence SEQ ID NO: 3, referenced by the NP_001766 NCBI accession number (version 2 of May 24, 2020). The numbering of amino acids of human CD38 as described herein corresponds to the numbering of amino acids of the human CD38 sequence set forth in SEQ ID NO: 3, and referenced by the NP_001766 NCBI accession number.

## SEQ ID NO: 3

MANCEFSPVSGDKPCCRLSRRAQLCLGVSILVLILVVVLAVVVPRWRQQWSGP

GTTKRFPETVLARCVKYTEIHPEMRHVDCQSVWDAFKGAFISKHPCNITEEDYQ

PLMKLGTQTVPCNKILLWSRIKDLAHQFTQVQRDMFTLEDTLLGYLADDLTWC

GEFNTSKINYQSCPDWRKDCSNNPVSVFWKTVSRRFAEAACDVVHVMLNGSR

SKIFDKNSTFGSVEVHNLQPEKVQTLEAWVIHGGREDSRDLCQDPTIKELESIIS

KRNIQFSCKNIYRPDKFLQCVKNPEDSSCTSEI

### Conjugate

With reference to a CD31 peptide, refers to a chimeric CD31 peptide which is bound to a payload, optionally through a linker, thereby forming a single molecule.

### Consist(s/ing) essentially of

With reference to a composition, pharmaceutical composition or medicament, is used herein to intend that the compound according to the present invention is the only one agent with a biologic activity within said composition, pharmaceutical composition or medicament.

### Fragment

With reference to a CD31 peptide, refers to a portion of said CD31 peptide retaining the same or substantially the same biological function, activity and/or local structure, with respect to the specific biological function, activity and/or local structure identified for the CD31 peptide.

### Homolog

With reference to human CD31, refers to a distinct protein from another family or species which is determined by functional, structural or genomic analyses to correspond to human CD31. Most often, homologs will have functional, structural, or genomic similarities. Techniques are known by which homologs of a protein can readily be cloned using genetic probes and PCR. The identity of cloned sequences as homologous can be confirmed using functional assays and/or by genomic mapping of the genes.

### Medicament

Is meant to encompass a composition suitable for administration to a subject or patient, such as a mammal, especially a human. In general, a "medicament" is sterile and is usually free of contaminants that are capable of eliciting an undesirable response within the subject (e.g., the compound(s) in the medicament is pharmaceutical grade). Medicaments can be designed for administration to subjects in need thereof via a number of different routes of administration including oral, buccal, rectal, parenteral, intraperitoneal, intradermal, subcutaneous, intranasal, intrathecal, perispinal and the like. The preferred form depends on the intended mode of administration and therapeutic application. Typical preferred form are oral, injectable or infusible solutions.

### Peptide

Is intended to refer to a molecule that is at least composed of amino acids. A peptide can also possess other molecular groups like polysaccharide chains or other post-translational modifications. **"Recombinant peptide"** specifically refers to a peptide which is produced, expressed, generated or isolated by recombinant means, such as peptides which are expressed using a recombinant expression vector transfected into a host cell. Recombinant peptides include, for example, chimeric peptides. **"Chimeric peptide"** is intended to refer to a peptide that is created through the joining of two or more genes that originally coded for separate proteins or protein fragments; or through the fusion of two or more proteins or protein fragments. Chimeric peptides include, for example, a peptide fused to the Fc region of an IgG, a peptide fused to human serum albumin (HSA) or a particular domain (such as, e.g., domain III) of HSA, or to transferrin (as in Strohl, 2015. BioDrugs. 29(4): 215-239.

### Subject

Refers to a mammal, preferably a human. In one embodiment, a subject may be a "patient", *i.e.,* a warm-blooded animal, more preferably a human, who/which is awaiting the receipt of, or is receiving medical care or was/is/will be the object of a medical procedure, or is monitored for the development of a disease. The term "mammal" refers here to any mammal, including humans, domestic and farm animals, and zoo, sports, or pet animals, such as dogs, cats, cattle, horses, sheep, pigs, goats, rabbits, etc. Preferably, the mammal is a primate, more preferably a human.

### Therapeutically effective amount

Means the level, amount or concentration of agent *(e.g.,* a CD31 peptide specifically binding to CD38) that is aimed at, without causing significant negative or adverse side effects to the subject, (1) delaying or preventing the onset of the targeted disease(s); (2) slowing down or stopping the progression, aggravation, or deterioration of one or more symptoms of the targeted disease(s); (3) bringing about ameliorations of the symptoms of the targeted disease(s); (4) reducing the severity or incidence of the targeted disease(s); or (5) curing the targeted disease(s). A therapeutically effective amount may be administered prior to the onset of the targeted disease(s), for a prophylactic or preventive action. Alternatively or additionally, the therapeutically effective amount may be administered after initiation of the targeted disease(s), for a therapeutic action.

### Treating, treatment, alleviation

Refer to both therapeutic and prophylactic (or preventative) measures; wherein the object is to slow down (or lessen) the targeted disease(s). Those in need of treatment include those already with the targeted disease(s) as well those suspected to have the targeted disease(s).

# EP 3 943 505 A1

## DETAILED DESCRIPTION

The present invention relates to peptides which specifically bind to CD38, preferably to human CD38.

In one embodiment, the peptides according to the present invention are CD31 peptides, preferably human CD31 peptides.

In one embodiment, the peptides according to the present invention are sCD31 peptides, preferably human sCD31 peptides.

In one embodiment, the peptides according to the present invention are isolated CD31 peptides, preferably isolated human CD31 peptides.

In one embodiment, the peptides according to the present invention are isolated sCD31 peptides, preferably isolated human sCD31 peptides.

In one embodiment, the peptides according to the present invention do not consist of the full-length CD31 protein, such as the full-length human CD31 protein.

In one embodiment, the peptides according to the present invention are linear CD31 peptides. By linear peptide, it is meant a peptide which is devoid of secondary, let alone of tertiary structure.

In one embodiment, the peptides according to the present invention are selected from the group comprising or consisting of the peptides of **Table 1.**

**Table 1:** linear CD31 peptides

| SEQ ID NO: 1 residues | | Amino acid sequence |
|---|---|---|
| **Start** | **End** | |
| 52 | 58 | NLTLQSF |
| 54 | 60 | TLQSFAD |
| 55 | 61 | LQSFADV |
| 56 | 62 | QSFADVS |
| 57 | 63 | SFADVST |
| 63 | 82 | TTSHVKPQHQMLFYKDDVLF |
| 64 | 83 | TSHVKPQHQMLFYKDDVLFY |
| 65 | 84 | SHVKPQHQMLFYKDDVLFYN |
| 66 | 80 | HVKPQHQMLFYKDDV |
| 67 | 81 | VKPQHQMLFYKDDVL |
| 67 | 86 | VKPQHQMLFYKDDVLFYNIS |
| 68 | 82 | KPQHQMLFYKDDVLF |
| 68 | 87 | KPQHQMLFYKDDVLFYNIS S |
| 70 | 84 | QHQMLFYKDDVLFYN |
| 71 | 85 | HQMLFYKDDVLFYNI |
| 72 | 86 | QMLFYKDDVLFYNIS |
| 73 | 79 | MLFYKDD |
| 73 | 87 | MLFYKDDVLFYNIS S |
| 74 | 80 | LFYKDDV |
| 74 | 88 | LFYKDDVLFYNIS SM |
| 76 | 82 | YKDDVLF |
| 76 | 95 | YKDDVLFYNISSMKSTESYF |
| 77 | 83 | KDDVLFY |
| 77 | 96 | KDDVLFYNISSMKSTESYFI |
| 78 | 84 | DDVLFYN |

7

(continued)

| SEQ ID NO: 1 residues | | Amino acid sequence |
|---|---|---|
| Start | End | |
| 78 | 97 | DDVLFYNISSMKSTESYFIP |
| 80 | 86 | VLFYNIS |
| 80 | 94 | VLFYNISSMKSTESY |
| 80 | 99 | VLFYNISSMKSTESYFIPEV |
| 82 | 96 | FYNISSMKSTESYFI |
| 83 | 97 | YNISSMKSTESYFIP |
| 84 | 98 | NISSMKSTESYFIPE |
| 85 | 99 | ISSMKSTESYFIPEV |
| 86 | 100 | SSMKSTESYFIPEVR |
| 88 | 102 | MKSTESYFIPEVRIY |
| 89 | 95 | KSTESYF |
| 89 | 103 | KSTESYFIPEVRIYD |
| 90 | 96 | STESYFI |
| 90 | 104 | STESYFIPEVRIYDS |
| 91 | 97 | TESYFIP |
| 91 | 105 | TESYFIPEVRIYDSG |
| 92 | 106 | ESYFIPEVRIYDSGT |
| 93 | 99 | SYFIPEV |
| 94 | 100 | YFIPEVR |
| 94 | 108 | YFIPEVRIYDSGTYK |
| 95 | 101 | FIPEVRI |
| 98 | 104 | EVRIYDS |
| 99 | 105 | VRIYDSG |
| 100 | 106 | RIYDSGT |
| 120 | 126 | TAEYQVL |
| 121 | 127 | AEYQVLV |
| 122 | 128 | EYQVLVE |
| 173 | 192 | KMVKLKREKNSRDQNFVILE |
| 174 | 193 | MVKLKREKNSRDQNFVILEF |
| 176 | 195 | KLKREKNSRDQNFVILEFPV |
| 177 | 196 | LKREKNSRDQNFVILEFPVE |
| 178 | 192 | KREKNSRDQNFVILE |
| 179 | 193 | REKNSRDQNFVILEF |
| 180 | 194 | EKNSRDQNFVIILEFP |
| 181 | 195 | KNSRDQNFVILEFPV |
| 182 | 196 | NSRDQNFVILEFPVE |
| 184 | 198 | RDQNFVILEFPVEEQ |

(continued)

| SEQ ID NO: 1 residues | | Amino acid sequence |
|---|---|---|
| Start | End | |
| 185 | 191 | DQNFVIL |
| 185 | 199 | DQNFVILEFPVEEQD |
| 186 | 192 | QNFVILE |
| 186 | 200 | QNFVILEFPVEEQDR |
| 187 | 193 | NFVILEF |
| 187 | 201 | NFVILEFPVEEQDRV |
| 188 | 202 | FVILEFPVEEQDRVL |
| 189 | 195 | VILEFPV |
| 190 | 196 | ILEFPVE |
| 195 | 201 | VEEQDRV |
| 256 | 270 | STIQVTHLAQEFPEI |
| 257 | 271 | TIQVTHLAQEFPEII |
| 258 | 272 | IQVTHLAQEFPEIII |
| 259 | 273 | QVTHLAQEFPEIIIQ |
| 260 | 274 | VTHLAQEFPEIIIQK |
| 263 | 269 | LAQEFPE |
| 264 | 270 | AQEFPEI |
| 265 | 271 | QEFPEII |
| 267 | 273 | FPEIIIQ |
| 304 | 323 | SKVESSRISKVSSIVVNITE |
| 306 | 325 | VES SRISKVS SIVVNITELF |
| 307 | 326 | ESSRISKVSSIVVNITELFS |
| 308 | 322 | SSRISKVSSIVVNIT |
| 310 | 324 | RISKVSSIVVNITEL |
| 311 | 325 | ISKVSSIVVNITELF |
| 312 | 326 | SKVSSIVVNITELFS |
| 313 | 327 | KVSSIVVNITELFSK |
| 315 | 321 | SSIVVNI |
| 316 | 322 | SIVVNIT |
| 317 | 323 | IVVNITE |
| 317 | 325 | IVVNITELF |
| 319 | 325 | VNITELF |
| 320 | 326 | NITELFS |
| 353 | 372 | PPANFTIQKEDTIVSQTQDF |
| 354 | 373 | PANFTIQKEDTIVSQTQDFT |
| 401 | 420 | QIVVCEMLSQPRISYDAQFE |
| 402 | 421 | IVVCEMLSQPRISYDAQFEV |

(continued)

| SEQ ID NO: 1 residues | | Amino acid sequence |
|---|---|---|
| Start | End | |
| 403 | 422 | VVCEMLSQPRISYDAQFEVI |
| 412 | 418 | RISYDAQ |
| 414 | 420 | SYDAQFE |
| 415 | 421 | YDAQFEV |
| 416 | 422 | DAQFEVI |
| 457 | 471 | NSNDPAVFKDNPTED |
| 458 | 472 | SNDPAVFKDNPTEDV |
| 460 | 474 | DPAVFKDNPTEDVEY |
| 461 | 475 | PAVFKDNPTEDVEYQ |
| 463 | 477 | VFKDNPTEDVEYQCV |
| 467 | 473 | NPTEDVE |
| 468 | 474 | PTEDVEY |
| 470 | 475 | EDVEYQ |
| 470 | 476 | EDVEYQC |
| 471 | 477 | DVEYQCV |

In one embodiment, the peptides according to the present invention are selected from the group comprising or consisting of the peptides of **Table 2.**

**Table 2:** linear CD31 peptides - core binding sequences

| SEQ ID NO: 1 residues | | Amino acid sequence |
|---|---|---|
| Start | End | |
| 68 | 82 | KPQHQMLFYKDDVLF |
| 74 | 82 | LFYKDDVLF |
| 74 | 84 | LFYKDDVLFYN |
| 80 | 96 | VLFYNISSMKSTESYFI |
| 82 | 96 | FYNISSMKSTESYFI |
| 91 | 96 | TESYFI |
| 91 | 103 | TESYFIPEVRIYD |
| 95 | 99 | FIPEV |
| 99 | 104 | VRIYDS |
| 122 | 126 | EYQVL |
| 174 | 193 | MVKLKREKNSRDQNFVILEF |
| 179 | 193 | REKNSRDQNFVILEF |
| 184 | 196 | RDQNFVILEFPVE |
| 185 | 196 | DQNFVILEFPVE |
| 187 | 192 | NFVILE |
| 259 | 272 | QVTHLAQEFPEIII |

(continued)

| SEQ ID NO: 1 residues | | Amino acid sequence |
|---|---|---|
| **Start** | **End** | |
| 267 | 270 | FPEI |
| 307 | 323 | ESSRISKVSSIVVNITE |
| 311 | 324 | ISKVSSIVVNITEL |
| 312 | 324 | SKVSSIVVNITEL |
| 317 | 323 | IVVNITE |
| 320 | 325 | NITELF |
| 353 | 372 | PPANFTIQKEDTIVSQTQDF |
| 403 | 422 | VVCEMLSQPRISYDAQFEVI |
| 416 | 420 | DAQFE |
| 463 | 471 | VFKDNPTED |
| 471 | 476 | DVEYQC |

In one embodiment, the peptides according to the present invention are selected from the group comprising or consisting of LFYKDDVLF (residues 74 to 82 of SEQ ID NO: 1); TESYFIPEVRIYDS (residues 90 to 104 of SEQ ID NO: 1); EYQVL (residues 122 to 126 of SEQ ID NO: 1); MVKLKREKNSRDQNFVILEFPVE (residues 174 to 196 of SEQ ID NO: 1); QVTHLAQEFPEIII (residues 259 to 272 of SEQ ID NO: 1); ESSRISKVSSIVVNITEL (residues 307 to 324 of SEQ ID NO: 1); VVSEMLSQP (residues 403 to 411 of SEQ ID NO: 1); DAQFE (residues 416 to 420 of SEQ ID NO: 1); and VFKDNPTEDVEYQS (residues 463 to 476 of SEQ ID NO: 1).

In one embodiment, the peptides according to the present invention are selected from the group comprising or consisting of STESYFI (residues 90 to 96 of SEQ ID NO: 1); MVKLKREKNSRDQNFVILEF (residues 174 to 193 of SEQ ID NO: 1); NSRDQNFVILEFPVE (residues 182 to 196 of SEQ ID NO: 1); RDQNFVILEFPVEEQ (residues 184 to 198 of SEQ ID NO: 1); NFVILEF (residues 187 to 193 of SEQ ID NO: 1); VEEQDRV (residues 195 to 201 of SEQ ID NO: 1); VESSRISKVSSIVVNITELF (residues 306 to 325 of SEQ ID NO: 1); IVVNITELF (residues 317 to 325 of SEQ ID NO: 1); PPANFTIQKEDTIVSQTQDF (residues 353 to 372 of SEQ ID NO: 1); EDVEYQ (residues 470 to 475 of SEQ ID NO: 1); and EDVEYQC (residues 470 to 476 of SEQ ID NO: 1).

In one embodiment, the peptides according to the present invention are 2-D structured CD31 peptides. By 2-D structured peptide, it is meant a peptide which adopts a secondary structure in solution, such as, *e.g.,* a single loop, double loop, triple loop, a β-sheet-like fold, an α-helix-like fold, etc. Such secondary structures can form naturally in solution and/or be constrained through the insertion of amino acid residues and/or chemical moieties triggering or stabilizing the formation of said secondary structures.

In one embodiment, the 2-D structured peptides according to the present invention are cyclized, i.e., they mimic a loop.

In one embodiment, cyclization to create a loop mimic may be carried out by any means known by the one skilled in the art, including, without limitation, the formation of a disulphide bridge between cysteine residues; the formation of a lactam bridge between glutamic or aspartic acid and lysine residues; the formation of a lactone or thiolactone bridge between amino acid residues containing carboxyl, hydroxyl or mercapto functional groups; the formation of a thioether or ether bridge between amino acid residues containing hydroxyl or mercapto functional groups; the formation of an amide bond between the N-terminal α-amino group and the C-terminal α-carboxy group; and the like.

In one embodiment, the reactive amino acid residues (e.g., the cysteine residues for the formation of a disulphide bridge, the glutamic or aspartic acid and lysine residues for the formation of a lactam bridge, etc.) may be already present in the peptide to be cyclized, or can alternatively be inserted at the N-terminal and C-terminal extremities of the peptide. In the latter case, it will be understood that reactive amino acid residue(s) present within the peptide sequence shall be either protected or mutated to another amino acid residue so as to avoid unwanted bridge formation.

In one embodiment, the 2-D structured peptides according to the present invention are selected from the group comprising or consisting of the peptides of **Table** 1, wherein:

(i) a cysteine residue is inserted at both N-terminal and C-terminal extremities; and
(ii) any cysteine residue within the peptide sequence is either protected or mutated to another amino acid residue, preferably to serine.

In one embodiment, the 2-D structured peptides according to the present invention are selected from the group comprising or consisting of the peptides of **Table 2,** wherein:

(i) a cysteine residue is inserted at both N-terminal and C-terminal extremities; and
(ii) any cysteine residue within the peptide sequence is either protected or mutated to another amino acid residue, preferably to serine.

In one embodiment, the 2-D structured peptides according to the present invention are selected from the group comprising or consisting of CLFYKDDVLFC (residues 74 to 82 of SEQ ID NO: 1 with a cysteine residue inserted at both N-terminal and C-terminal extremities); CTESYFIPEVRIYDSC (residues 90 to 104 of SEQ ID NO: 1 with a cysteine residue inserted at both N-terminal and C-terminal extremities); EYQVL (residues 122 to 126 of SEQ ID NO: 1); CMVKLKREKNSRDQNFVILEFPVEC (residues 174 to 196 of SEQ ID NO: 1 with a cysteine residue inserted at both N-terminal and C-terminal extremities); CQVTHLAQEFPEIIIC (residues 259 to 272 of SEQ ID NO: 1 with a cysteine residue inserted at both N-terminal and C-terminal extremities); CESSRISKVSSIVVNITELC (residues 307 to 324 of SEQ ID NO: 1 with a cysteine residue inserted at both N-terminal and C-terminal extremities); CVVSEMLSQPC (residues 403 to 411 of SEQ ID NO: 1 with a cysteine residue inserted at both N-terminal and C-terminal extremities); CDAQFEC (residues 416 to 420 of SEQ ID NO: 1 with a cysteine residue inserted at both N-terminal and C-terminal extremities); and CVFKDNPTEDVEYQSC (residues 463 to 476 of SEQ ID NO: 1 with a cysteine residue inserted at both N-terminal and C-terminal extremities).

In one embodiment, the 2-D structured peptides according to the present invention are selected from the group comprising or consisting of CSTESYFIC (residues 90 to 96 of SEQ ID NO: 1 with a cysteine residue inserted at both N-terminal and C-terminal extremities); CMVKLKREKNSRDQNFVILEFC (residues 174 to 193 of SEQ ID NO: 1 with a cysteine residue inserted at both N-terminal and C-terminal extremities); CNSRDQNFVILEFPVEC (residues 182 to 196 of SEQ ID NO: 1 with a cysteine residue inserted at both N-terminal and C-terminal extremities); CRDQNFVILEFPVEEQC (residues 184 to 198 of SEQ ID NO: 1 with a cysteine residue inserted at both N-terminal and C-terminal extremities); CNFVILEFC (residues 187 to 193 of SEQ ID NO: 1 with a cysteine residue inserted at both N-terminal and C-terminal extremities); CVEEQDRVC (residues 195 to 201 of SEQ ID NO: 1 with a cysteine residue inserted at both N-terminal and C-terminal extremities); CVESSRISKVSSIVVNITELFC (residues 306 to 325 of SEQ ID NO: 1 with a cysteine residue inserted at both N-terminal and C-terminal extremities); CIVVNITELFC (residues 317 to 325 of SEQ ID NO: 1 with a cysteine residue inserted at both N-terminal and C-terminal extremities); CPPANFTIQKEDTIVSQTQDFC (residues 353 to 372 of SEQ ID NO: 1 with a cysteine residue inserted at both N-terminal and C-terminal extremities); CEDVEYQC (residues 470 to 475 of SEQ ID NO: 1 with a cysteine residue inserted at both N-terminal and C-terminal extremities); and CEDVEYQSC (residues 470 to 476 of SEQ ID NO: 1 with a cysteine residue inserted at both N-terminal and C-terminal extremities and a C476S mutation).

In one embodiment, the 2-D structured peptides according to the present invention are beta-turned, *i.e.,* they mimic a β-sheet-like fold.

In one embodiment, constraining a peptide into a β-sheet-like fold may be carried out by any means known by the one skilled in the art, including, without limitation, the insertion of a Pro-Gly dipeptide to induce a type II' β-turn, the insertion of a Asp-Gly dipeptide to induce a type I' β-turn, and the like.

Preferably, the dipeptide is inserted - or amino acid residues in the native peptide sequence are mutated into the desired dipeptide - at a position which is central in the peptide, *i.e.,* the number of amino acid residues on one side of the dipeptide is not 50%, preferably 20%, more preferably 10% higher or lower than the number of amino acid residues on the other side of the dipeptide. One skilled in the art will readily understand that the dipeptide shall be at a position which is central in the peptide so as to allow a complete turn of the peptide to mimic a β-sheet-like fold.

It is readily understandable that the expression "the dipeptide is inserted" also encompasses cases where amino acid residues in the native peptide sequence are mutated into the desired dipeptide.

In one embodiment, constraining a peptide into a β-sheet-like fold may further require cyclization of the peptide by any means known by the one skilled in the art, including those described hereinabove.

In one embodiment, the 2-D structured peptides according to the present invention are selected from the group comprising or consisting of the peptides of Table **1,** wherein:

(i) a Pro-Gly or Asp-Gly dipeptide, preferably a Pro-Gly dipeptide, is inserted at a position which is central in the peptide; and optionally
(ii) a cysteine residue is inserted at both N-terminal and C-terminal extremities; and
(iii) any cysteine residue within the peptide sequence is either protected or mutated to another amino acid residue, preferably to serine.

In one embodiment, the 2-D structured peptides according to the present invention are selected from the group comprising or consisting of the peptides of **Table 2,** wherein:

(i) a Pro-Gly or Asp-Gly dipeptide, preferably a Pro-Gly dipeptide, is inserted at a position which is central in the peptide; and optionally
(ii) a cysteine residue is inserted at both N-terminal and C-terminal extremities; and
(iii) any cysteine residue within the peptide sequence is either protected or mutated to another amino acid residue, preferably to serine.

In one embodiment, the peptides according to the present invention are selected from the group comprising or consisting of the peptides of **Table 3.**

**Table 3.**

| SEQ ID NO: 1 residues | | Amino acid sequence | Structure | SEQ ID NO: |
|---|---|---|---|---|
| Start | Stop | | | |
| 90 | 96 | STESYFI | Linear | 4 |
| 90 | 96 | CSTESYFIC | Loop | 5 |
| 182 | 196 | NSRDQNFVILEFPVE | Linear | 6 |
| 187 | 193 | NFVILEF | Linear | 7 |
| 187 | 193 | CNFVILEFC | Loop | 8 |
| 184 | 198 | CRDQNFVILEFPVEEQC | Loop | 9 |
| 174 | 193 | CMVKLKREKNPGDQNFVILEFC | β-sheet-like fold | 10 |
| 195 | 201 | CVEEQDRVC | Loop | 11 |
| 317 | 325 | CIVVNITELFC | Loop | 12 |
| 306 | 322 | CVESSRISKVPGIVVNITELFC | β-sheet-like fold | 13 |
| 353 | 372 | CPPANFTIQKPGTIVSQTQDFC | β-sheet-like fold | 14 |
| 470 | 475 | CEDVEYQC | Loop | 15 |

In one embodiment, the peptides according to the present invention are selected from the group comprising or consisting of the peptides set forth in SEQ ID NOs: 4, 5, 6, 7, 8, 9, 10, 11, 12 and 13.

In one embodiment, the peptides according to the present invention are selected from the group comprising or consisting of the peptides set forth in SEQ ID NOs: 6, 7, 8, 9 and 10.

When reciting "the peptides according to the present invention" or "the CD31 peptide(s)", also encompassed are CD31 peptide variants, preferably human CD31 peptide variants.

CD31 peptide variants encompass (1) peptides of human CD31 homologs, (2) fragments of the CD31 peptides described above, (3) mutants of the CD31 peptides described above, (4) peptidomimetics of the CD31 peptides described above, and (5) conjugates of the CD31 peptides described above, including any combinations thereof.

Homologs of human CD31 include, but are not limited to, CD31 from *Mus musculus* (UniProtKB entry: Q08481), from *Rattus norvegicus* (UniProtKB entry: Q3 SWT0), from *Sus scrofa* (UniProtKB entry: Q95242), and from *Bos taurus* (UniProtKB entry: P51866).

In one embodiment, a fragment of a peptide as described herein above comprises at least 70%, preferably at least 75%, 80%, 85%, 90%, 95%, 96%, 97%, 98%, 99% or more amino acids of said peptide.

In one embodiment, a mutant of a peptide as described herein above shares at least 70%, preferably at least 75%, 80%, 85%, 90%, 95%, 96%, 97%, 98%, 99% or more sequence identity, preferably local sequence identity with said peptide.

Sequence identity refers to the number of identical or similar amino acids in a comparison between a test and a reference sequence. Sequence identity can be determined by sequence alignment of protein sequences to identify regions of similarity or identity. For purposes herein, sequence identity is generally determined by alignment to identify identical residues. The alignment can be local or global. Matches, mismatches and gaps can be identified between compared sequences. Gaps are null amino acids inserted between the residues of aligned sequences so that identical or similar characters are aligned. Generally, there can be internal and terminal gaps. When using gap penalties, sequence identity can be determined with no penalty for end gaps (e.g., terminal gaps are not penalized). Alternatively, sequence identity can be determined without taking into account gaps as $\dfrac{\text{number of identical positions}}{\text{length of the total aligned sequence}} \times 100$.

A global alignment is an alignment that aligns two sequences from beginning to end, aligning each letter in each sequence only once. An alignment is produced, regardless of whether or not there is similarity or identity between the sequences. For example, 50% sequence identity based on global alignment means that in an alignment of the full sequence of two compared sequences, each of 100 nucleotides in length, 50% of the residues are the same. It is understood that global alignment can also be used in determining sequence identity even when the length of the aligned sequences is not the same. The differences in the terminal ends of the sequences will be taken into account in determining sequence identity, unless the "no penalty for end gaps" is selected. Generally, a global alignment is used on sequences that share significant similarity over most of their length. Exemplary algorithms for performing global alignment include the Needleman-Wunsch algorithm (Needleman & Wunsch, 1970. J Mol Biol. 48(3):443-53). Exemplary programs and software for performing global alignment are publicly available and include the Global Sequence Alignment Tool available at the National Center for Biotechnology Information (NCBI) website (http://ncbi.nlm.nih.gov), and the program available at deepc2.psi.iastate.edu/aat/align/align.html.

A local alignment is an alignment that aligns two sequence, but only aligns those portions of the sequences that share similarity or identity. Hence, a local alignment determines if sub-segments of one sequence are present in another sequence. If there is no similarity, no alignment will be returned. Local alignment algorithms include BLAST or Smith-Waterman algorithm (Smith & Waterman, 1981. Adv Appl Math. 2(4):482-9). For example, 50% sequence identity based on local alignment means that in an alignment of the full sequence of two compared sequences of any length, a region of similarity or identity of 100 nucleotides in length has 50% of the residues that are the same in the region of similarity or identity.

For purposes herein, sequence identity can be determined by standard alignment algorithm programs used with default gap penalties established by each supplier. Default parameters for the GAP program can include:

(1) a unary comparison matrix (containing a value of 1 for identities and 0 for non-identities) and the weighted comparison matrix of Gribskov & Burgess (1986. Nucleic Acids Res. 14(16):6745-63), as described by Schwartz & Dayhoff (1979. Matrices for detecting distant relationships. In Dayhoff (Ed.), Atlas of protein sequences. 5:353-358. Washington, DC: National Biomedical Research Foundation);
(2) a penalty of 3.0 for each gap and an additional 0.10 penalty for each symbol in each gap; and
(3) no penalty for end gaps.

Whether any peptide and peptide mutant have amino acid sequences that are at least 70%, preferably at least 75%, 80%, 85%, 90%, 95%, 96%, 97%, 98%, 99% or more "identical", or other similar variations reciting a percent identity, can be determined using known computer algorithms based on local or global alignment (see, *e.g.,* https://en.wikipedia.org/wiki/List_of_sequence_alignment_software [last accessed on July 2021, 2020], providing links to dozens of known and publicly available alignment databases and programs).

Generally, for purposes herein, sequence identity is determined using computer algorithms based on global alignment, such as the Needleman-Wunsch Global Sequence Alignment tool available from NCBI/BLAST (http://blast.ncbi.nlm.nih.gov/Blast.cgi); or LAlign (William Pearson implementing the Huang and Miller algorithm [Huang & Miller, 1991. Adv Appl Math. 12(3):337-57).

Typically, the full-length sequence of each of the compared peptides is aligned across the full-length of each sequence in a global alignment. Local alignment also can be used when the sequences being compared are substantially the same length.

Therefore, the term identity represents a comparison or alignment between a test and a reference peptide. In one exemplary embodiment, "at least 70% of sequence identity" refers to percent identities from 70 to 100% relative to the reference peptide. Identity at a level of 70% or more is indicative of the fact that, assuming for exemplification purposes a test and reference peptide length of 100 amino acids are compared, no more than 30 out of 100 amino acids in the test peptide differ from those of the reference peptide. Such differences can be represented as point mutations randomly distributed over the entire length of an amino acid sequence or they can be clustered in one or more locations of varying length up to the maximum allowable, *e.g.,* 30/100 amino acid difference (approximately 70% identity). Differences can also be due to deletions or truncations of amino acid residues. Differences are defined as amino acid substitutions, insertions or deletions. Depending on the length of the compared sequences, at the level of homologies or identities above about 85-90%, the result can be independent of the program and gap parameters set; such high levels of identity can be assessed readily, often without relying on software.

Peptidomimetics of the peptides described hereinabove comprise, but are not limited to, retro analogues, inverso analogues and retroinverso analogues of said peptides.

The term **"retro analogue"** with reference to a peptide means a reversed sequence of amino acid residues of said peptide; the term **"inverso analogue"** with reference to a peptide means the same sequence of amino acid residues of said peptide but with D-amino acid residues instead of L-amino acid residues. The term **"retroinverso analogue"** with reference to a peptide encompasses both a reversed sequence of amino acid residues of said peptide, with D-amino acid residues instead of L-amino acid residues.

In one embodiment, the peptidomimetics is a retro analogue. In one embodiment, the peptidomimetics is an inverso analogue. In one embodiment, the peptidomimetics is a retroinverso analogue.

In one embodiment, a conjugate of a peptide according to the present invention comprises or consists of said peptide fused to a payload.

In one embodiment, the payload may be a therapeutic or diagnostic payload, *i.e.,* a fused therapeutic or diagnostic molecule which role is to support, supplement, synergize and/or target the therapeutic or diagnostic role of the peptide according to the present invention.

Examples of therapeutic or diagnostic payloads suitable for conjugation with peptides according to the present invention include, but are not limited to, peptides, polypeptides, proteins, polymers, nucleic acid molecules, small molecules, mimetic agents, synthetic drugs, inorganic molecules, organic molecules, radioisotopes, nanoparticles, vectors, and the like.

Alternatively, or additionally, examples of therapeutic or diagnostic payloads suitable for conjugation with peptides according to the present invention include, but are not limited to, chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, hormones, coding or non-coding oligonucleotides, photodetectable labels, contrast agents, radiolabels, and the like.

It will be apparent that some therapeutic or diagnostic payloads may fall into more than one category. It will also be readily understood that the conjugation of a peptide according to the present invention with a particular therapeutic or diagnostic payload, which may be chosen among those recited herein but without being limited thereto, depends on the intended use of said conjugate, e.g., on a specific disease or condition to be prevented and/or treated.

In one embodiment, the therapeutic or diagnostic payload is a chemotherapeutic agent.

As used herein, the term **"chemotherapeutic agent"** refers to any molecule that is effective in inhibiting tumor growth.

Suitable examples of chemotherapeutic agents include, but are not limited to:

- alkylating agents, such as, *e.g.:*

  ■ nitrogen mustards, including chlormethine, cyclophosphamide, ifosfamide, trofosfamide, chlorambucil, melphalan, prednimustine, bendamustine, uramustine, chlornaphazine, cholophosphamide, estrarnustine, mechlorethamine, mechlorethamine oxide hydrochloride, novembichin, phenesterine, uracil mustard and the like;
  ■ nitrosoureas, including carmustine, lomustine, semustine, fotemustine, nimustine, ranimustine, streptozocin, chlorozotocin, and the like;
  ■ alkyl sulfonates, including busulfan, mannosulfan, treosulfan, and the like;
  ■ aziridines, including carboquone, thiotepa, triaziquone, triethylenemelamine, benzodopa, meturedopa, uredopa, and the like; hydrazines, including procarbazine, and the like;
  ■ triazenes, including dacarbazine, temozolomide, and the like; ethylenimines and methylamelamines, including altretamine, triethylenemelamine, trietylenephosphoramide, triethyl enethiophosphaorarnide, trimethylolomelamine and the like;
  ■ and others, including mitobronitol, pipobroman, actinomycin, bleomycin, mitomycins (including mitomycin C, and the like), plicamycin, and the like;

- acetogenins, such as, *e.g.,* bullatacin, bullatacinone, and the like;
- benzodiazepines, such as, *e.g.,* 2-oxoquazepam, 3-hydroxyphenazepam, bromazepam, camazepam, carburazepam, chlordiazepoxide, cinazepam, cinolazepam, clonazepam, cloniprazepam, clorazepate, cyprazepam, delorazepam, demoxepam, desmethylflunitrazepam, devazepide, diazepam, diclazepam, difludiazepam, doxefazepam, elfazepam, ethyl carfluzepate, ethyl dirazepate, ethyl loflazepate, flubromazepam, fletazepam, fludiazepam, flunitrazepam, flurazepam, flutemazepam, flutoprazepam, fosazepam, gidazepam, halazepam, iclazepam, irazepine, kenazepine, ketazolam, lorazepam, lormetazepam, lufuradom, meclonazepam, medazepam, menitrazepam, metaclazepam, motrazepam, *N*-desalkylflurazepam, nifoxipam, nimetazepam, nitemazepam, nitrazepam, nitrazepate, nordazepam, nortetrazepam, oxazepam, phenazepam, pinazepam, pivoxazepam, prazepam, proflazepam, quazepam, QH-II-66, reclazepam, RO4491533, Ro5-4864, SH-I-048A, sulazepam, temazepam, tetrazepam, tifluadom, tolufazepam, triflunordazepam, tuclazepam, uldazepam, arfendazam, clobazam, CP-1414S, lofendazam, triflubazam, girisopam, GYKI-52466, GYKI-52895, nerisopam, talampanel, tofisopam, adinazolam, alprazolam, bromazolam, clonazolam, estazolam, flualprazolam, flubromazolam, flunitrazolam, nitrazolam, pyrazolam, triazolam, bretazenil, climazolam, EVT-201, FG-8205, flumazenil, GL-II-73, imidazenil, $^{123}$I-iomazenil, L-655,708, loprazolam, midazolam, PWZ-029, remimazolam, Ro15-4513, Ro48-6791, Ro48-8684, Ro4938581, sarmazenil, SH-053-R-CH3-2'F, cloxazolam, flutazolam, haloxazolam, mexazolam, oxazolam, bentazepam, clotiazepam, brotizolam, ciclotizolam, deschloroetizolam, etizolam, fluclotizolam, israpafant, JQ1, metizolam, olanzapine, telenzepine, lopirazepam, zapizolam, razobazam, ripazepam, zolazepam, zomebazam, zometapine, premazepam, clazolam, anthramycin, avizafone, rilmazafone, and the like;

- antimetabolites, such as, *e.g.:*

   ▪ antifolates, including aminopterin, methotrexate, pemetrexed, pralatrexed, pteropterin, raltitrexed, denopterin, trimetrexate, pemetrexed, and the like;
   ▪ purine analogues, including pentostatin, cladribine, clofarabine, fludarabine, nelarabine, tioguanine, mercaptopurine, and the like;
   ▪ pyrimidine analogues, including fluorouracil, capecitabine, doxifluridine, tegafur, tegafur/gimeracil/oteracil, carmofur, floxuridine, cytarabine, gemcitabine, azacytidine, decitabine, and the like; and
   ▪ hydroxycarbamide;

- androgens, such as, *e.g.,* calusterone, dromostanolone propionate, epitiostanol, mepitiostane, testolactone, and the like;
- anti-adrenals, such as, *e.g.,* aminoglutethimide, mitotane, trilostane, and the like;
- folic acid replenishers, such as, *e.g.,* frolinic acid, and the like;
- maytansinoids, such as, *e.g.,* maytansine, ansamitocins, and the like;
- platinum analogs, such as, *e.g.,* platinum, carboplatin, cisplatin, dicycloplatin, nedaplatin, oxaliplatin, satraplatin, and the like;
- antihormonal agents, such as, *e.g.:*

   ▪ anti-estrogens, including tamoxifen, raloxifene, aromatase inhibiting 4(5)-imidazoles, 4-hydroxytamoxifen, trioxifene, keoxifene, LY117018, onapristone, toremifene, and the like;
   ▪ anti-androgens, including flutamide, nilutamide, bicalutamide, leuprolide, goserelin, and the like;

- trichothecenes, such as, *e.g.,* T-2 toxin, verracurin A, roridinA, anguidine and the like;
- toxoids, such as, e.g., cabazitaxel, docetaxel, larotaxel, ortataxel, paclitaxel, tesetaxel, and the like;
- others, such as, e.g., camptothecin (including the synthetic analogue topotecan); bryostatin; callystatin; CC-1065 (including its adozelesin, carzelesin and bizelesin synthetic analogues); cryptophycins (including cryptophycin 1 and cryptophycin 8); dolastatin; duocarmycin (including its synthetic analogues KW-2189 and CBI-TMI); eleutherobin; pancratistatin; sarcodictyin; spongistatin; aclacinomysins; authramycin; azaserine; bleomycin; cactinomycin; carabicin; canninomycin; carzinophilin; chromomycins; dactinomycin; daunorubicin; detorubicin; 6-diazo-5-oxo-L-norleucine; doxorubicin (including morpholino-doxorubicin, cyanomorpholino-doxorubicin, 2-pyrrolino-doxorubicin, deoxydoxorubicin, and the like); epirubicin; esorubicin; idanrbicin; marcellomycin; mycophenolic acid; nogalarnycin; olivomycins; peplomycin; potfiromycin; puromycin; quelamycin; rodorubicin; streptomgrin; streptozocin; tubercidin; ubenimex; zinostatin; zorubicin; aceglatone; aldophospharnide glycoside; aminolevulinic acid; amsacrine; bestrabucil; bisantrene; edatraxate; defofamine; demecolcine; diaziquone; elfornithine; elliptinium acetate; epothilone; etoglucid; gallium nitrate; hydroxyurea; lentinan; lonidamine; mitoguazone; mitoxantrone; mopidamol; nitracrine; phenamet; pirarubicin; podophyllinic acid; 2-ethylhydrazide; PSK®; razoxane; rhizoxin; sizofiran; spirogennanium; tenuazonic acid; 2,2',2"-trichlorotriethylarnine; urethan; vindesine; dacarbazine; mannomustine; mitobromtol; mitolactol; pipobroman; gacytosine; arabinoside; 6-thioguanine; vinblastine; etoposide; vincristine; vinorelbine; navelbine; novantrone; teniposide; daunomycin; xeloda; ibandronate; CPT-11; topoisomerase inhibitor RFS 2000; topoisomerase I inhibitor SN38; difluoromethylornithine; retinoic acid; and the like.

In one embodiment, the therapeutic or diagnostic payload is a targeted therapy agent.
As used herein, the term **"targeted therapy agent"** refers to any molecule which aims at one or more particular target molecules (such as, *e.g.,* proteins) involved in tumor genesis, tumor progression, tumor metastasis, tumor cell proliferation, cell repair, and the like.
Suitable examples of targeted therapy agents include, but are not limited to, tyrosine-kinase inhibitors, serine/threonine kinase inhibitors, monoclonal antibodies and the like.
Suitable examples of targeted therapy agents include, but are not limited to, HER1/EGFR inhibitors (such as, *e.g.,* brigatinib, erlotinib, gefitinib, olmutinib, osimertinib, rociletinib, vandetanib, and the like); HER2/neu inhibitors (such as, *e.g.,* afatinib, lapatinib, neratinib, and the like); C-kit and PDGFR inhibitors (such as, *e.g.,* axitinib, masitinib, pazopanib, sunitinib, sorafenib, toceranib, and the like); FLT3 inhibitors (such as, *e.g.,* lestaurtinib, and the like); VEGFR inhibitors (such as, *e.g.,* axitinib, cediranib, lenvatinib, nintedanib, pazopanib, regorafenib, semaxanib, sorafenib, sunitinib, tivozanib, toceranib, vandetanib, and the like); RET inhibitors (such as, *e.g.,* vandetanib, entrectinib, and the like); c-MET inhibitors (such as, *e.g.,* cabozantinib, and the like); bcr-abl inhibitors (such as, *e.g.,* imatinib, dasatinib, nilotinib, ponatinib, radotinib, and the like); Src inhibitors (such as, *e.g.,* bosutinib, dasatinib, and the like); Janus kinase inhibitors (such as, *e.g.,* lestaurtinib, momelotinib, ruxolitinib, pacritinib, and the like); MAP2K inhibitors (such as, *e.g.,* cobimetinib, selumetinib, trametinib, binimetinib, and the like); EML4-ALK inhibitors (such as, *e.g.,* alectinib, brigatinib, ceritinib, crizotinib, and the like); Bruton's inhibitors (such as, *e.g.,* ibrutinib, and the like); mTOR inhibitors (such as, *e.g.,* everolimus, temsirolimus, and the like); hedgehog inhibitors (such as, *e.g.,* sonidegib, vismodegib, and the like); CDK inhibitors (such as, *e.g.,* palbociclib, ribociclib, and the like); anti-HER1/EGFR monoclonal antibodies (such as, e.g., cetuximab, necitumumab, panitumumab, and the like); anti-HER2/neu monoclonal antibodies (such as, *e.g.,* ado-trastuzumab emtansine, pertuzumab, trastuzumab, trastuzumab-dkst, and the like); anti-EpCAM monoclonal antibodies (such as, e.g., catumaxomab, edrecolomab, and the like); anti-VEGF monoclonal antibodies (such as, e.g., bevacizumab, bevacizumab-awwb, and the like); anti-CD20 monoclonal antibodies (such as, e.g., ibritumomab, obinutuzumab, ocrelizumab, ofatumumab, rituximab, tositumomab, and the like); anti-CD30 monoclonal antibodies (such as, *e.g.,* brentuximab, and the like); anti-CD33 monoclonal antibodies (such as, *e.g.,* gemtuzumab, and the like); and anti-CD52 monoclonal antibodies (such as, *e.g.,* alemtuzumab, and the like).

In one embodiment, the therapeutic or diagnostic payload is a cytotoxic agent.

As used herein, the term **"cytotoxic agent"** refers to any molecule that results in cell death by any mechanism.

Suitable examples of cytotoxic agents include, but are not limited to, anthracyclines, vinca alkaloids, steroids, and chemotherapeutic agents.

Suitable examples of anthracyclines include, but are not limited to, aclarubicin, amrubicin, daunorubicin, doxorubicin, epirubicin, idarubicin, pirarubicin, valrubicin and zorubicin.

Suitable examples of vinca alkaloids include, but are not limited to, vinblastine, vincristine, vinflunine, vindesine and vinorelbine.

Suitable examples of steroids include, but are not limited to, estrogen receptor modulators, androgen receptor modulators and progesterone receptor modulators.

Suitable examples of chemotherapeutic agents have been described hereinabove.

In one embodiment, the therapeutic or diagnostic payload is an antibiotic.

Suitable examples of antibiotics include, but are not limited to:

- aminoglycosides, such as, *e.g.,* amikacin, gentamicin, kanamycin, neomycin, netilmicin, streptomycin, tobramycin, paromycin, and the like;
- ansamycins, such as, *e.g.,* geldanamycin, herbimycin and the like;
- carbacephems, such as, *e.g.,* loracarbef and the like;
- carbapenems, such as, *e.g.,* ertapenum, doripenem, imipenem, cilastatin, meropenem, and the like;
- first generation cephalosporins, such as, *e.g.,* cefadroxil, cefazolin, cefalotin, cephalexin, and the like;
- second generation cephalosporins, such as, *e.g.,* ceflaclor, cefamandole, cefoxitin, cefprozil, cefuroxime, and the like;
- third generation cephalosporins, such as, *e.g.,* cefixime, cefdinir, cefditoren, cefoperazone, cefotaxime, cefpodoxime, ceftazidime, ceftibuten, ceftizoxime, ceftriaxone, and the like;
- fourth generation cephalosporins, such as, *e.g.,* cefepime and the like;
- fifth generation cephalosporins, such as, *e.g.,* ceftobiprole, and the like;
- glycopeptides, such as, *e.g.,* teicoplanin, vancomycin, and the like;
- macrolides, such as, *e.g.,* axithromycin, clarithromycin, dirithromycine, erythromycin, roxithromycin, troleandomycin, telithromycin, spectinomycin, and the like;
- monobactams, such as, *e.g.,* axtreonam, and the like;
- penicilins, such as, *e.g.,* amoxicillin, ampicillin, axlocillin, carbenicillin, cloxacillin, dicloxacillin, flucloxacillin, mezlocillin, meticillin, nafcilin, oxacillin, penicillin, peperacillin, ticarcillin, and the like;
- antibiotic polypeptides, such as, *e.g.,* bacitracin, colistin, polymyxin B, and the like;
- quinolones, such as, *e.g.,* ciprofloxacin, enoxacin, gatifloxacin, levofloxacin, lemefloxacin, moxifloxacin, norfloxacin, orfloxacin, trovafloxacin, and the like;
- sulfonamides, such as, *e.g.,* mafenide, prontosil, sulfacetamide, sulfamethizole, sulfanilamide, sulfasalazine, sulfisoxazole, trimethoprim, trimethoprim- sulfamethoxazole, and the like;
- tetracyclines, such as, *e.g.,* demeclocycline, doxycycline, minocycline, oxytetracycline, tetracycline, and the like; and
- others such as, *e.g.,* arspenamine, chloramphenicol, clindamycin, lincomycin, ethambutol, fosfomycin, fusidic acid, furazolidone, isoniazid, linezolid, metronidazole, mupirocin, nitrofurantoin, platensimycin, pyrazinamide, quinupristin/dalfopristin, rifampin/rifampicin, tinidazole, and the like.

In one embodiment, the therapeutic or diagnostic payload is an antiviral.

Suitable examples of antivirals include, but are not limited to, acemannan, acyclovir, acyclovir sodium, adamantanamine, adefovir, adenine arabinoside, alovudine, alvircept sudotox, amantadine hydrochloride, aranotin, arildone, atevirdine mesylate, avridine, cidofovir, cipamfylline, cytarabine hydrochloride, BMS 806, C31G, carrageenan, zinc salts, cellulose sulfate, cyclodextrins, dapivirine, delavirdine mesylate, desciclovir, dextrin 2-sulfate, didanosine, disoxaril, dolutegravir, edoxudine, enviradene, envirozime, etravirine, famciclovir, famotine hydrochloride, fiacitabine, fialuridine, fosarilate, foscarnet sodium, fosfonet sodium, FTC, ganciclovir, ganciclovir sodium, GSK 1265744, 9-2-hydroxy-ethoxy methylguanine, ibalizumab, idoxuridine, interferon, 5-iodo-2'-deoxyuridine, IQP-0528, kethoxal, lamivudine, lobucavir, maraviroc, memotine pirodavir, penciclovir, raltegravir, ribavirin, rimantadine hydrochloride, rilpivirine (TMC-278), saquinavir mesylate, SCH-C, SCH-D, somantadine hydrochloride, sorivudine, statolon, stavudine, T20, tilorone hydrochloride, TMC120, TMC125, trifluridine, trifluorothymidine, tenofovir, tenofovir alefenamide, tenofovir disoproxyl fumarate, prodrugs of tenofovir, UC-781, UK-427, UK-857, valacyclovir, valacyclovir hydrochloride, vidarabine, vidarabine phosphate, vidarabine sodium phosphate, viroxime, zalcitabene, zidovudine, and zinviroxime.

In one embodiment, the therapeutic or diagnostic payload is a cell cycle-synchronizing agent.

As used herein, the term **"cell cycle-synchronizing agent"** refers to any molecule able to for unify the cell cycle of a population of cells to the same phase upon administration.

Suitable examples of cell cycle-synchronizing agents include, but are not limited to, aphidicolin, butyrolactone I, colchicine, cycloheximide, demecolcine, dimethyl sulfoxide, 5-fluorodeoxyuridine, Hoechst 33342, mimosine, nocodazole, roscovitine, and thymidine.

In one embodiment, the therapeutic or diagnostic payload is a ligand for a cellular receptor.

As used herein, the term **"ligand for a cellular receptor"** refers to any molecule binding to a cellular receptor (such as a cell surface receptor, an intracellular receptor or a co-receptor, including transcription factors and the like), including agonists and antagonists, as well as partial agonists, inverse agonists, and allosteric modulators.

Suitable examples of ligands for cellular receptors include, but are not limited to, ligands binding to the AATYK receptors, the acetylcholine receptors, the ADGRG receptors, the adiponectin receptors, the adrenergic α1 receptors, the adrenergic α2 receptors, the adrenergic β1 receptors, the adrenergic β2 receptors, the adrenergic β3 receptors, the adrenomedullin receptor, the AMPA receptors, the anaphylatoxin receptors, the angiopoietin receptors, the angiotensin receptors, the anti-Mullerian hormone receptor, the apelin receptor, the asialoglycoprotein receptors, the AXL receptors, the benzodiazepine receptor, the bile acid receptor, the bombesin receptors, the bone morphogenetic protein receptors , the bradykinin receptors, the brain-specific angiogenesis inhibitors, the cadherin receptors, the calcitonin receptor, the calcitonin receptor-like receptor, the calcium-sensing receptor, the cannabinoid receptors, the CD97 receptor, the chemokine receptors, the cholecystokinin receptors, the complement receptors, the corticotropin-releasing hormone receptors, the CysLT receptors, the cytokine receptors, the DDR receptors, the dopamine receptors, the EBI2 receptor, the ectodysplasin A receptor, the EGF module-containing mucin-like hormone receptors, the EGF receptors, the endothelin receptors, the EPH receptors, the estrogen receptor, the FGF receptors, the free fatty acid receptors, the frizzled receptors, the FSH receptor, the GABAB receptors, the galanin receptors, the GHB receptor, the ghrelin receptor, the glucagon receptors, the glucagon-like peptide receptors, the glutamate receptors, the glycine receptors, the gonadotropin receptors, the gonadotropin-releasing hormone receptors, the GPRC6A receptor, the growth factor receptors, the growth hormone receptors, the growth-hormone-releasing hormone receptor, the guanylate cyclase-coupled receptors, the HGF receptors, the histamine receptors, the hydroxycarboxylic acids receptors, the immunoglobulin immune receptors, the insulin receptors, the ainite receptors, the KiSSI-derived peptide receptor, the latrophilin receptors, the leptin receptor, the leukotriene B4 receptors, the lipoprotein receptor-related protein receptors, the LTK receptors, the luteinizing hormone/choriogonadotropin receptor, the lysophosphatidic acid receptors, the lysophospholipid receptors, the mannose receptor, the MAS receptors, the melanin-concentrating hormone receptors, the melanocortin receptors, the melatonin receptors, the methuselah-like proteins receptors, the motilin receptor, the MuSK receptors, the N-acetylglucosamine receptor, the neuromedin receptors, the neuropeptide B/W receptors, the neuropeptide FF receptors, the neuropeptide S receptor, the neuropeptide Y receptors, the neuropilins receptor, the neurotensin receptors, the N-formyl peptide receptor, the nicotinic acetylcholine receptors, the NMDA receptors, the nuclear receptors, the olfactory receptor, the opioid receptors, the opsin receptors, the orexin receptors, the oxoeicosanoid receptor, the oxoglutarate receptor, the oxytocin receptor, the parathyroid hormone receptors, the PDGF receptors, the pituitary adenylate cyclase-activating polypeptide type I receptor, the platelet-activating factor receptor, the progestin and adipoQ receptors, the prokineticin receptors, the prolactin receptor, the prolactin-releasing peptide receptor, the prostacyclin receptor, the prostaglandin receptors, the protease-activated receptor, the PTK7 receptors, the purinergic adenosine receptors, the purinergic P2X receptors, the purinergic P2Y receptors, the relaxin receptors, the RET receptors, the retinoic acid-inducible orphan G-protein-coupled receptors, the ROR receptors, the ROS receptors, the RYK receptors, the scavenger receptors, the secretin receptor, the serine/threonine-specific protein kinase receptors, the serotonine receptors, the smoothened receptor, the somatostatin receptors, the sphingosine-1-phosphate receptors, the SREB receptors, the stimulator of interferon genes (STING) receptor, the succinate receptor, the tachykinin receptors, the thromboxane receptor, the thyrotropin receptor, the thyrotropin-releasing hormone receptor, the toll-like receptors, the trace-amine associated receptors, the transferrin receptor, the Trk receptors, the tumor necrosis factor receptors, the tyrosine phosphatase receptors, the urotensin-II receptor, the vasoactive intestinal peptide receptors, the vasoactive intestine peptide receptors, the vasopressin receptors, the VEGF receptors, the vomeronasal receptor, and the zinc-activated ion channel receptor.

In one embodiment, the therapeutic or diagnostic payload is an immunomodulatory agent.

Suitable examples of immunomodulatory agents include, but are not limited to, immunostimulatory agents and immunosuppressor agents.

Suitable examples of immunostimulatory agents include, but are not limited to, cytokines (such as, e.g., filgrastim, pegfilgrastim, lenograstim, molgramostim, sargramostim, ancestim, albinterferon, interferon alfa natural, interferon alfa 2a, peginterferon alfa-2a, interferon alfa 2b, peginterferon alfa-2b, interferon alfa n1, interferon alfacon-1, interferon alpha-n3, interferon beta natural, interferon beta 1a, interferon beta 1b, interferon gamma, aldesleukin, oprelvekin, and the like); immune checkpoint inhibitors (such as, e.g., inhibitors of CTLA4, PD-1, PD-L1, LAG-3, B7-H3, B7-H4, TIM3, A2AR, and/or IDO, including nivolumab, pembrolizumab, pidilizumab, AMP-224, MPDL3280A, MDX-1105, MEDI-4736, arelumab, ipilimumab, tremelimumab, pidilizumab, IMP321, MGA271, BMS-986016, lirilumab, urelumab, PF-05082566, IPH2101, MEDI-6469, CP-870,893, mogamulizumab, varlilumab, avelumab, galiximab, AMP-514, AUNP 12, indoximod, NLG-919, INCB024360, and the like); toll-like receptor agonists (such as, e.g., buprenorphine, carbamazepine, ethanol, fentanyl, GS-9620, imiqimod, lefitolimod, levorphanol, methadone, morphine, (+)-morphine, morphine-3-glucuronide, oxcarbazepine, oxycodone, pethidine, resiquimod, SD-101, tapentadol, tilsotolimod, VTX-2337, glucuronoxylomannan from *Cryptococcus,* MALP-2 from *Mycoplasma,* MALP-404 from *Mycoplasma,* OspA from *Borrelia,* porin from *Neisseria* or *Haemophilus,* hsp60, hemmaglutinin, LcrV from *Yersinia,* bacterial flagellin, lipopolysaccharide, lipoteichoic acid, lipomannan from *Mycobacterium,* glycosylphosphatidylinositol, lysophosphatidylserine, lipophosphoglycan from *Leishmania,* zymosan from *Saccharomyces,* Pam2CGDPKHPKSF, Pam3CSK4, CpG oligodeoxynucleotides, poly(I:C) nucleic acid sequences, poly(A:U) nucleic acid sequences, double-stranded viral RNA, and the like); STING receptor agonists (such as, e.g., those described in WO2017100305, vadimezan, CL656, ADU-S100, 3'3'-cGAMP, 2'3'-cGAMP, ML RR-S2 CDG, ML RR-S2 cGAMP, cyclic di-GMP, DMXAA, DiABZI, and the like); CD1 ligands; growth hormone; immunocyanin; pegademase; prolactin; tasonermin; female sex steroids; histamine dihydrochloride; poly ICLC; vitamin D; lentinan; plerixafor; roquinimex; mifamurtide; glatiramer acetate; thymopentin; thymosin α1; thymulin; polyinosinic:polycytidylic acid; pidotimod; Bacillus Calmette-Guerin; melanoma vaccine; sipuleucel-T; and the like.

Suitable examples of immunosuppressor agents include, but are not limited to:

- antimetabolites, such as, e.g.:

    ▪ antifolates, including aminopterin, methotrexate, pemetrexed, pralatrexate, pteropterin, raltitrexed, denopterin, trimetrexate, pemetrexed, and the like;
    ▪ purine analogues, including pentostatin, cladribine, clofarabine, fludarabine, nelarabine, tioguanine, mercaptopurine, and the like;
    ▪ pyrimidine analogues, including fluorouracil, capecitabine, doxifluridine, tegafur, tegafur/gimeracil/oteracil, carmofur, floxuridine, cytarabine, gemcitabine, azacytidine, decitabine, and the like; and
    ▪ hydroxycarbamide);

- macrolides, such as, e.g., tacrolimus, ciclosporin, pimecrolimus, abetimus, gusperimus, and the like;
- immunomodulatory imide drugs, such as, e.g., lenalidomide, pomalidomide, thalidomide, apremilast, and the like;
- IL-1 receptor antagonists, such as, e.g., anakinra, and the like);
- mTOR inhibitors, such as, e.g., sirolimus, everolimus, ridaforolimus, temsirolimus, umirolimus, zotarolimus, and the like);
- serum-targeting antibodies, such as, e.g., eculizumab, adalimumab, afelimomab, certolizumab pegol, golimumab, infliximab, nerelimomab, mepolizumab, omalizumab, faralimomab, elsilimomab, lebrikizumab, ustekinumab, secukinumab, and the like;
- cell-targeting antibodies, such as, e.g., muromonab-CD3, otelixizumab, teplizumab, visilizumab, clenoliximab, keliximab, zanolimumab, efalizumab, erlizumab, obinutuzumab, rituximab, ocrelizumab, pascolizumab, gomiliximab, lumiliximab, teneliximab, toralizumab, aselizumab, galiximab, gavilimomab, ruplizumab, belimumab, blisibimod, ipilimumab, tremelimumab, bertilimumab, lerdelimumab, metelimumab, natalizumab, tocilizumab, odulimomab, basiliximab, daclizumab, inolimomab, zolimomab aritox, atorolimumab, cedelizumab, fontolizumab, maslimomab, morolimumab, pexelizumab, reslizumab, rovelizumab, siplizumab, talizumab, telimomab aritox, vapaliximab, vepalimomab, and the like;
- fusion antibodies, such as, e.g., abatacept, belatacept, etanercept, pegsunercept, aflibercept, alefacept, rilonacept and the like.

In one embodiment, the therapeutic or diagnostic payload is a pro-apoptotic agent.

As used herein, the term **"pro-apoptotic agent"** refers to any molecule able to induce apoptosis or programmed cell death in a cell upon administration.

Suitable examples of pro-apoptotic agents include, but are not limited to, histone deacetylase inhibitors (such as, *e.g.,* sodium butyrate, depsipeptide and the like), bortezomib, deguelin, favopiridol, fenretinide, fludarabine, kaempferol, miltefosine, narciclasine, obatoclax, oblimersen, and oncrasin.

In one embodiment, the therapeutic or diagnostic payload is an anti-angiogenic agent.

As used herein, the term **"anti-angiogenic agent"** refers to a molecule that reduces or prevents angiogenesis, which is responsible for the growth and development of blood vessels.

Suitable examples of anti-angiogenic agents include, but are not limited to, inhibitors of any of the vascular endothelial growth factor VEGF-A, VEGF-B, VEGF-C, or VEGF-D, which are major inducers of angiogenesis in normal and pathological conditions, and are essential in embryonic vasculogenesis.

Additionally or alternatively, an anti-angiogenic agent also can inhibit other angiogenic factors, such as, without limitation, a member of the fibroblast growth factor (FGF) family such as FGF-1 (acidic), FGF-2 (basic), FGF-4 or FGF-5; or angiopoietin-1, a factor that signals through the endothelial cell-specific Tie2 receptor tyrosine kinase; or the receptor of any of these angiogenic factors.

In one embodiment, the therapeutic or diagnostic payload is a cytokine.

Suitable examples of cytokines include, but are not limited to, chemokines, tumor necrosis factors, interleukins, and colony-stimulating factors.

Suitable examples of chemokines include, but are not limited to, chemokine C-C motif ligand (CCL) 1, CCL2, CCL3, CCL4, CCL5, CCL6, CCL7, CCL8, CCL9, CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, chemokine C-X-C motif ligand (CXCL) 1, CXCL2, CXCL3, CXCL4, CXCL5, CXCL6, CXCL7, CXCL8, CXCL9, CXCL10, CXCL11, CXCL12, CXCL13, CXCL14, CXCL15, CXCL16, CXCL17, fractalkine, chemokine C motif ligand (XCL) 1, and XCL2.

Suitable examples of tumor necrosis factors include, but are not limited to, tumor necrosis factor (TNF) $\alpha$, lymphotoxin, OX40L, CD40LG, Fas ligand, CD70, CD153, 4-1BB ligand, TNF-related apoptosis-inducing ligand (TRAIL), receptor activator of nuclear factor $\kappa$-B ligand (RANKL), a proliferation-inducing ligand (APRIL), B-cell activating factor (BAFF), and ectodysplasin A (EDA).

Suitable examples of interleukins include, but are not limited to, interleukin- (IL-) 1$\alpha$, IL-1$\beta$, IL-1Ra, IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34, IL-35, IL-36$\alpha$, IL-36$\beta$, IL-36$\gamma$, IL-36Ra, IL-37, IL-38, interferon (IFN) $\alpha$, IFN$\beta$, IFN$\kappa$, and IFN$\omega$.

Suitable examples of colony-stimulating factors include, but are not limited to, granulocyte-macrophage colony-stimulating factor (GM-CSF) (including granulocyte-colony stimulating factor (G-CSF) and macrophage colony-stimulating factor (M-CSF)), haematopoietin, and thrombopoietin.

In one embodiment, the therapeutic or diagnostic payload is a growth factor.

Suitable examples of growth factors include, but are not limited to, fibroblast growth factor (FGF) 1, FGF2, FGF3, FGF4, FGF5, FGF6, FGF7, FGF8, FGF9, FGF10, FGF11, FGF12, FGF13, FGF14, FGF16, FGF17, FGF18, FGF19, FGF20, FGF21, FGF23, transforming growth factor (TGF) $\alpha$, epidermal growth factor (EGF), heparin-binding EGF-like growth factor (HB-EGF), transforming growth factor (TGF) $\beta$, insulin-like growth factor (IGF) 1, IGF2, Platelet-derived growth factor (PDGF) subunit A (PDGFA), PDGF subunit B (PDGFB), PDGF subunit C (PDGFC), PDGF subunit D (PDGFD), vascular endothelial growth factor (VEGF)-A, VEGF-B, VEGF-C, VEGF-D, placental growth factor (PGF), nerve growth factor (NGF), and hepatocyte growth factor (HGF).

In one embodiment, the therapeutic or diagnostic payload is an antibody or an antigen-binding fragment thereof.

Suitable examples of antibodies or antigen-binding fragments thereof include, but are not limited to, monoclonal antibodies, polyclonal antibodies, bispecific antibodies, multispecific antibodies, antibody fragments, and antibody mimetics, such as, *e.g.,* scFv, di-scFv, tri-scFv, single domain antibodies, nanobodies, bispecific T-cell engagers (BiTEs), Fab, F(ab')2, Fab', chemically linked Fab, X-Link Fab, tandem-scFv/BiTE, diabodies, tandem diabodies, diabody-Fc fusions, tandem diabody-Fc fusion, tandem diabody-CH3 fusion, tetra scFv-Fc fusion, dual variable domain immunoglobulin, knob-hole, strand exchange engineered domain, CrossMab, quadroma-derived bispecific antibody, single domain based antibody, affibodies, affilins, affimers, affitins, alphabodies, anticalins, avimer, DARPins, Kunitz domain peptides, monobodies and nanoCLAMPs.

In one embodiment, the therapeutic or diagnostic payload is a hormone.

Suitable examples of hormones include, but are not limited to, GnRH, TRH, dopamine, CRH, GHRH, somatostatin, MCH, oxytocin, vasopressin, FSH, LH, TSH, prolactin, POMC, CLIP, ACTH, MSH, endorphins, lipotropin, GH, aldosterone, cortisol, cortisone, DHEA, DHEA-S, androstenedione, epinephrine, norepinephrine, thyroid hormone T3, thyroid hormone T4, calcitonin, PTH, testosterone, AMH, inhibin, estradiol, progesterone, activin, relaxin, GnSAF, hCG, HPL, estrogen, glucagon, insulin, amylin, pancreatic polypeptide, melatonin, *N,N*-dimethyltryptamine, 5-methoxy-*N,N*-dimethyltryptamine, thymosin α1, beta thymosins, thymopoietin, thymulin, gastrin, ghrelin, CCK, GIP, GLP-1, secretin, motilin, VIP, enteroglucagon, peptide YY, IGF-1, IGF-2, leptin, adiponectin, resistin, osteocalcin, renin, EPO, calcitriol, prostaglandin, ANP, and BNP.

In one embodiment, the therapeutic or diagnostic payload is a coding or non-coding oligonucleotide.

Suitable examples of coding or non-coding oligonucleotides include, but are not limited to, messenger RNA (mRNA), antisense RNA (asRNA), small interfering RNA (siRNA), microRNA (miRNA), anti-miRNA (antimiR), long non-coding RNA (lncRNA) (such as, e.g., transfer RNA [tRNA], ribosomal RNA [rRNA], and the like), small temporal RNA (stRNA), trans-acting siRNA, short hairpin RNA (shRNA), cis-natural antisense transcripts (NATs), CRISPR RNA, long noncoding RNA, piwi-interacting RNA (piRNA), repeat-associated siRNA (rasiRNA), RNA aptamers, ribozymes, and the like.

Further suitable examples of coding or non-coding oligonucleotides include, but are not limited to, recapuldencel-T, TriMix, BI-1361849, nusinersen, volanesorsen sodium, eteplirsen, ATL1105, ASM-8, inclisiran, patisiran, RXI-109, fitusiran, cemdisiran, QPI-1002, BMS-986263, PF-655, pegaptanib, avacincaptad pegol sodium, olaptesed pegol, emapticap pegol, SPC3649, bevasiranib, AGN-745, QPI-1007, TD101, SYL040012, SYL1001, Excellair, ALN-RSV01, CEQ508, siG12D LODER, TKM-ApoB, TKM-PLK1, ALN-VSP02, ALN-TTR01, Bcr-Abl siRNA, Atu027, I5NP, CALAA-01, FANG vaccine, iPsiRNA, Tat/Rev shRNA, ARC 1779, ARC 19499, AS1411 (AGRO001), Fovista, NOX-A12, NOX-E36, NOX-H94, NU172, RB006 plus RB007, ARC 1905, as well as those described in Table 1 and Table 2 of Crooke et al., 2018 (Cell Metab. 27(4):714-739), herein incorporated by reference.

In one embodiment, the therapeutic or diagnostic payload is a photodetectable label.

As used herein, the terms **"photodetectable label"** or **"fluorophore"** refer to a molecule that can re-emit light upon light excitation.

Suitable examples of photodetectable labels include, but are not limited to, Alexa Fluor® dyes, BODIPY® dyes, fluorescein, 5-carboxyfluorescein, 5-(4,6-dichlorotriazin-2-yl) aminofluorescein, 2'7'-dimethoxy-4'5'-dichloro-6-carboxyfluorescein, fluorescein isothiocyanate (FITC), QFITC, Oregon Green® 488, Oregon Green® 514, rhodamine and derivatives thereof (such as, *e.g.,* rhodamine green, rhodamine green-X, rhodamine red-X, X-rhodamine, 6-carboxy-X-rhodamine (ROX), 6-carboxyrhodamine (R6G), *N,N,N',N'*-tetramethyl-6-carboxyrhodamine (TAMRA), lissamine rhodamine B, rhodamine 123, rhodamine X isothiocyanate, sulforhodamine B, sulforhodamine 101 (Texas Red), tetramethyl rhodamine, tetramethyl rhodamine isothiocyanate (TRITC)), eosin, eosin isothiocyanate, erythrosine, erythrosine B, erythrosin isothiocyanate, Texas Red®, Texas Red®-X, naphthofluorescein, malachite green, malachite green isothiocyanate, coumarin derivatives, Pacific Orange, cascade blue, cascade yellow, dansyl chloride, dapoxyl dye, 1-dimethylamine-*N*(2-azido-ethyl)naphthalene-5-sulfonamide, 6-(6-amino-2-(2-azidoethyl)-1,3-dioxo-1*H*-benzo(*de*)-2(3*H*)isoquinoline, 6-(6-amino-2-(2-propinyl)-1,3-dioxo-1*H*-benzo(*de*)-2(3*H*)isoquinoline, 8-(4-azidoethyloxyphenyl)-2,6-diethyl-1,3,5,7-tetramethyl-4,4-difluoro-4-bora-3a,4a-diaza-*s*-indacene, 8-(4-propynyioxyphenyl)-2,6-diethyl-1,3,5,7-tetramethyl-4,4-difluoro-4-bora-3a,4a-diaza-*s*-indacene, 1-(3-azido-propoxy)-7-methylamino-phenoxazin-3-one, 1-(2-propynyl)-7-methylamino-phenoxazm-3-one, *N*-(5-(3-azidopropylamino)-9*H*-benzo(*a*)-phenoxa-2-in-9-ylidene)-*N*-methyl-methanaminium chloride, *N*-(5-(3-propynyl-amino)-9*H*-benzo(*a*)-phenoxazin-9-ylene)-*N*-methyl-methanaminium chloride, (9-(3-azido-propoxy)-7-piperidin-1-yl-phenoxazin-3-ylidene)-dimethylammonium perchlorate, 4-acetamido-4'-isothiocyanatostilbene-2,2'-disulfonic acid, acridine, acridine isothiocyanate, 5-(2'-aminoethyl)aminonaphthalene-1-sulfonic acid, 4-amino-*N*-[3-vinylsulfonyl)phenyl]naphthalimide-3,5 disulfonate, *N*-(4-anilino-1-naphthyl)maleimide, anthranilamide, Brilliant Yellow, coumarin, coumarin derivatives, 7-amino-4-methylcoumarin, 7-amino-trifluoromethylcoiluarin, cyanosine, 4',6-diaminidino-2-phenylindole, 5',5"-dibromopyrogallol-sulfonephthalein, 7-diethylamino-3-(4'-isothiocyanatophenyl)-4-methylcoumarin-4,4'-diisothiocyanatodihydro-stilbene-2,2'-disulfonic acid, 4,4'-diisothiocyanatostilbene-2,2'-disulfonic acid, ethidium, IR144, IR1446, 4-methylumbelliferone, o-cresolphthalein, nitrotyrosine, pararosaniline, Phenol Red, B-phycoerythrin, o-phthaldialdehyde, pyrene, pyrene butyrate, succinimidyl 1-pyrene butyrate, Reactive Red 4, riboflavin, rosolic acid, lanthanide chelates, quantum dots, cyanines, pyrelium dyes, and squaraines.

In one embodiment, the therapeutic or diagnostic payload is a contrast agent.

As used herein, the term **"contrast agent"** refers to any molecule used to increase the contrast of structures or fluids within the body in medical imaging. Contrast agents absorb or alter external electromagnetism or ultrasound (which differs from radiolabels which emit radiation themselves).

Suitable examples of contrast agents include, but are not limited to, diatrizoic acid, metrizoic acid, iodamide, iotalamic acid, ioxitalamic acid, ioglicic acid, acetrizoic acid, iocarmic acid, methiodal, diodone, metrizamide, iohexol, ioxaglic acid, iopamidol, iopromide, iotrolan, ioversol, iopentol, iodixanol, iomeprol, iobitridol, ioxilan, iodoxamic acid, iotroxic acid, ioglycamic acid, adipiodone, iobenzamic acid, iopanoic acid, iocetamic acid, sodium iopodate, tyropanoic acid, calcium iopodate, iopydol, propyliodone, iofendylate, lipiodol, barium sulfate, gadobenic acid, gadobutrol, gadodiamide, gadofosveset, gadolinium, gadopentetic acid, gadoteric acid, gadoteridol, gadoversetamide, gadoxetic acid, ferric ammonium citrate, mangafodipir, ferumoxsil, ferristene, perflubron, microspheres of human albumin, microparticles of galactose, perflenapent, microspheres of phospholipids, sulfur hexafluoride, and the like.

In one embodiment, the therapeutic or diagnostic payload is a radiolabel.

As used herein, the terms **"radiolabel"** or **"radiopharmaceutical"** refer to any molecule which emits radiation. Radiolabels can be used for therapeutics or diagnostic purposes.

Suitable examples of radiolabels include, but are not limited to, $^{99m}$Tc compounds (such as, e.g., exametazime, medronic acid, macroaggregated albumin, sestamibi, tetrofosmin, exametazime, sulesomab, tilmanocept, arcitumomab, votumumab, hynic-octreotide, and the like); $^{123}$I, $^{125}$I or $^{131}$I compounds (such as, e.g., ioflupane, iofetamine, iomazenil, sodium iodohippurate, iobenguane, iodocholesterol, minretumomab, tositumomab, and the like); $^{18}$F compounds (such as, e.g., florbetapir, flutemetamol, fluciclovine, fludeoxyglucose, fluoroethyltyrosine, sodium fluoride, and the like); $^{64}$Cu compounds (such as, e.g., Cu-ETS2, and the like); $^{75}$Se compounds (such as, e.g., SeHCAT); $^{111}$In compounds (such as, e.g., imciromab, capromab pendetide, satumomab pendetide, and the like); $^{82}$Rb compounds (such as, e.g., rubidium chloride); $^{153}$Sm compounds (such as, e.g., lexidronam, and the like); $^{89}$Sr compounds (such as, e.g., strontium-89 chloride, and the like); $^{90}$Y compounds (such as, e.g., ibritumomab tiuxetan, and the like); $^{223}$Ra compounds (such as, e.g., radium-223 chloride, and the like); $^{177}$Lu compounds (such as, e.g., oxodotreotide, and the like); and any compounds comprising at least one $^{2}$H, $^{3}$H, $^{11}$C, $^{13}$N, $^{14}$C, $^{15}$O, $^{18}$F, $^{22}$Na, $^{24}$Na, $^{32}$P, $^{47}$Ca, $^{51}$Cr, $^{57}$Co, $^{58}$Co, $^{59}$Fe, $^{64}$Cu, $^{67}$Ga, $^{68}$Ga, $^{75}$Se, $^{81m}$Kr, $^{82}$Rb, $^{89}$Sr, $^{90}$Y, $^{99m}$Tc, $^{111}$In, $^{123}$I, $^{125}$I, $^{131}$I, $^{133}$Xe, $^{153}$Sm, $^{165}$Dy, $^{169}$Er, $^{177}$Lu, $^{186}$Re, $^{198}$Au, $^{201}$Tl and/or $^{223}$Ra atom.

Additionally or alternatively, the payload may be a carrier, i.e., a fused moiety which role is to improve the pharmacokinetics and/or biodistribution of the peptide according to the present invention. Such conjugates are sometimes referred to as "biobetters". For a review, see, e.g., Strohl, 2015. BioDrugs. 29(4):215-239.

In one embodiment, the carrier is chosen in the group comprising or consisting of a Fc domain of an immunoglobulin, transferrin or a domain thereof, albumin or a domain thereof, an XTEN sequence, a homo-amino acid polymer (HAP), a proline-alanine-serine polymer (PAS), an elastin-like peptide (ELP), polyethylene glycol, and hyaluronic acid.

In one embodiment, the carrier is chosen in the group comprising or consisting of a Fc domain of an immunoglobulin, transferrin or a domain thereof, and albumin or a domain thereof.

In one embodiment, the carrier is a domain of human serum albumin, such as, e.g., domain III of human serum albumin. Domain III of human serum albumin comprises or consists of amino acid residues 404 to 601 of human serum albumin (Uniprot accession number and version: P02768-1, last modified on April 1, 1990; Checksum: F88FF61DD242E818).

In one embodiment where the carrier is a Fc domain of an immunoglobulin, the peptide conjugate does not mediate, trigger or enhance antibody-dependent cellular cytotoxicity (ADCC), antibody-dependent cellular phagocytosis (ADCP) and/or complement-dependent cytotoxicity (CDC).

In one embodiment, the carrier is a fatty acid that binds to human serum albumin.

In one embodiment, the carrier is an adsorption enhancer. In one embodiment, the carrier increases transcellular stomach adsorption. In one embodiment, the carrier comprises or consists of sodium N-[8-(2-hydroxybenzoyl)amino]caprylate.

The specific binding between the peptides according to the present invention and CD38 implies that said peptides exhibit appreciable affinity for CD38.

The affinity of the peptides according to the present invention for CD38 can be determined by various methods well known from the one skilled in the art. These methods can be qualitative or semi-quantitative (such as, e.g., co-immunoprecipitation, pull-down assay, and the like), or can be quantitative (such as, e.g., isothermal titration calorimetry, surface plasmon resonance, biolayer interferometry, and the like).

Alternatively or additionally, whether the peptides according to the present invention bind to CD38 can be tested readily by, inter alia, comparing the reaction of said peptides with CD38 or a fragment thereof (in particular, a fragment comprising or consisting of the binding site) with the reaction of said peptides with proteins or antigens other than CD38 or a fragment thereof.

In one embodiment, the peptides according to the present invention bind specifically to CD38 with a dissociation constant ($K_d$) lower than about $1\times10^{-6}$ M, such as, lower than about $9\times10^{-7}$ M, $8\times10^{-7}$ M, $7\times10^{-7}$ M, $6\times10^{-7}$ M, $5\times10^{-7}$ M, $4\times10^{-7}$ M, $3\times10^{-7}$ M, $2\times10^{-7}$ M, $1\times10^{-7}$ M, $9\times10^{-8}$ M, $8\times10^{-8}$ M, $7\times10^{-8}$ M, $6\times10^{-8}$ M, $5\times10^{-8}$ M, $4\times10^{-8}$ M, $3\times10^{-8}$ M, $2\times10^{-8}$ M, $1\times10^{-8}$ M, $9\times10^{-9}$ M, $8\times10^{-9}$ M, $7\times10^{-9}$ M, $6\times10^{-9}$ M, $5\times10^{-9}$ M, $4\times10^{-9}$ M, $3\times10^{-9}$ M, $2\times10^{-9}$ M, $1\times10^{-9}$ M, $9\times10^{-10}$ M, $8\times10^{-10}$ M, $7\times10^{-10}$ M, $6\times10^{-10}$ M, $5\times10^{-10}$ M, $4\times10^{-10}$ M, $3\times10^{-10}$ M, $2\times10^{-10}$ M, $1\times10^{-10}$ M or less.

In one embodiment, the peptides according to the present invention specifically bind to at least one binding site within CD38.

In one embodiment, the compound according to the present invention specifically binds to at least one binding site within human CD38.

In one embodiment, said binding site is linear. In one embodiment, said binding site is conformational.

In one embodiment, said binding site comprises 71, 70, 69, 68, 67, 66, 65, 64, 63, 62, 61, 60, 59, 58, 57, 56, 55, 54, 53, 52, 51, 50, 49, 48, 47, 46, 45, 44, 43, 42, 41, 40, 39, 38, 37, 36, 35, 34, 33, 32, 31, 30, 29, 28, 27, 26, 25, 24, 23, 22, 21, 20, 19, 18, 17, 16, 15, 14, 13, 12, 11, 10, 9, 8, 7, 6, 5, 4, 3, 2 contiguous or non-contiguous amino acid residues.

In one embodiment, said binding site extends at least throughout amino acid residue 1 to amino acid residue 300 of human CD38 having SEQ ID NO: 3, or throughout the corresponding amino acid residues in homologous CD38 from other species.

In one embodiment, said binding site extends at least throughout amino acid residue 1 to amino acid residue 296 of human CD38 having SEQ ID NO: 3, or throughout the corresponding amino acid residues in homologous CD38 from other species.

In one embodiment, said binding site extends at least throughout amino acid residue 1 to amino acid residue 294 of human CD38 having SEQ ID NO: 3, or throughout the corresponding amino acid residues in homologous CD38 from other species.

In one embodiment, said binding site extends at least throughout amino acid residue 1 to amino acid residue 285 of human CD38 having SEQ ID NO: 3, or throughout the corresponding amino acid residues in homologous CD38 from other species.

In one embodiment, said binding site extends at least throughout amino acid residue 70 to amino acid residue 300 of human CD38 having SEQ ID NO: 3, or throughout the corresponding amino acid residues in homologous CD38 from other species.

In one embodiment, said binding site extends at least throughout amino acid residue 70 to amino acid residue 296 of human CD38 having SEQ ID NO: 3, or throughout the corresponding amino acid residues in homologous CD38 from other species.

In one embodiment, said binding site extends at least throughout amino acid residue 70 to amino acid residue 294 of human CD38 having SEQ ID NO: 3, or throughout the corresponding amino acid residues in homologous CD38 from other species.

In one embodiment, said binding site extends at least throughout amino acid residue 70 to amino acid residue 285 of human CD38 having SEQ ID NO: 3, or throughout the corresponding amino acid residues in homologous CD38 from other species.

In one embodiment, said binding site extends at least throughout amino acid residue 189 to amino acid residue 300 of human CD38 having SEQ ID NO: 3, or throughout the corresponding amino acid residues in homologous CD38 from other species.

In one embodiment, said binding site extends at least throughout amino acid residue 189 to amino acid residue 296 of human CD38 having SEQ ID NO: 3, or throughout the corresponding amino acid residues in homologous CD38 from other species.

In one embodiment, said binding site extends at least throughout amino acid residue 189 to amino acid residue 294 of human CD38 having SEQ ID NO: 3, or throughout the corresponding amino acid residues in homologous CD38 from other species.

In one embodiment, said binding site extends at least throughout amino acid residue 189 to amino acid residue 285 of human CD38 having SEQ ID NO: 3, or throughout the corresponding amino acid residues in homologous CD38 from other species.

In one embodiment, said binding site extends at least throughout amino acid residue 219 to amino acid residue 300 of human CD38 having SEQ ID NO: 3, or throughout the corresponding amino acid residues in homologous CD38 from other species.

In one embodiment, said binding site extends at least throughout amino acid residue 219 to amino acid residue 296 of human CD38 having SEQ ID NO: 3, or throughout the corresponding amino acid residues in homologous CD38 from other species.

In one embodiment, said binding site extends at least throughout amino acid residue 219 to amino acid residue 294 of human CD38 having SEQ ID NO: 3, or throughout the corresponding amino acid residues in homologous CD38 from other species.

In one embodiment, said binding site extends at least throughout amino acid residue 219 to amino acid residue 285 of human CD38 having SEQ ID NO: 3, or throughout the corresponding amino acid residues in homologous CD38 from other species.

In one embodiment, said binding site extends at least throughout amino acid residue 220 to amino acid residue 300 of human CD38 having SEQ ID NO: 3, or throughout the corresponding amino acid residues in homologous CD38 from other species.

In one embodiment, said binding site extends at least throughout amino acid residue 220 to amino acid residue 296 of human CD38 having SEQ ID NO: 3, or throughout the corresponding amino acid residues in homologous CD38 from other species.

In one embodiment, said binding site extends at least throughout amino acid residue 220 to amino acid residue 294 of human CD38 having SEQ ID NO: 3, or throughout the corresponding amino acid residues in homologous CD38 from other species.

In one embodiment, said binding site extends at least throughout amino acid residue 220 to amino acid residue 285 of human CD38 having SEQ ID NO: 3, or throughout the corresponding amino acid residues in homologous CD38 from other species.

In one embodiment, said binding site extends at least throughout amino acid residue 233 to amino acid residue 300 of human CD38 having SEQ ID NO: 3, or throughout the corresponding amino acid residues in homologous CD38 from other species.

In one embodiment, said binding site extends at least throughout amino acid residue 233 to amino acid residue 296 of human CD38 having SEQ ID NO: 3, or throughout the corresponding amino acid residues in homologous CD38 from other species.

In one embodiment, said binding site extends at least throughout amino acid residue 233 to amino acid residue 294 of human CD38 having SEQ ID NO: 3, or throughout the corresponding amino acid residues in homologous CD38 from other species.

In one embodiment, said binding site extends at least throughout amino acid residue 233 to amino acid residue 285 of human CD38 having SEQ ID NO: 3, or throughout the corresponding amino acid residues in homologous CD38 from other species.

In one embodiment, said binding site extends at least throughout amino acid residue 220 to amino acid residue 241 and amino acid residue 273 to amino acid residue 285 of human CD38 having SEQ ID NO: 3, or throughout the corresponding amino acid residues in homologous CD38 from other species.

In one embodiment, said binding site comprises at least cysteine 254 of human CD38 having SEQ ID NO: 3 or the corresponding cysteine in homologous CD38 from other species.

In one embodiment, said binding site comprises at least cysteine 275 of human CD38 having SEQ ID NO: 3 or the corresponding cysteine in homologous CD38 from other species.

In one embodiment, said binding site comprises at least cysteine 254 and cysteine 275 of human CD38 having SEQ ID NO: 3 or the corresponding cysteine in homologous CD38 from other species.

In one embodiment, said binding site comprises the 5th (penultimate) C-terminal disulfide loop involving cysteine 254 and cysteine 275 of human CD38 having SEQ ID NO: 3 or the corresponding cysteine in homologous CD38 from other species. In particular, the 5th (penultimate) C-terminal disulfide loop of human CD38 comprises amino acid residues 220 to amino acid residue 285 of human CD38 having SEQ ID NO: 3, or the corresponding cysteine in homologous CD38 from other species.

In one embodiment, said binding site does not comprise cysteine 287 of human CD38 having SEQ ID NO: 3 or the corresponding cysteine in homologous CD38 from other species.

In one embodiment, said binding site does not comprise cysteine 290 of human CD38 having SEQ ID NO: 3 or the corresponding cysteine in homologous CD38 from other species.

In one embodiment, said binding site does not comprise cysteine 287 and cysteine 290 of human CD38 having SEQ ID NO: 3 or the corresponding cysteine in homologous CD38 from other species.

In one embodiment, said binding site does not comprise the 6th C-terminal disulfide loop involving cysteine 287 and cysteine 296 of human CD38 having SEQ ID NO: 3 or the corresponding cysteine in homologous CD38 from other species. In particular, the 6th C-terminal disulfide loop of human CD38 comprises amino acid residues 285 to amino acid residue 300 of human CD38 having SEQ ID NO: 3, or the corresponding cysteine in homologous CD38 from other species.

In one embodiment, the peptides according to the present invention directly protect dopaminergic neurons from mitochondrial inhibition induced by the neurotoxin MPP$^+$.

In one embodiment, the peptides according to the present invention have at least one, preferably at least two, more preferably the three following properties:

- they protect dopaminergic neurons against energy deficit due to mitochondrial complex I inhibition, in particular against the mitochondrial neurotoxin MPP$^+$;
- they increase the release of the anti-inflammatory cytokine interleukin-10 (IL-10) *in vitro* using human peripheral blood mononuclear cells (PBMC); and/or
- they increase IL-10 levels *in vivo* when injected to mice.

In one embodiment, the peptides according to the present invention compete with endogenous CD31 for CD38 binding, preferably with endogenous human CD31, and induce tyrosine phosphorylation. In particular, the peptides according to the present invention induce tyrosine phosphorylation of discrete cytoplasmic substrates that is inhibited in the presence of genistein.

Examples of discrete cytoplasmic substrates phosphorylated include, but are not limited to, the *c-cbl* proto-oncogene, the protein kinase *syk,* the p85 subunit of phosphatidylinositol-3 kinase, phospholipase C-$\gamma$ (PLC-$\gamma$), the Raf-1/MAP kinase, the CD3-$\zeta$/ZAP-70 signaling pathway.

In one embodiment, the peptides according to the present invention compete with endogenous CD31 for CD38 binding, preferably with endogenous human CD31, and trigger CD38 internalization.

In one embodiment, the peptides according to the present invention compete with endogenous CD31 for CD38 binding, preferably with endogenous human CD31, and induce lysosomal exocytosis. In particular, the peptides according to the present invention induce lysosomal exocytosis that is inhibited in the presence of vacuolin-1.

In one embodiment, the peptides according to the present invention compete with endogenous CD31 for CD38 binding, preferably with endogenous human CD31, and increase glucose uptake.

In one embodiment, the peptides according to the present invention comprise or consist of less than 50 amino acid residues, preferably less than 45, 40, 35, 30 or 25 amino acid residues.

In one embodiment, the peptides according to the present invention comprise or consist of less than 50 contiguous amino acid residues from human sCD31 having SEQ ID NO: 2, preferably less than 45, 40, 35, 30 or 25 contiguous amino acid residues from human sCD31 having SEQ ID NO: 2.

In one embodiment, the peptides according to the present invention comprise or consist of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 amino acid residues.

In one embodiment, the peptides according to the present invention comprise or consist of 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 contiguous amino acid residues from human sCD31 having SEQ ID NO: 2.

In one embodiment, the peptides according to the present invention comprise or consist of 7, 9, 15, 17, 20 or 22 amino acid residues.

In one embodiment, the peptides according to the present invention are not antibodies or antigen-binding fragment thereof.

The present invention also relates to a composition comprising, consisting of, or consisting essentially of, at least one peptide according to the present invention, which specifically binds to CD38.

The present invention also relates to a pharmaceutical composition comprising at least one peptide according to the present invention, which specifically binds to CD38; and at least one pharmaceutically acceptable excipient.

The term **"pharmaceutically acceptable excipient"** includes any and all solvents, dispersion media, coatings, antibacterial and antifungal agents, isotonic and absorption delaying agents and the like. Said excipient does not produce an adverse, allergic or other untoward reaction when administered to an animal, preferably a human. For human administration, preparations should meet sterility, pyrogenicity, and general safety and purity standards as required by regulatory offices, such as, *e.g.,* FDA Office or EMA.

Pharmaceutically acceptable excipients that may be used in these pharmaceutical compositions include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances (e.g., sodium carboxymethylcellulose), polyethylene glycol, polyacrylates, waxes, polyethylene- polyoxypropylene- block polymers, polyethylene glycol and wool fat.

In one embodiment, the pharmaceutical compositions according to the present invention comprise vehicles which are pharmaceutically acceptable for a formulation capable of being injected to a subject. These may be in particular isotonic, sterile, saline solutions (monosodium or disodium phosphate, sodium, potassium, calcium or magnesium chloride and the like or mixtures of such salts), or dry, especially freeze-dried compositions which upon addition, depending on the case, of sterilized water or physiological saline, permit the constitution of injectable solutions.

The present invention also relates to a medicament comprising, consisting of, or consisting essentially of, at least one peptide according to the present invention, which specifically binds to CD38.

The present invention also relates to methods of preventing and/or treating a disease in a subject in need thereof, comprising administering to said subject a peptide according to the present invention, or the composition, the pharmaceutical composition or the medicament according to the present invention.

The present invention also relates to a peptide according to the present invention, or to the composition, the pharmaceutical composition or the medicament according to the present invention, for use as a drug.

The present invention also relates to a peptide according to the present invention, or to the composition, the pharmaceutical composition or the medicament according to the present invention, for use in preventing and/or treating a disease in a subject in need thereof.

In one embodiment, the disease is related to, due to or caused by increased soluble CD38 levels in the subject. In particular, increased soluble CD38 levels may be detected in a blood sample (including whole blood, plasma and serum, preferably in a plasma sample), in a cerebrospinal fluid sample and/or in a muscle biopsy.

In one embodiment, the disease is selected from the group comprising, or consisting of: inflammatory diseases; autoimmune diseases; metabolic and endocrine diseases; neurodegenerative diseases; neuroinflammatory diseases;

ocular diseases; age-related diseases and aging; and cancer and metastasis.

In one embodiment, the disease is selected from the group comprising, or consisting of, a metabolic and endocrine disease, a neurodegenerative disease, a neuroinflammatory disease, an inflammatory disease, an autoimmune disease, and a combination thereof. In one embodiment, the disease is selected from the group comprising, or consisting of, a neurodegenerative disease, a neuroinflammatory disease, an inflammatory disease, an autoimmune disease, a metabolic and endocrine disease, and a combination thereof. In one embodiment, the disease is selected from the group comprising, or consisting of, an inflammatory disease, an autoimmune disease, a metabolic and endocrine disease, and a combination thereof. In one embodiment, the disease is selected from the group comprising, or consisting of, an autoimmune disease, a metabolic and endocrine disease, and a combination thereof.

It is to be understood that the present disclosure also encompasses symptoms of the recited diseases.

In one embodiment, the disease is an inflammatory disease.

Examples of inflammatory diseases include, but are not limited to, arthritis (including, e.g., osteoarthritis, rheumatoid arthritis, spondyloarthropathies and psoriatic arthritis); asthma (including, *e.g.,* atopic asthma, nonatopic asthma, allergic asthma, exercise-induced asthma, drug-induced asthma, occupational asthma and late stage asthma); inflammatory bowel disease (including, *e.g.,* Crohn's disease, ulcerative colitis and colitis); inflammatory skin disorders (including, *e.g.,* psoriasis, atopic dermatitis and contact hypersensitivity); multiple sclerosis; osteoporosis; tendonitis; allergic disorders (including, *e.g.,* rhinitis, conjunctivitis and urticaria); inflammation in response to an insult to the host (including, *e.g.,* injury or infection); transplant rejection; graft versus host disease; sepsis; and systematic lupus erythematosus.

In one embodiment, the disease is an autoimmune disease.

Examples of autoimmune diseases include, but are not limited to, alopecia areata; ankylosing spondylitis; arthritis; antiphospholipid syndrome; autoimmune Addison's disease; autoimmune hemolytic anemia; autoimmune inner ear disease (also known as Ménière's disease); autoimmune lymphoproliferative syndrome; autoimmune thrombocytopenic purpura; autoimmune hemolytic anemia; autoimmune hepatitis; Bechet's disease; Crohn's disease; diabetes mellitus type 1; glomerulonephritis; Graves' disease; Guillain-Barre syndrome; inflammatory bowel disease; lupus nephritis; multiple sclerosis; myasthenia gravis; pemphigus; pernicous anemia; polyarteritis nodosa; polymyositis; primary biliary cirrhosis; psoriasis; Raynaud's phenomenon; rheumatic fever; rheumatoid arthritis; scleroderma; Sjögren's syndrome; systemic lupus erythematosus; ulcerative colitis; vitiligo; and Wegener's granulomatosis.

In one embodiment, the disease is a metabolic and endocrine disease.

Examples of metabolic and endocrine diseases include, but are not limited to, diabetes (*e.g.,* type 1 diabetes, type 2 diabetes, gestational diabetes); hyperglycemia; insulin resistance; impaired glucose tolerance; hyperinsulinism; diabetic complication; dyslipidemia; hypercholesterolemia; hypertriglyceridemia; HDL hypocholesterolemia; LDL hypercholesterolemia and/or HLD non-cholesterolemia; VLDL hyperproteinemia; dyslipoproteinemia; apolipoprotein A-1 hypoproteinemia; metabolic syndrome; syndrome X; obesity; non-alcoholic fatty liver disease (NAFLD); non-alcoholic steatohepatitis; and adrenal leukodystrophy.

In one embodiment, the disease is a neurodegenerative disease.

Examples of neurodegenerative diseases include, but are not limited to, Parkinson's disease and related disorders (including, *e.g.,* Parkinson's disease, Parkinson-dementia, autosomal recessive PARK2 and PARK6-linked Parkinsonism, atypical parkinsonian syndromes, including, progressive supranuclear palsy, corticobasal degeneration syndrome, Lewy bodies dementia, multiple system atrophy, Guadeloupean Parkinsonism and Lytigo-bodig disease); motor neuron diseases (including, *e.g.,* amyotrophic lateral sclerosis, frontotemporal dementia, progressive bulbar palsy, pseudobulbar palsy, primary lateral sclerosis, progressive muscular atrophy, spinal muscular atrophy and post-polio syndrome); neuro-inflammatory diseases; Alzheimer's disease and related disorders (including, *e.g.,* early stage of an Alzheimer's disorder, mild stage of an Alzheimer's disorder, moderate stage of an Alzheimer's disorder, mild to moderate stage of an Alzheimer's disorder, advanced stage of an Alzheimer's disorder, mild cognitive impairment, vascular dementia, mixed dementia, Pick's disease, argyrophilic grain disease, posterior cortical atrophy, and Wernicke-Korsakoff syndrome); prion diseases; lysosomal storage diseases; leukodystrophies; Huntington's disease; multiple sclerosis; Down syndrome; spinal and bulbar muscular atrophy; HIV-associated neurocognitive disorder; Tourette syndrome; autosomal dominant spinocerebellar ataxia; Friedreich's ataxia; dentatorubral pallidoluysian atrophy; myotonic dystrophy; schizophrenia; age associated memory impairment; autism and autism spectrum disorders; attention-deficit hyperactivity disorder; chronic pain; alcohol-induced dementia; progressive non-fluent aphasia; semantic dementia; spastic paraplegia; fibromyalgia; post-Lyme disease; neuropathies; withdrawal symptoms; Alpers' disease; cerebro-oculo-facio-skeletal syndrome; Wilson's disease; Cockayne syndrome; Leigh's disease; neurodegeneration with brain iron accumulation; dyskinesia; dystonia (including, *e.g.,* status dystonicus, spasmodic torticollis, Meige's syndrome, and blepharospasm); athetosis; chorea; choreoathetosis; opsoclonus myoclonus syndrome; myoclonus; myoclonic epilepsy; akathisia; tremor (including, *e.g.,* essential tremor, and intention tremor); restless legs syndrome; stiff-person syndrome; alpha-methylacyl-CoA racemase deficiency; Andermann syndrome; Arts syndrome; Marinesco-Sjögren syndrome; mitochondrial membrane protein-associated neurodegeneration; pantothenate kinase-associated neurodegeneration; polycystic lipomembranous osteodysplasia with sclerosing leukoencephalopathy; riboflavin transporter deficiency neuronopathy; and ataxia telangiectasia.

In one embodiment, the disease is a neuroinflammatory disease.

Examples of neuroinflammatory diseases include, but are not limited to, multiple sclerosis; encephalitis (including, e.g., viral encephalitis, herpesviral encephalitis, limbic encephalitis, and encephalitis lethargica); myelitis (including, *e.g.,* poliomyelitis, acute disseminated encephalomyelitis, transverse myelitis, myalgic encephalomyelitis, and neuromyelitis optica); meningoencephalitis; optic neuritis; tropical spastic paraparesis; cavernous sinus thrombosis; brain abscess; epidural abscess; and the like. In some instances, primary conditions with secondary neuroinflammation (*e.g.,* traumatic brain injury with secondary neuroinflammation) may also be considered a neuroinflammatory disease.

In one embodiment, the disease is cancer.

Examples of cancers include those listed in the 10th revision of the International Statistical Classification of Diseases and Related Health Problems (ICD), under chapter II, blocks C00 to D48.

Further examples of cancers include, but are not limited to, recurrent, metastatic or multi-drug resistant cancer.

Further examples of cancers include, but are not limited to, adenofibroma, adenoma, agnogenic myeloid metaplasia, AIDS-related malignancies, ameloblastoma, anal cancer, angiofollicular mediastinal lymph node hyperplasia, angiokeratoma, angiolymphoid hyperplasia with eosinophilia, angiomatosis, anhidrotic ectodermal dysplasia, anterofacial dysplasia, apocrine metaplasia, apudoma, asphyxiating thoracic dysplasia, astrocytoma (including, *e.g.,* cerebellar astrocytoma and cerebral astrocytoma), atriodigital dysplasia, atypical melanocytic hyperplasia, atypical metaplasia, autoparenchymatous metaplasia, basal cell hyperplasia, benign giant lymph node hyperplasia, bile duct cancer (including, e.g., extrahepatic bile duct cancer), bladder cancer, bone cancer, brain tumor (including, *e.g.,* brain stem glioma, cerebellar astrocytoma glioma, malignant glioma, supratentorial primitive neuroectodermal tumors, visual pathway and hypothalamic glioma, ependymoma, medulloblastoma, gestational trophoblastic tumor glioma, and paraganglioma), branchionia, female breast cancer, male breast cancer, bronchial adenomas/carcinoids, bronchopulmonary dysplasia, cancer growths of epithelial cells, pre-cancerous growths of epithelial cells, metastatic growths of epithelial cells, carcinoid heart disease, carcinoid tumor (including, *e.g.,* gastrointestinal carcinoid tumor), carcinoma (including, *e.g.,* carcinoma of unknown primary origin, adrenocortical carcinoma, islet cells carcinoma, adeno carcinoma, adeoncortical carcinoma, basal cell carcinoma, basosquamous carcinoma, bronchiolar carcinoma, Brown-Pearce carcinoma, cystadenocarcinoma, ductal carcinoma, hepatocarcinoma, Krebs carcinoma, papillary carcinoma, oat cell carcinoma, small cell lung carcinoma, non-small cell lung carcinoma, squamous cell carcinoma, transitional cell carcinoma, Walker carcinoma, Merkel cell carcinoma, and skin carcinoma), cementoma, cementum hyperplasia, cerebral dysplasia, cervical cancer, cervical dysplasia, cholangioma, cholesteatoma, chondroblastoma, chondroectodermal dysplasia, chordoma, choristoma, chrondroma, cleidocranial dysplasia, colon cancer, colorectal cancer, local metastasized colorectal cancer, congenital adrenal hyperplasia, congenital ectodermal dysplasia, congenital sebaceous hyperplasia, connective tissue metaplasia, craniocarpotarsal dysplasia, craniodiaphysial dysplasia, craniometaphysial dysplasia, craniopharyngioma, cylindroma, cystadenoma, cystic hyperplasia (including, e.g., cystic hyperplasia of the breast), cystosarconia phyllodes, dentin dysplasia, denture hyperplasia, diaphysial dysplasia, ductal hyperplasia, dysgenninoma, dysplasia epiphysialis hemimelia, dysplasia epiphysialis multiplex, dysplasia epiphysialis punctate, ectodermal dysplasia, Ehrlich tumor, enamel dysplasia, encephaloophthalmic dysplasia, endometrial cancer (including, *e.g.,* ependymoma and endometrial hyperplasia), ependymoma, epithelial cancer, epithelial dysplasia, epithelial metaplasia, esophageal cancer, Ewing's family of tumors (including, *e.g.,* Ewing's sarcoma), extrahepatic bile duct cancer, eye cancer (including, *e.g.,* intraocular melanoma and retinoblastoma), faciodigitogenital dysplasia, familial fibrous dysplasia of jaws, familial white folded dysplasia, fibroma, fibromuscular dysplasia, fibromuscular hyperplasia, fibrous dysplasia of bone, florid osseous dysplasia, focal epithelial hyperplasia, gall bladder cancer, ganglioneuroma, gastric cancer (including, *e.g.,* stomach cancer), gastrointestinal carcinoid tumor, gastrointestinal tract cancer, gastrointestinal tumors, Gaucher's disease, germ cell tumors (including, *e.g.,* extracranial germ cell tumors, extragonadal germ cell tumors, and ovarian germ cell tumors), giant cell tumor, gingival hyperplasia, glioblastoma, glomangioma, granulosa cell tumor, gynandroblastoma, hamartoma, head and neck cancer, hemangioendothelioma, hemangioma, hemangiopericytoma, hepatocellular cancer, hepatoma, hereditary renal-retinal dysplasia, hidrotic ectodermal dysplasia, histiocytonia, histiocytosis, hypergammaglobulinemia, hypohidrotic ectodermal dysplasia, hypopharyngeal cancer, inflammatory fibrous hyperplasia, inflammatory papillary hyperplasia, intestinal cancers, intestinal metaplasia, intestinal polyps, intraocular melanoma, intravascular papillary endothelial hyperplasia, kidney cancer, laryngeal cancer, leiomyoma, leukemia (including, *e.g.,* acute lymphoblastic leukemia, acute lymphocytic leukemia, acute myeloid leukemia, acute myelogenous leukemia, acute hairy cell leukemia, acute B-cell leukemia, acute T-cell leukemia, acute HTLV leukemia, chronic lymphoblastic leukemia, chronic lymphocytic leukemia, chronic myeloid leukemia, chronic myelogenous leukemia, chronic hairy cell leukemia, chronic B-cell leukemia, chronic T-cell leukemia, and chronic HTLV leukemia), Leydig cell tumor, lip and oral cavity cancer, lipoma, liver cancer, lung cancer (including, *e.g.,* small cell lung cancer and non-small cell lung cancer), lymphangiomyoma, lymphaugioma, lymphoma (including, *e.g.,* AIDS-related lymphoma, central nervous system lymphoma, primary central nervous system lymphoma, Hodgkin's lymphoma, non-Hodgkin's lymphoma, Hodgkin's lymphoma during pregnancy, non-Hodgkin's lymphoma during pregnancy, mast cell lymphoma, B-cell lymphoma, adenolymphoma, Burkitt's lymphoma, cutaneous T-cell lymphoma, large cell lymphoma, and small cell lymphoma), lymphopenic thymic dysplasia, lymphoproliferative disorders, macroglobulinemia (including, *e.g.,* Waldenstrom's macroglobulinemia), malignant carcinoid syndrome, malignant mesothelioma, malignant thymoma, mammary dysplasia, mandibulofacial dysplasia, medulloblastoma, meningioma, mesenchymoma, mesonephroma, mesothelioma (including, *e.g.,* malignant mesothelioma), metaphysial dysplasia, metaplastic anemia, metaplastic ossification, metaplastic polyps, metastatic squamous neck cancer (including, *e.g.,* metastatic squamous neck cancer with occult primary), Mondini dysplasia, monostotic fibrous dysplasia, mucoepithelial dysplasia, multiple endocrine neoplasia syndrome, multiple epiphysial dysplasia, multiple myeloma/plasma cell neoplasm, mycosis fungoides, myelodysplastic syndrome, myeloid metaplasia, myeloproliferative disorders, chronic myeloproliferative disorders, myoblastoma, myoma, myxoma, nasal cavity and paranasal sinus cancer, nasopharyngeal cancer, prostatic neoplasm, colon neoplasm, abdomen neoplasm, bone neoplasm, breast neoplasm, digestive system neoplasm, liver neoplasm, pancreas neoplasm, peritoneum neoplasm, endocrine glands neoplasm (including, *e.g.,* adrenal neoplasm, parathyroid neoplasm, pituitary neoplasm, testicles neoplasm, ovary neoplasm, thymus neoplasm, and thyroid neoplasm), eye neoplasm, head and neck neoplasm, nervous system neoplasm (including, *e.g.,* central nervous system neoplasm and peripheral nervous system neoplasm), lymphatic system neoplasm, pelvic neoplasm, skin neoplasm, soft tissue neoplasm, spleen neoplasm, thoracic neoplasm, urogenital tract neoplasm, neurilemmoma, neuroblastoma, neuroepithelioma, neurofibroma, neurofibromatosis, neuroma, nodular hyperplasia of prostate, nodular regenerative hyperplasia, oculoauriculovertebral dysplasia, oculodentodigital dysplasia, oculovertebral dysplasia, odontogenic dysplasia, odontoma, opthalmomandibulomelic dysplasia, oropharyngeal cancer, osteoma, ovarian cancer (including, *e.g.,* ovarian epithelial cancer and ovarian low malignant potential tumor), pancreatic cancer (including, e.g., islet cell pancreatic cancer and exocrine pancreatic cancer), papilloma, paraganglioma, nonchromaffin paraganglioma, paranasal sinus and nasal cavity cancer, paraproteinemias, parathyroid cancer, periapical cemental dysplasia, pheochromocytoma (including, *e.g.,* penile cancer), pineal and supratentorial primitive neuroectodermal tumors, pinealoma, pituitary tumor, plasma cell neoplasm/multiple myeloma, plasmacytoma, pleuropulmonary blastoma, polyostotic fibrous dysplasia, polyps, pregnancy cancer, pre-neoplastic disorders (including, *e.g.,* benign dysproliferative disorders such as benign tumors, fibrocystic conditions, tissue hypertrophy, intestinal polyps, colon polyps, esophageal dysplasia, leukoplakia, keratoses, Bowen's disease, Farmer's skin, solar cheilitis, and solar keratosis), primary hepatocellular cancer, primary liver cancer, primary myeloid metaplasia, prostate cancer, pseudoachondroplastic spondyloepiphysial dysplasia, pseudoepitheliomatous hyperplasia, purpura, rectal cancer, renal cancer (including, *e.g.,* kidney cancer, renal pelvis, ureter cancer, transitional cell cancer of the renal pelvis and ureter), reticuloendotheliosis, retinal dysplasia, retinoblastoma, salivary gland cancer, sarcomas (including, *e.g.,* uterine sarcoma, soft tissue sarcoma, carcinosarcoma, chondrosarcoma, fibrosarcoma, hemangiosarcoma, Kaposi's sarcoma, leiomyosarcoma, liposarcoma, lymphangiosarcoma, myosarcoma, myxosarcoma, rhabdosarcoma, sarcoidosis sarcoma, osteosarcoma, Ewing sarcoma, malignant fibrous histiocytoma of bone, and clear cell sarcoma of tendon sheaths), sclerosing angioma, secondary myeloid metaplasia, senile sebaceous hyperplasia, septooptic dysplasia, Sertoli cell tumor, Sezary syndrome, skin cancer (including, *e.g.,* melanoma skin cancer and non-melanoma skin cancer), small intestine cancer, spondyloepiphysial dysplasia, squamous metaplasia (including, *e.g.,* squamous metaplasia of amnion), stomach cancer, supratentorial primitive neuroectodermal and pineal tumors, supratentorial primitive neuroectodermal tumors, symptomatic myeloid metaplasia, teratoma, testicular cancer, theca cell tumor, thymoma (including, *e.g.,* malignant thymoma), thyroid cancer, trophoblastic tumors (including, *e.g.,* gestational trophoblastic tumors), ureter cancer, urethral cancer, uterine cancer, vaginal cancer, ventriculoradial dysplasia, verrucous hyperplasia, vulvar cancer, Waldenstrom's macroglobulinemia, and Wilms' tumor.

In one embodiment, the disease is an ocular disease.

Examples of ocular diseases include, but are not limited to, age-related macular degeneration, glaucoma, macular edema, diabetic retinopathy, uveitis, allergic conjunctivitis, scleritis, keratitis, keratoconjunctivitis, vernal keratoconjunctivitis, atopic keratoconjunctivitis, cicatrizing conjunctivitis, blepharitis, endophthalmitis, retinitis, retinopathies, choroiditis, Sjogren's syndrome and retinal necrosis.

In one embodiment, the disease is an age-related disease.

Examples of age-related diseases include, but are not limited to, benign tumors; cardiovascular diseases, such as stroke, atherosclerosis and hypertension; angioma; osteoporosis; insulin-resistance and type II diabetes, including diabetic retinopathy and neuropathy; Alzheimer's disease; Parkinson's disease; age-related macular degeneration; arthritis; seborrheic keratosis; actinic keratosis; photoaged skin; skin spots; skin cancer; systemic lupus erythematosus; psoriasis; smooth muscle cell proliferation and intimal thickening following vascular injury; inflammation; arthritis; side effects of chemotherapy; benign prostatic hyperplasia; as well as less common diseases wherein their incidence is higher in elderly people than in young people.

In one embodiment, the disease is aging.

**"Aging",** also referred to as **"organismal aging"**, is manifested by age-related diseases, the incidence of which increases with age. Death from aging means death from age-related diseases. Suppression or alleviation of aging delays one, some or most age-related diseases. Slow aging is manifested by delayed age-related diseases. Slow aging is considered to be a type healthy aging.

In one embodiment, the methods according to the present invention are for delaying organismal aging.

In one embodiment, the peptide according to the present invention, or the composition, the pharmaceutical composition or the medicament according to the present invention, is for use in delaying organismal aging.

In one embodiment, the disease is selected from the group comprising or consisting of multiple sclerosis; rheumatoid arthritis; systemic lupus erythematosus; diabetes; obesity; non-alcoholic steatohepatitis; amyotrophic lateral sclerosis; Parkinson's disease and related disorders; Alzheimer's disease and related disorders; Huntington disease; Gaucher's disease; age-related macular degeneration; and glaucoma. In one embodiment, the disease is selected from the group comprising or consisting of multiple sclerosis; rheumatoid arthritis; systemic lupus erythematosus; diabetes; obesity; and non-alcoholic steatohepatitis. In one embodiment, the disease is selected from the group comprising or consisting of amyotrophic lateral sclerosis; Parkinson's disease and related disorders; Alzheimer's disease and related disorders; and multiple sclerosis. In some embodiments, the disease is multiple sclerosis. In some embodiments, the disease is rheumatoid arthritis. In some embodiments, the disease is systemic lupus erythematosus. In some embodiments, the disease is diabetes. In some embodiments, the disease is obesity. In some embodiments, the disease is non-alcoholic steatohepatitis. In some embodiments, the disease is amyotrophic lateral sclerosis. In some embodiments, the disease is Parkinson's disease and related disorders. In some embodiments, the disease is Alzheimer's disease and related disorders. In some embodiments, the disease is Huntington disease. In some embodiments, the disease is Gaucher's disease. In some embodiments, the disease is age-related macular degeneration. In some embodiments, the disease is glaucoma.

The present invention also relates to methods of increasing the level of at least one anti-inflammatory cytokine in a subject in need thereof, in particular in the blood of said subject, comprising administering to said subject a peptide according to the present invention, or the composition, the pharmaceutical composition or the medicament according to the present invention.

The present invention also relates to a peptide according to the present invention, or to the composition, the pharmaceutical composition or the medicament according to the present invention, for use in increasing the level of at least one anti-inflammatory cytokine in a subject in need thereof, in particular in the blood of said subject.

Examples of anti-inflammatory cytokines include, but are not limited to, interleukin-10 (IL-10), transforming growth factor β (TGF-β), interleukin-1ra (IL-1ra), interleukin-4 (IL-4), interleukin-6 (IL-6), interleukin-11 (IL-11), interleukin-13 (IL-13) and interleukin-22 (IL-22).

Methods to measure blood cytokine levels in a subject are known in the art. Such methods include, for example, the use of ELISA test, such as the one described in the Example section below.

In particular, the present invention relates to a method of increasing the level of IL-10 in a subject in need thereof, in particular in the blood of said subject, comprising administering to said subject a peptide according to the present invention, or the composition, the pharmaceutical composition or the medicament according to the present invention.

In particular, the present invention relates to a peptide according to the present invention, or to the composition, the pharmaceutical composition or the medicament according to the present invention, for use in increasing the level of IL-10 in a subject in need thereof, in particular in the blood of said subject.

Methods to measure IL-10 levels in a blood sample from a subject are well known in the art and include, *e.g.,* the use of an ELISA test.

In one embodiment, the peptide according to the present invention, or the composition, the pharmaceutical composition or the medicament according to the present invention triggers an increase of IL-10 levels in the blood of a subject by at least 100%, 200%, 300%, in particular at least 400% and more particularly at least 500%, as compared to the administration of negative control molecule.

The present invention also relates to methods of lowering glucose levels in a subject in need thereof, in particular in the blood of said subject, comprising administering to said subject a peptide according to the present invention, or the composition, the pharmaceutical composition or the medicament according to the present invention.

The present invention also relates to a peptide according to the present invention, or to the composition, the pharmaceutical composition or the medicament according to the present invention, for use in lowering glucose levels in a subject in need thereof, in particular in the blood of said subject.

Methods to measure blood glucose levels in a subject are known in the art, and include, without limitation, chemical methods (such as, *e.g.,* the Folin-Wu method, the Benedict's method, the Nelson-Somogyi method, the neocuproine method, the Shaeffer-Hartmann-Somogyi method, the Hagedorn-Jensen method, the ortho-toluidine method, and the anthrone method) and enzymatic methods (such as, *e.g.,* the Saifer-Gerstenfeld method, the Trinder method, the Kodak Ektachem method, and the use of glucometers, either electronic or in the form of strips impregnated with a glucose oxidase reagent).

In humans, so-called "normal" blood glucose levels range from about 4.4 mmol/L to about 6.1 mmol/L, that is to say from about 79 mg/dL to about 110 mg/dL.

In one embodiment, the expression **"lowering glucose levels"** refer to reducing or decreasing the level or measurement (when compared to baseline) of one or more of (i) fasting whole blood glucose test; (ii) fasting plasma glucose test; (iii) fasting serum glucose test; (iv) non-fasting whole blood glucose test; (v) non-fasting plasma glucose test; (vi) non-fasting serum glucose test; or (vii) OGTT, by any amount compared to baseline. In one embodiment, a decrease in blood glucose level is determined relative to the starting blood glucose level of the subject (for example, prior to treatment with a peptide according to the present invention, or the composition, the pharmaceutical composition or the medicament according to the present invention).

In one embodiment, the glucose level is lowered or reduced by at least 1 mg/dL, such as by 5 mg/dL, 10 mg/dL, 20 mg/dL, 30 mg/dL, 40 mg/dL, 50 mg/dL, 60 mg/dL, 70 mg/dL, 80 mg/dL, 90 mg/dL, 100 mg/dL or more.

In one embodiment, the subject is an animal. In one embodiment, the subject is a mammal.

Examples of mammals include, but are not limited to, primates (including human and non-human), cattle (including cows), horses, pigs, sheep, goats, dogs and cats.

In a preferred embodiment, the subject is a human.

In one embodiment, the subject is an adult (*e.g.,* a subject above the age of 18 in human years or a subject after reproductive capacity has been attained). In another embodiment, the subject is a child (for example, a subject below the age of 18 in human years or a subject before reproductive capacity has been attained).

In one embodiment, the subject is a male. In one embodiment, the subject is a female.

In one embodiment, the subject is/was diagnosed with a disease selected from the group comprising or consisting of inflammatory diseases; autoimmune diseases; metabolic and endocrine diseases; neurodegenerative diseases; neuroinflammatory diseases; ocular diseases; age-related diseases; and cancer and metastasis.

In one embodiment, the subject is/was diagnosed with a disease selected from the group comprising or consisting of amyotrophic lateral sclerosis; Parkinson's disease and related disorders; Alzheimer's disease and related disorders; Huntington disease; multiple sclerosis; rheumatoid arthritis; systemic lupus erythematosus; diabetes; obesity; non-alcoholic steatohepatitis; age-related macular degeneration; and glaucoma.

In one embodiment, the subject is at risk of developing a disease selected from the group comprising or consisting of inflammatory diseases; autoimmune diseases; metabolic and endocrine diseases; neurodegenerative diseases; neuroinflammatory diseases; ocular diseases; age-related diseases; and cancer and metastasis.

In one embodiment, the subject is at risk of developing a disease selected from the group comprising or consisting of amyotrophic lateral sclerosis; Parkinson's disease and related disorders; Alzheimer's disease and related disorders; Huntington disease; multiple sclerosis; rheumatoid arthritis; systemic lupus erythematosus; diabetes; obesity; non-alcoholic steatohepatitis; age-related macular degeneration; and glaucoma.

In one embodiment, the subject is/was diagnosed with increased plasma soluble CD38 levels.

In one embodiment, the subject is/was diagnosed with increased cerebrospinal fluid soluble CD38 levels.

In one embodiment, the subject is/was diagnosed with increased soluble CD38 levels in a muscle biopsy.

Methods to detect and measure soluble CD38 levels are well known to the one skilled in the art. In particular, means and kits for detecting and measuring soluble CD38 levels are commercially available.

In one embodiment, the peptide, composition, pharmaceutical composition or medicament according to the present invention will be formulated for administration to the subject.

In one embodiment, the peptide, composition, pharmaceutical composition or medicament according to the present invention is to be administered systemically or locally.

In one embodiment, the peptide, composition, pharmaceutical composition or medicament according to the present invention is to be administered by injection, orally, topically, nasally, buccally, rectally, vaginaly, intratracheally, by endoscopy, transmucosally, or by percutaneous administration.

In one embodiment, the peptide, composition, pharmaceutical composition or medicament according to the present invention is to be injected, preferably systemically inj ected.

Examples of formulations adapted for injection include, but are not limited to, solutions, such as, for example, sterile aqueous solutions, gels, dispersions, emulsions, suspensions, solid forms suitable for using to prepare solutions or suspensions upon the addition of a liquid prior to use, such as, for example, powder, liposomal forms and the like.

Examples of systemic injections include, but are not limited to, intravenous (iv), subcutaneous (sq), intradermal (id), intramuscular (im), intraarterial, intraparenteral, intranodal, intralymphatic, intraperitoneal (ip), intracranial, intracardiac, intralesional, intraprostatic, intravaginal, intrarectal, intrathecal, intranasal, intratumoral (it), intravesicular, and perfusion.

In one embodiment, when injected, the peptide, composition, pharmaceutical composition or medicament according to the present invention is sterile. Methods for obtaining a sterile composition include, but are not limited to, GMP synthesis (where GMP stands for "Good manufacturing practice").

Sterile injectable forms of a composition may be aqueous or an oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent. Among the acceptable vehicles and solvents that may be employed are water, Ringer's solution and isotonic sodium chloride solution. In addition, sterile, fixed oils are conventionally employed as a solvent or suspending medium. For this purpose, any bland fixed oil may be employed including synthetic mono- or diglycerides. Fatty acids, such as oleic acid and its glyceride derivatives are useful in the preparation of injectables, as are natural pharmaceutically acceptable oils, such as olive oil or castor oil, especially in their polyoxyethylated versions. These oil solutions or suspensions may also contain a long-chain alcohol diluent or dispersant, such as carboxymethyl cellulose or similar dispersing agents that are commonly used in the formulation of pharmaceutically acceptable dosage forms including emulsions and suspensions. Other commonly used surfactants, such as Tweens, Spans and other emulsifying agents or bioavailability enhancers which are commonly used in the manufacture of pharmaceutically acceptable solid, liquid, or other dosage forms may also be used for the purposes of formulation.

In one embodiment, the peptide, composition, pharmaceutical composition or medicament according to the present invention is to be administered orally. Example of formulations adapted for oral administration include, but are not limited to, solid forms, liquid forms and gels. Examples of solid forms adapted to oral administration include, but are not limited to, tablets, capsules (each of which includes sustained-release or timed-release formulations), pills, powders, granules, wafers, as well as effervescent tablet and any other solid forms suitable for solution in, or suspension in, liquid prior to oral administration. Examples of liquid forms adapted to oral administration include, but are not limited to, solutions, suspensions, emulsions, elixirs, tinctures, potions, syrups and liquors.

It will be understood that other suitable routes of administration are also contemplated in the present invention, and the administration mode will ultimately be decided by the attending physician within the scope of sound medical judgment. Apart from administration by injection (iv, ip, im and the like), other routes are available, such as nebulization (Respaud et al., 2014. MAbs. 6(5):1347-55; Guilleminault et al., 2014. J Control Release. 196:344-54; Respaud et al., 2015. Expert Opin Drug Deliv. 12(6):1027-39) or subcutaneous administration (Jackisch et al., 2014. Geburtshilfe Frauenheilkd. 74(4):343-349; Solal-Celigny, 2015. Expert Rev Hematol. 8(2):147-53).

In one embodiment, the peptide, composition, pharmaceutical composition or medicament according to the present invention is able to cross the blood-brain barrier (BBB), preferably when using an oral, intravenous or subcutaneous route of administration.

In one embodiment, the peptide, composition, pharmaceutical composition or medicament according to the present invention is to be administered to the subject in need thereof in a therapeutically effective amount.

Such therapeutically amount can be determined by one skilled in the art by routine tests including assessment of the effect of administration of the peptide, composition, pharmaceutical composition or medicament on the diseases which are sought to be prevented and/or to be treated by the administration of said peptide, composition, pharmaceutical composition or medicament according to the present invention.

For example, such tests can be implemented by analyzing both quantitative and qualitative effect of the administration of different amounts of said aforementioned peptide, composition, pharmaceutical composition or medicament according to the present invention on a set of markers (biological and/or clinical) characteristics of said diseases, in particular from a biological sample of a subject.

It will be however understood that the total daily usage of the peptide, composition, pharmaceutical composition or medicament according to the present invention will be decided by the attending physician within the scope of sound medical judgment. The specific therapeutically effective dose level for any particular patient will depend upon a variety of factors including the disease being treated and the severity of the disease; activity of the peptide, composition, pharmaceutical composition or medicament employed; the age, body weight, general health, sex and diet of the subject; the time of administration, route of administration, and rate of excretion of the peptide, composition, pharmaceutical composition or medicament employed; the duration of the treatment; drugs used in combination or coincidental with the peptide, composition, pharmaceutical composition or medicament employed; and like factors well known in the medical arts. For example, it is well within the skill of the art to start doses of the peptide at levels lower than those required to achieve the desired therapeutic effect and to gradually increase the dosage until the desired effect is achieved. The total dose required for each treatment may be administered by multiple doses or in a single dose.

In one embodiment, a therapeutically effective amount of the peptide, composition, pharmaceutical composition or medicament according to the present invention ranges from about 1 ng/kg to about 10 $\mu$g/kg (human equivalent dose (HED)). In one embodiment, a therapeutically effective amount of the peptide, composition, pharmaceutical composition or medicament according to the present invention is about 1 ng/kg, 5 ng/kg, 10 ng/kg, 25 ng/kg, 50 ng/kg, 75 ng/kg, 100 ng/kg, 150 ng/kg, 200 ng/kg, 250 ng/kg, 300 ng/kg, 350 ng/kg, 400 ng/kg, 450 ng/kg, 500 ng/kg, 550 ng/kg, 600 ng/kg, 650 ng/kg, 700 ng/kg, 750 ng/kg, 800 ng/kg, 850 ng/kg, 900 ng/kg, 950 ng/kg, 1 $\mu$g/kg, 1.5 $\mu$g/kg, 2$\mu$g/kg, 2.5 $\mu$g/kg, 3 $\mu$g/kg, 3.5 $\mu$g/kg, 4 $\mu$g/kg, 4.5 $\mu$g/kg, 5 $\mu$g/kg, 5.5 $\mu$g/kg, 6 $\mu$g/kg, 6.5 $\mu$g/kg, 7 $\mu$g/kg, 7.5 $\mu$g/kg, 8 $\mu$g/kg, 8.5 $\mu$g/kg, 9 $\mu$g/kg, 9.5 $\mu$g/kg, 10 $\mu$g/kg.

In one embodiment, a therapeutically effective amount of the peptide, composition, pharmaceutical composition or medicament according to the present invention ranges from about 50 ng/day to about 1 mg/day (human equivalent dose (HED)). In one embodiment, a therapeutically effective amount of the peptide, composition, pharmaceutical composition or medicament according to the present invention is about 50 ng/day, 100 ng/day, 200 ng/day, 300 ng/day, 400 ng/day, 500 ng/day, 600 ng/day, 700 ng/day, 800 ng/day, 900 ng/day, 1 $\mu$g/day, 10 $\mu$g/day, 20 $\mu$g/day, 30 $\mu$g/day, 40 $\mu$g/day, 50 $\mu$g/day, 60 $\mu$g/day, 70 $\mu$g/day, 80 $\mu$g/day, 90 $\mu$g/day, 100 $\mu$g/day, 150 $\mu$g/day, 200 $\mu$g/day, 250 $\mu$g/day, 300 $\mu$g/day, 350 $\mu$g/day, 400 $\mu$g/day, 450 $\mu$g/day, 500 $\mu$g/day, 550 $\mu$g/day, 600 $\mu$g/day, 650 $\mu$g/day, 700 $\mu$g/day, 750 $\mu$g/day, 800 $\mu$g/day, 850 $\mu$g/day, 900 $\mu$g/day, 950 $\mu$g/day, 1 mg/day.

In one embodiment, a therapeutically effective amount of the peptide, composition, pharmaceutical composition or medicament according to the present invention is to be administered once a day, twice a day, three times a day or more.

In one embodiment, a therapeutically effective amount of the peptide, composition, pharmaceutical composition or medicament according to the present invention is to be administered every day, every two days, every three days, every four days, every five days, every six days.

In one embodiment, a therapeutically effective amount of the peptide, composition, pharmaceutical composition or medicament according to the present invention is to be administered every week, every two weeks, every three weeks.

In one embodiment, a therapeutically effective amount of the peptide, composition, pharmaceutical composition or medicament according to the present invention is to be administered every month, every two months, every three months, every four months, every five months, every six months.

In a preferred embodiment, a therapeutically effective amount of the peptide, composition, pharmaceutical composition or medicament according to the present invention is to be administered every 12 hours, every 24 hours, every 36 hours, every 48 hours, every 60 hours, every 72 hours, every 96 hours.

In a preferred embodiment, a therapeutically effective amount of the peptide, composition, pharmaceutical composition or medicament according to the present invention is to be administered every 60 hours.

In one embodiment, the peptide, composition, pharmaceutical composition or medicament according to the present invention is for acute administration. In one embodiment, the peptide, composition, pharmaceutical composition or medicament according to the present invention is for chronic administration.

In one embodiment, a therapeutically effective amount of the peptide, composition, pharmaceutical composition or medicament according to the present invention is to be administered for about 5 days, 7 days, 10 days, 14 days, 21 days, 28 days, 1 month, 2 months, 3 months, 6 months, 1 year or more.

In one embodiment, the peptide, composition, pharmaceutical composition or medicament according to the present invention is to be administered before, concomitantly with or after an additional therapeutic or diagnostic agent.

Suitable additional therapeutic or diagnostic agents include all those described hereinabove as therapeutic or diagnostic payloads of the peptide conjugates, including, but are not limited to, chemotherapeutic agents, targeted therapy agents, cytotoxic agents, antibiotics, antivirals, cell cycle-synchronizing agents, ligands for cellular receptor(s), immunomodulatory agents, pro-apoptotic agents, anti-angiogenic agents, cytokines, growth factors, antibodies or antigen-binding fragments thereof, hormones, coding or non-coding oligonucleotides, photodetectable labels, contrast agents, radiolabels, and the like.

One skilled in the art will readily understand that these therapeutic or diagnostic agents can, additionally or alternatively, be provided as unconjugated to the peptides according to the present invention.

## BRIEF DESCRIPTION OF THE DRAWINGS

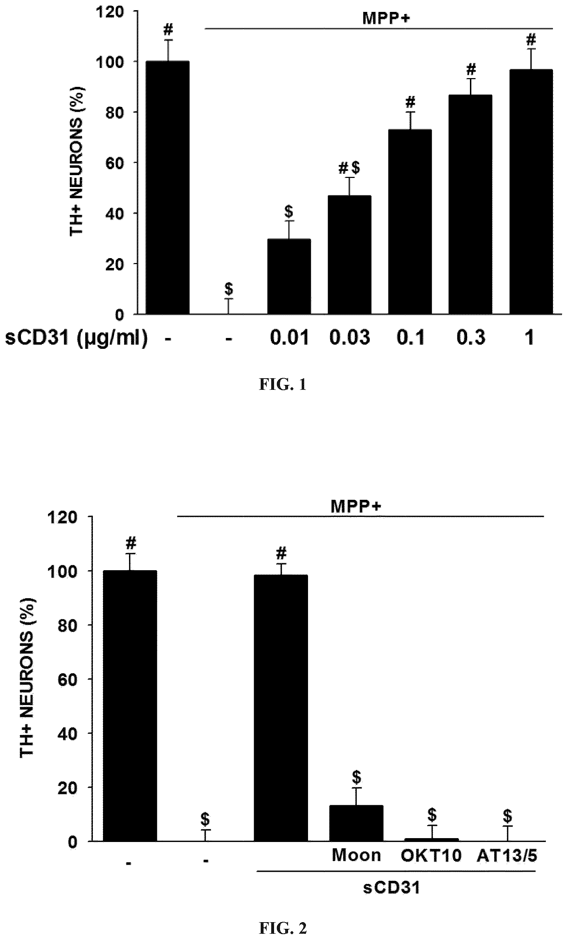

Figure 1 is a plot showing the neuroprotective effect of sCD31 against MPP$^+$-induced dopaminergic (DA) cell death. DA (TH$^+$) cell survival in midbrain cultures treated with MPP$^+$ (3 $\mu$M) between 5 and 7 DIV exposed or not to sCD31 (0.01, 0.03, 0.1, 0.3, 1 $\mu$g/mL). Results are expressed in % of corresponding control cultures. $ P < 0.05 one-way ANOVA vs control treatment; # P < 0.05 one-way ANOVA *vs* MPP$^+$ treatment.

Figure 2 is a bar graph showing the neuroprotective effect of sCD31 against MPP$^+$-induced DA cell death. DA (TH$^+$) cell survival in midbrain cultures treated with MPP$^+$ (3 $\mu$M) between 5 and 7 DIV exposed or not to sCD31 (1 $\mu$g/mL) in the presence or not of anti-CD31 antibody clone Moon-1 (1 $\mu$g/mL). of the antagonistic anti-CD38 antibody clone OKT10 (OKT, 1 $\mu$g/mL). or of the antagonistic anti-CD38 antibody clone AT13/5 (1 $\mu$g/mL). Results are expressed in % of corresponding control cultures. $ P < 0.05 one-way ANOVA vs control treatment; # P < 0.05 one-way ANOVA vs MPP$^+$ treatment.

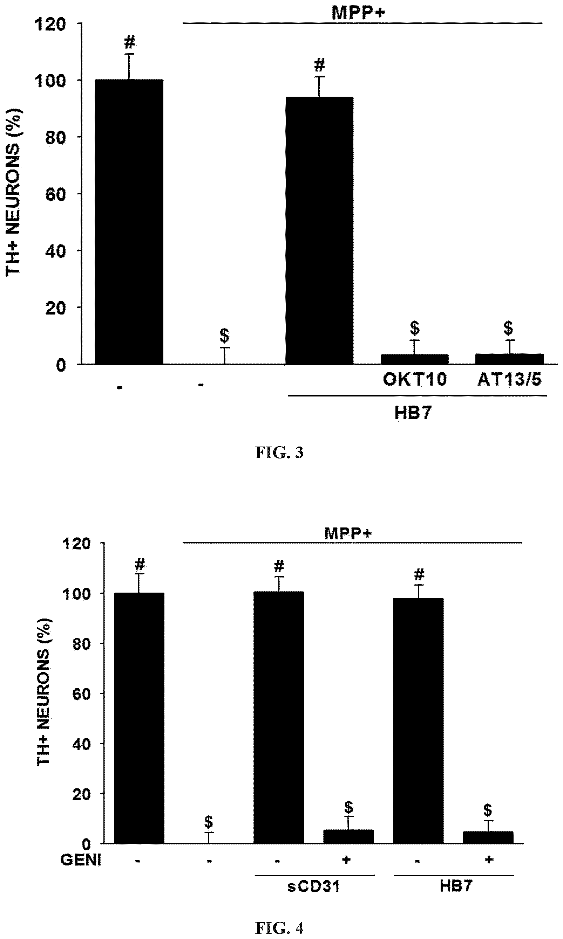

Figure 3 is a bar graph showing the neuroprotective effect of agonistic anti-CD38 antibody clone HB7 against MPP$^+$-induced DA cell death. DA (TH$^+$) cell survival in midbrain cultures treated with MPP$^+$ (3 $\mu$M) between 5 and 7 DIV exposed or not to agonistic anti-CD38 antibody (clone HB7, 1 $\mu$g/mL) in the presence or not of the antagonistic anti-CD38 antibody clone OKT10 (OKT, 1 $\mu$g/mL). or of the antagonistic anti-CD38 antibody clone AT13/5 (1 $\mu$g/mL). Results are expressed in % of corresponding control cultures. $ P < 0.05 one-way ANOVA vs control treatment; # P < 0.05 one-way ANOVA vs MPP$^+$ treatment.

**Figure 4** is a bar graph showing the neuroprotective effect of sCD31 or agonistic anti-CD38 antibody clone HB7 against MPP$^+$-induced DA cell death in the presence or absence of the tyrosine kinase inhibitor genistein. DA (TH$^+$) cell survival in midbrain cultures treated with MPP$^+$ (3 $\mu$M) between 5 and 7 DIV exposed or not to sCD31 (1 $\mu$g/mL) or agonistic anti-CD38 antibody (clone HB7, 1 $\mu$g/mL) in the presence or not of genistein (GENI, 10 nM), an inhibitor of tyrosine kinase. Results are expressed in % of corresponding control cultures. $ P < 0.05 one-way ANOVA *vs* control treatment; # P < 0.05 one-way ANOVA vs MPP$^+$ treatment.

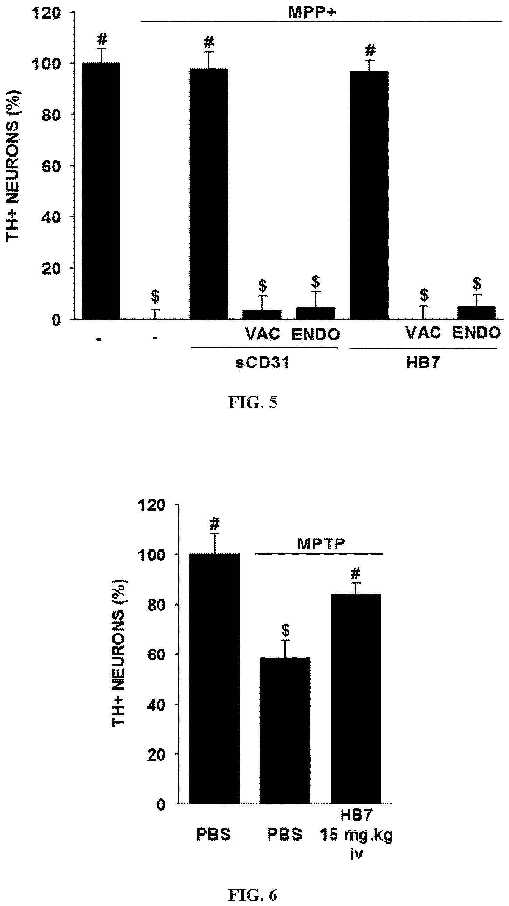

**Figure 5** is a bar graph showing the neuroprotective effect of sCD31 or agonistic anti-CD38 antibody clone HB7 against MPP$^+$-induced DA cell death in the presence or absence of the lysosomal exocytosis inhibitor vacuolin-1 or endosidin2. DA (TH$^+$) cell survival in midbrain cultures treated with MPP$^+$ (3 $\mu$M) between 5 and 7 DIV exposed or not to sCD31 (1 $\mu$g/ml) or agonistic anti-CD38 antibody (clone HB7, 1 $\mu$g/ml) in the presence or not of vacuolin-1 (VAC, 10 $\mu$M), or endosidin2 (40 $\mu$M), two inhibitors of lysosomal maturation and exocytosis. Results are expressed in % of corresponding control cultures. $ P < 0.05 one-way ANOVA *vs* control treatment; # P < 0.05 one-way ANOVA *vs* MPP$^+$ treatment.

**Figure 6** is a bar graph showing the effect of agonistic anti-CD38 HB7 antibody injected intravenously (15 mg/kg iv, two days before MPTP injections) in the *in vivo* MPTP mouse model on the number of DA (TH$^+$) neurons in the *substantia nigra pars compacta.* Results are expressed in % of control (PBS-injected) mice. PBS group: n = 5; MPTP group: n = 6; MPTP + HB7 15 mg/kg iv group: n = 8. $ P < 0.05 one-way ANOVA *vs* control (PBS-injected) group. # P < 0.05 one-way ANOVA vs MPTP group.

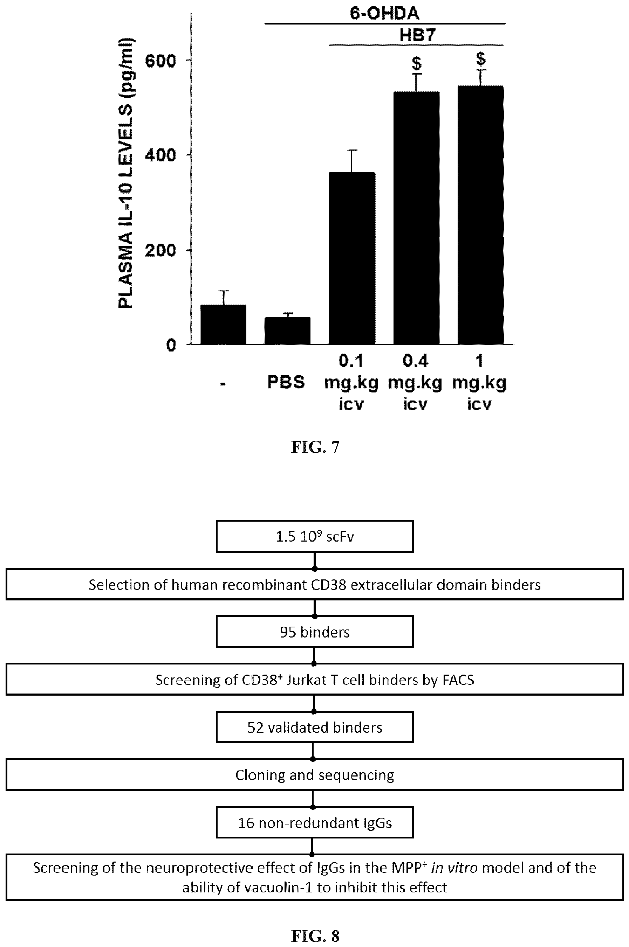

**Figure 7** is a bar graph showing the effect of intravenous injection of agonistic anti-CD38 clone HB7 on IL-10 levels in mice. $ P < 0.05 one-way ANOVA *vs* control (PBS-injected) group.

**Figure 8** is a diagram depicting the process used to identify anti-CD38 antibodies that competes with CD31.

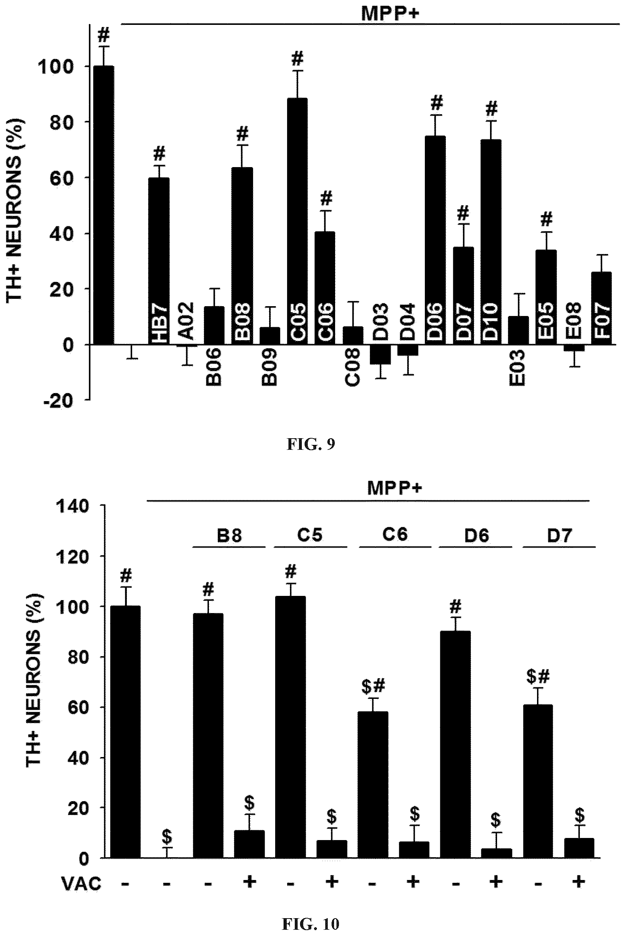

**Figure 9** is a bar graph showing the neuroprotective effect of new humanized anti-CD38 antibodies (clone A02, B06, B08, B09, C05, C06, C08, D03, D04, D06, D07, D10, E03, E05, E08, F07) identified by phage display or anti-CD38 antibody clone HB7 against MPP$^+$-induced DA cell death. DA (TH$^+$) cell survival in midbrain cultures treated with MPP$^+$ (3 $\mu$M) between 5 and 7 DIV exposed or not to anti-CD38 antibodies (clone A02, B06, B08, B09, C05, C06, C08, D03, D04, D06, D07, D10, E03, E05, E08, F07; 0.5 $\mu$g/mL) or anti-CD38 antibody clone HB7 (0.5 $\mu$g/mL). Results are expressed in % of corresponding control cultures. # P < 0.05 one-way ANOVA *vs* MPP$^+$ treatment.

**Figure 10** is a bar graph showing the neuroprotective effect of new humanized anti-CD38 antibodies (clones B08, C05, C06, D06 and D07) identified by phage display against MPP$^+$-induced DA cell death in the presence or absence of the lysosomal exocytosis inhibitor vacuolin-1. DA (TH$^+$) cell survival in midbrain cultures treated with MPP$^+$ (3 $\mu$M) between 5 and 7 DIV exposed or not to anti-CD38 antibodies (clones B08, C05, C06, C08, D06 and D07; 1 $\mu$g/ml) in the presence or not of vacuolin-1 (VAC, 10 $\mu$M), an inhibitor of lysosomal exocytosis. Results are expressed in % of corresponding control cultures. $ P < 0.05 one-way ANOVA *vs* control treatment; # P < 0.05 one-way ANOVA vs MPP$^+$ treatment.

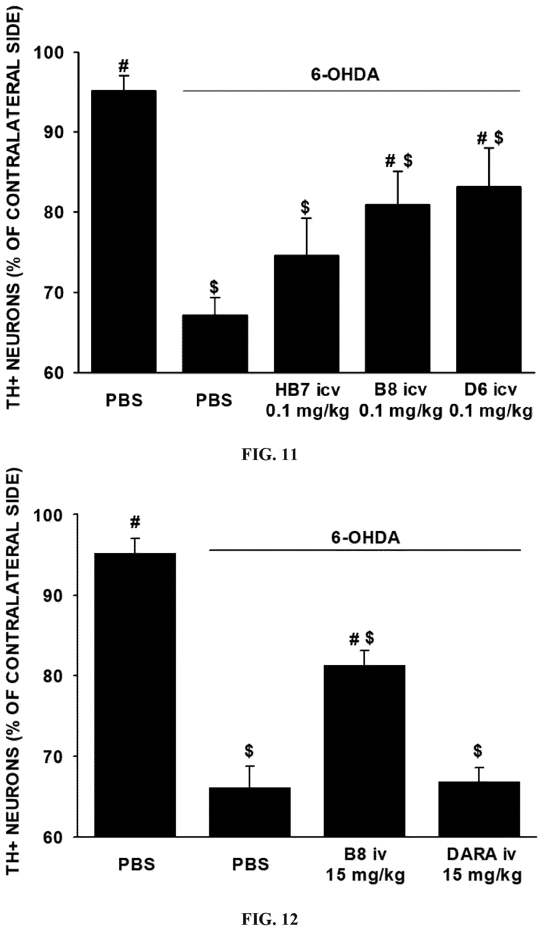

**Figure 11** is a bar graph showing the effect of anti-CD38 antibodies (clone HB7, B08 or D06) injected intracerebrally (0.1 mg/kg icv, concomitantly with 6-OHDA icv injection) in the *in vivo* 6-OHDA mouse model on the number of DA (TH$^+$) neurons in the *substantia nigra pars compacta.* Results are expressed in % of contralateral (non-lesioned) side. PBS group: n = 8 (PBS); 6-OHDA + PBS group: n = 8 (PBS); 6-OHDA + HB7 0.1 mg/kg icv group: n = 10 (HB7 icv 0.1 mg/kg); 6-OHDA + B08 0.1 mg/kg icv group: n = 7 (B8 icv 0.1 mg/kg); 6-OHDA + D06 0.1 mg/kg icv group: n = 7 (D6 icv 0.1 mg/kg). $ P < 0.05 one-way ANOVA vs control (PBS-injected) group. # P < 0.05 one-way ANOVA *vs* 6-OHDA group.

**Figure 12** is a bar graph showing the effect of anti-CD38 antibodies (clone B08 or Daratumumab, DARA) injected intravenously (15 mg/kg iv, one day before 6-OHDA icv injection) in the *in vivo* 6-OHDA mouse model on the number of DA (TH$^+$) neurons in the *substantia nigra pars compacta.* Results are expressed in % of contralateral (non-lesioned) side. PBS group: n = 8 (PBS); 6-OHDA group: n = 8 (PBS); 6-OHDA + B08 15 mg/kg iv group: n = 10 (B8 iv 15 mg/kg); 6-OHDA + Daratumumab 15 mg/kg iv group: n = 10 (DARA iv 15 mg/kg). $ P < 0.05 one-way ANOVA *vs* control (PBS-injected) group. # P < 0.05 one-way ANOVA vs 6-OHDA group.

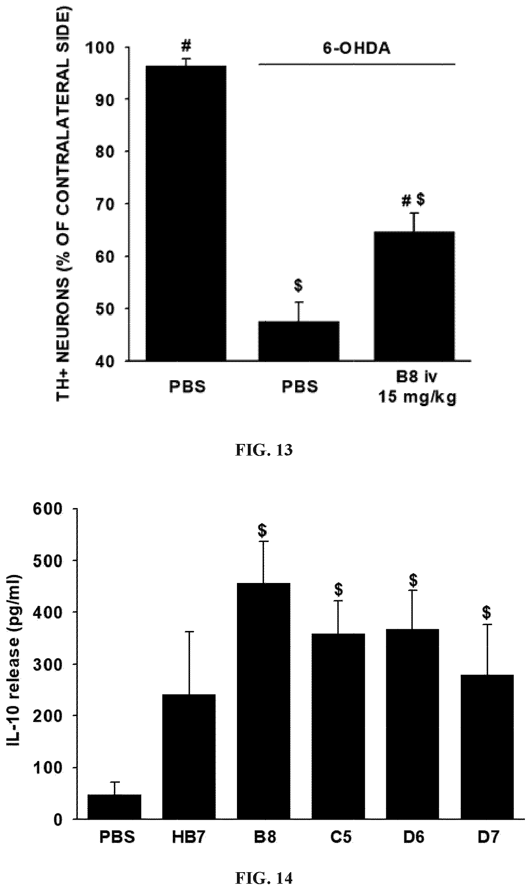

**Figure 13** is a bar graph showing the effect of the B08 anti-CD38 antibody injected intravenously (15 mg/kg iv, two days after 6-OHDA icv injection) in the *in vivo* 6-OHDA mouse model on the number of DA (TH$^+$) neurons in the *substantia nigra pars compacta.* Results are expressed in % of contralateral (non-lesioned) side. PBS group: n = 12 (PBS); 6-OHDA + PBS group: n = 12 (PBS); 6-OHDA + B08 15 mg/kg iv group: n = 12 (B8 iv 15 mg/kg). $ P < 0.05 one-way ANOVA *vs* control (PBS-injected) group. # P < 0.05 one-way ANOVA *vs* 6-OHDA group.

**Figure 14** is a bar graph showing the effect of anti-CD38 antibodies (clone HB7, B08, C05, D06 or D07) on the release of the anti-inflammatory cytokine IL-10 using human peripheral blood mononuclear cells from 3 healthy donors (pooled results). $ P < 0.05 one-way ANOVA vs control (PBS-treated) group.

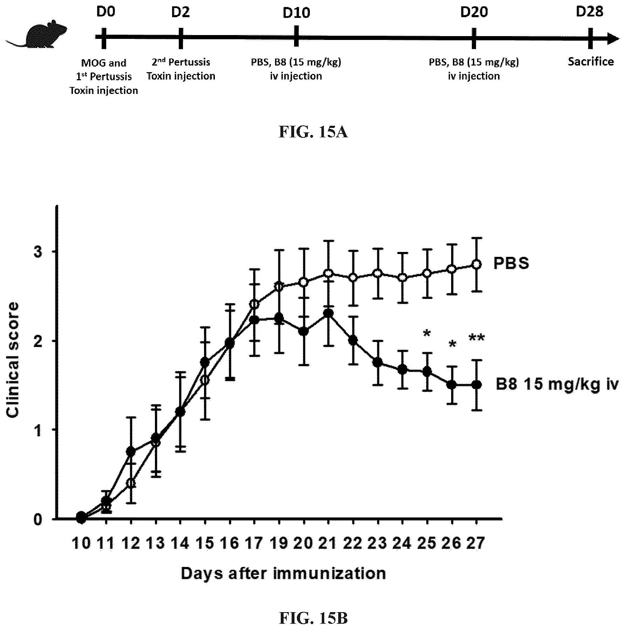

**Figure 15** is a set of scheme and graph. PBS group: n = 10 (PBS); B08 15 mg/kg iv group: n = 10 (B8 15 mg/kg iv). * P < 0.05 two-way ANOVA *vs* PBS group. ** P < 0.01 two-way ANOVA *vs* PBS group. Clinical score is calculated as described in the material and method section.

**Figure 15A** is a scheme representing the protocol.

**Figure 15B** is a bar graph showing the effect of anti-CD38 antibody (clone B08) injected intravenously (15 mg/kg iv, 10 and 20 days after MOG injection) in the *in vivo* EAE mouse model on the clinical score.

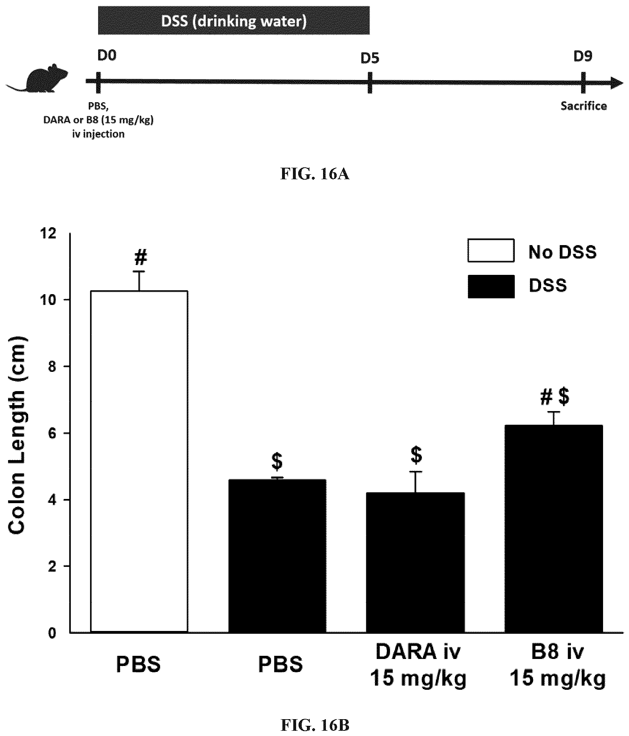

**Figure 16** is a set of schema and graph. No-DSS group: n = 5 (PBS; white bar); DSS + PBS group: n = 6 (PBS; black bar); DSS + DARA 15 mg/kg iv group: n = 7 (DARA iv 15 mg/kg; black bar); DSS + B08 15 mg/kg iv group: n = 6 (B8 iv 15 mg/kg; black bar). $ P < 0.05 one-way ANOVA *vs* no-DSS group. # P < 0.05 one-way ANOVA *vs* DSS group.

**Figure 16A** is a scheme representing the protocol.

**Figure 16B** is a bar graph showing the effect of anti-CD38 antibodies (clone B08 or Daratumumab, DARA) injected intravenously (15 mg/kg iv, injected at D0) in the *in vivo* DSS mouse model on colon length.

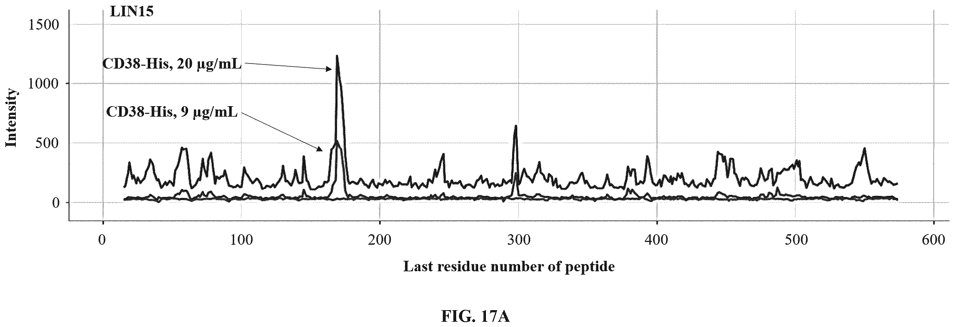

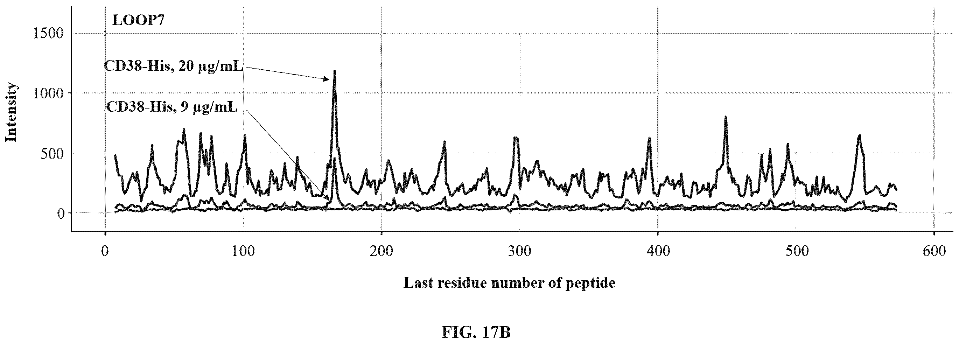

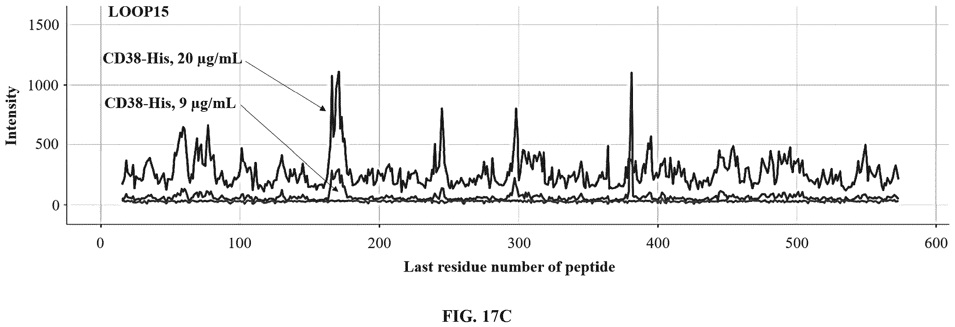

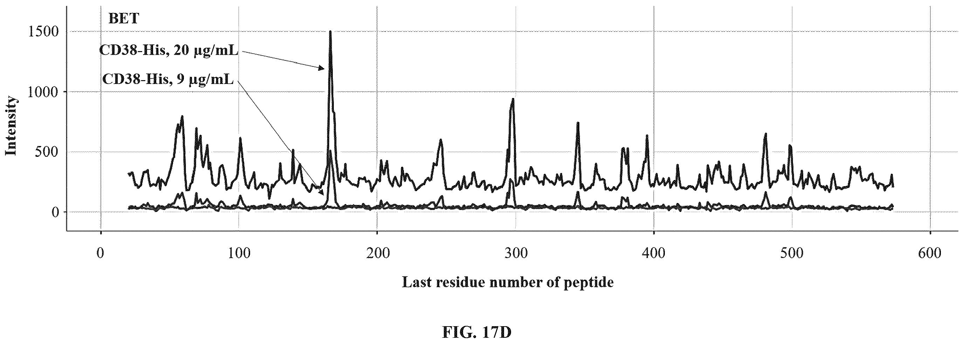

**Figure 17** is a set of four graphs showing the intensity profiles recorded for His-tagged CD38 at 20 $\mu$g/mL and 9 $\mu$g/mL on the CD31 peptide arrays. Signal intensities are plotted as a line plot indicating the binding intensity on the *y*-axis and CD31 peptide position in the sequence (indicated by the amino acid number of the last residue of each peptide; numbering based on residues 28 to 601 of SEQ ID NO: 1) on the *x*-axis.

**Figure 17A** shows the intensity profiles recorded for His-tagged CD38 on the linear peptide arrays (LIN15).

**Figure 17B** shows the intensity profiles recorded for His-tagged CD38 on the conformational peptide arrays (LOOP7).

**Figure 17C** shows the intensity profiles recorded for His-tagged CD38 on the conformational peptide arrays (LOOP 15).

**Figure 17D** shows the intensity profiles recorded for His-tagged CD38 on the conformational peptide arrays (BET).

**EXAMPLES**

The present invention is further described in the following examples, but the technical scope of the present invention is not limited to these examples.

**Materials and methods (Examples 1-7)**

*In vitro* experiments

*Midbrain cell cultures*

Animals were treated in accordance with the Guide for the Care and Use of Laboratory Animals (National Research Council, 1996), European Directive 86/609, and the guidelines of the local institutional animal care and use committee. Cultures were prepared from the ventral mesencephalon of gestational age 15.5 Wistar rat embryos (Janvier Breeding Center, Le Genest St Isle, France). Dissociated cells in suspension obtained by mechanical trituration of midbrain tissue pieces were seeded at a density of 1.2-1.5$\times$10$^5$ cells/cm$^2$ onto tissue culture supports precoated with 1 mg/mL polyethylenimine diluted in borate buffer pH 8.3 as described (Michel et al., 1997. J Neurochem. 69(4):1499-507).

The cultures were then maintained in N5 medium supplemented with 5 mM glucose, 5 % horse serum, and 0.5 % fetal calf serum, except for the first 3 DIV, when the concentration of fetal calf serum was 2.5% to favor initial maturation of the cultures (Guerreiro et al., 2008. Mol Pharmacol. 74(4):980-9). They were fed daily by replacing 70% of the medium. Routinely, mesencephalic cultures were established on Nunc 24-well culture plates (Thermofischer Scientific, Rochester, NY). Note that these cultures contain tyrosine hydroxylase (TH)+ neurons that were exclusively dopaminergic (Traver et al., 2006. Mol Pharmacol. 70(1):30-40). TH+ neurons represented approximately 1-2 % of the total number of neuronal cells present in these cultures. The evaluation of the survival of DA neurons was performed by counting cells immunopositive for TH as described (Toulorge et al., 2011. Faseb J. 25(8):2563-73).

*MPP+ intoxication model*

Treatments with MPP+ were performed in cultures where the spontaneous death process was prevented by long-term exposure to depolarizing concentrations of K+ (30 mM), in the presence of the glutamate receptor antagonist MK801 (5 $\mu$M) to prevent unwanted excitotoxic insults as described previously (Douhou et al., 2001. J Neurochem. 78(1):163-74). Treatments with MPP+ and potential neuroprotective molecules were carried out between 5 and 7 DIV.

*Quantification of neuronal survival*

The cultures were fixed for 12 min using 4 % formaldehyde in Dulbecco's phosphate-buffered saline (PBS), then washed twice with PBS before an incubation step at 4°C for 24 hours with the following antibodies. A monoclonal anti-TH antibody diluted 1/5000 (ImmunoStar, Inc., Hudson, WI) was used to assess the survival of DA neurons. Antibody was diluted in PBS containing 0.2 % Triton X-100. Detection of the primary antibodies was performed with an Alexa Fluor-488 conjugate of an anti-mouse IgG antibody.

*Human PBMCs cell culture*

3 healthy volunteers (Donor 1: woman, 23 years old; Donor 2: man, 29 years old; Donor 3: woman, 20 years old) served as blood donors. Venous blood (5 mL) was collected into heparinized tubes and immediately processed. Briefly, blood was diluted 1/1 in pre-warmed RPMI 1640 medium. PBMCs were isolated using gradient centrifugation in Ficoll solution (Sigma; 300×g, 30 minutes). Isolated PBMCs were re-suspended in 1 volume of pre-warmed RPMI 1640 medium and centrifuged again (300×g, 30 minutes). PBMCs were re-suspended in RPMI 1640 medium supplemented with 10 % human serum (Sigma-Aldrich) at a concentration of $10^7$ cells per mL. Cells were seeded in 96 well microplate (100 $\mu$L per well). Test compounds (10 $\mu$g/mL). LPS (500 ng/mL) and interferon-y 1B (100 ng/mL) were added to PBMCs culture immediately after plating. After 24 hours, cell culture supernatants were collected and frozen at - 80°C for further processing.

*IL-10 ELISA assay*

IL-10 levels in cell culture supernatants were assayed using an ELISA human IL-10 kit (Ozyme®) according to the indications for use specified by the manufacturer.

*Statistical Analysis*

Simple comparisons between two groups were performed with Student's t test. Multiple comparisons against a single reference group were made by one-way analysis of variance followed by Dunnett's test when possible. When all pairwise comparisons were required, the Student-Newman-Keuls test was used. S.E.M. values were derived from at least three independent experiments.

*In vivo* experiments

*Animals*

Animals were treated in accordance with the Guide for the Care and Use of Laboratory Animals (National Research Council, 1996), with the European Directive 2010/63/EU, and the guidelines of the local institutional animal care and use committee. For all studies, 8 to 10 weeks-old male C57B1/6 mice (Janvier, France) were used. They were maintained on a 12:12 hours light/dark cycle with lights on at 8 a.m. The room temperature was kept at 20°C, with free access to standard diet and tap water.

*6-OHDA and MPTP mouse models*

*Experimental design*

Two *in vivo* mouse models were used: the 6-OHDA mouse model and the MPTP mouse model. For the 6-OHDA mouse model, the intoxication protocol was based on the unilateral stereotaxic intrastriatal injection of 6-OHDA in the right striatum. Intracerebral or intravenous injections of treatments were made either the day before the surgical stereotaxic procedure, concomitantly with 6-OHDA during the surgical stereotaxic procedure, or two days after 6-OHDA injection. Animals were sacrificed 8 days after the surgical stereotaxic procedure.

For the MPTP mouse model, 4 injections of MPTP (20 mg/kg) were made. Intravenous injection of anti-CD38 clone HB7 antibody was done 2 days before MPTP injection. Mice were sacrificed 7 days after MPTP injections.

*Surgical stereotaxic procedure*

For unilateral 6-OHDA mouse model, animals were anesthetized using Chloral hydrate (400 mg/kg, i.p.) and placed into a stereotactic frame adapted to mice. The injection was performed using a Hamilton syringe at the following coordinates: AP: + 0.85 cm, ML: $\pm$ 2 cm, DV: - 3.4 cm (corresponding to the atlas of Franklin and Paxinos, 1997). A total volume of 2.5 $\mu$L containing or not 5 $\mu$g of 6-OHDA diluted in PBS in the presence or the absence of test treatments was injected. The needle was left in place for 10 minutes after the injection before retraction.

*Tissue preparation*

Mice were sacrificed by cervical dislocation, and beheaded to collect brain and blood samples. The brain was fixed in a paraformaldehyde solution (4 %) during 1 day before being soaked in sucrose (30 %) during 2 days and then froze at -80°C for further analysis.

*Brain slicing, immunofluorescence and neurons enumeration*

Each brain was sliced using a freezing microtome at -30°C. The thickness of each slice was 20 $\mu$m. The slicing was done around the *substantia nigra* (80 slices from slice 51 to 63 according to Allen Mouse Brain).

Slices were then washed out in PBS and incubated with anti-Tyrosine Hydroxylase to stain dopaminergic neurons (Abcam®) during 2 days, at 4 °C, with agitation. Slices were incubated with corresponding secondary antibody in the presence of DAPI (Sigma Aldrich®) to stain cell's nuclei during 2 hours at room temperature, and mounted on gelatin-coated slides.

The slices were imaged using a Nikon TE2000 U equipped with a Hamamatsu ORCE-ER camera or with a Zeiss Axio Vert.A1 equipped with an axiocam 503 mono camera. Image analysis was done using Image J software or Zen (Zeiss®).

*IL-10 ELISA assay*

Blood samples collected in collection tube (Microvette® 500 EDTA-K3, SARSTEDT) were centrifuged at 3000 rotations per minute during 15 minutes. Supernatants (plasma) were collected and transferred into tubes and froze at -80°C for further analysis. IL-10 levels in plasma samples were assayed using an ELISA IL-10 kit (BioLegend®) according to the indications for use specified by the manufacturer.

*EAE mouse model*

*Experimental design*

Animals were anesthetized via inhalation with isoflurane (15 minutes). Mouse were immunized by subcutaneous injection of an emulsion containing 100 $\mu$g MOG peptide 35-55 in Complete Freund's Adjuvant and 500 $\mu$g HKMT (heat-killed *Mycobacterium tuberculosis)* on day 0. Two hours later, animals received an intraperitoneal injection of 500 ng of pertussis toxin on day 0 and day 2. Test compounds were injected intravenously 10 and 20 days post-emulsion subcutaneous injection. Animals were sacrificed at day 28.

*Clinical score evaluation*

After immunization, animals were daily monitored for weight and clinical symptoms. Clinical scores were graded according to the following scale: 0: no clinical signs; 0.5: partially limp tail; 1: paralyzed tail, normal gait; 1.5: paralyzed tail, uncoordinated movement, hind limb paresis; 2: one hind limb paralyzed or does not respond to pinch; 2.5: one hind limb paralyzed and weakness of the other; 3: two hind limbs paralyzed; 3.5: two hind limbs paralyzed and weakness of forelimbs; 4: moribund state.

*DSS mouse model*

*Experimental design*

Experimental colitis was induced in mice by giving drinking water ad libitum containing 3 % (w/v) DSS for 5 days, while control mice received tap water only. Test compounds were intravenously administered (15 mg/kg) when DSS treatment began. Mice were sacrificed 9 days after the beginning of DSS treatment.

*Statistical Analysis*

The statistical analyses and figures were made using the software Sigma Plot. Different tests may be performed depending on the data: One-way or two-way ANOVA and post hoc tests, if the conditions of application are respected.

**Example 1: Neuroprotection of midbrain dopaminergic neurons by sCD31 is mediated through interaction with CD38**

Protection against the mitochondrial neurotoxin MPP$^+$

We report here that sCD31 is neuroprotective in a situation in which DA cell death was caused by mitochondrial poisoning with 1-methyl-4-phenylpyridinium (MPP$^+$), the active metabolite of the DA neurotoxin 1-methyl-4-phenyl-1,2,3,6-tetrahydropyridine (MPTP).

For this purpose, spontaneously occurring DA cell death was prevented by a treatment combining depolarizing concentrations of K$^+$ (30 mM) and MK801 (5 $\mu$M), a glutamate receptor antagonist used to prevent unwanted excitotoxic insult. The cultures were then exposed to 3 $\mu$M MPP$^+$ between 5 and 7 days *in vitro* (DIV) to achieve a loss of approximately 50% of DA neurons. When the cultures were exposed to murine sCD31 (having SEQ ID NO: 18) during the intoxication period, MPP$^+$-induced DA cell loss was prevented by this compound **(Figure 1)** with an EC$_{50}$ of 36 ng/mL.

Of note, these effects are true, direct neuroprotective effects, and are not due to a possible interference with CD38 localized on immune cells (as illustrated in Hara-Yokoyama et al., 2008. Int Immunopharmacol. 8(1):59-70) since there are no peripheral immune cells in this *in vitro* cell culture setting.

The neuroprotective effect of sCD31 is antagonized in the presence of anti-CD31 antibody clone Moon-1 or antagonistic anti-CD38 antibodies (clone OKT-10 or AT13/5)

CD31 is known to interact with 3 ligands: CD31 itself, CD38 and the $\alpha v \beta_3$ integrin (Kalinowska & Losy, 2006. Eur J Neurol. 13(12):1284-90). However, CD31 is not expressed in neuronal cells (Williams et al., 1996. J Neurosci Res. 45(6):747-57). Interaction of CD31 with CD38 was previously shown to be antagonized in the presence of the anti-CD31 antibody clone Moon-1 or by antagonistic anti-CD38 antibodies (Deaglio et al., 1996. J Immunol. 156(2):727-34; Deaglio et al., 1998. J Immunol. 160(1):395-402).

To test whether the neuroprotective effect of sCD31 is mediated through CD38, we studied the neuroprotective effect of sCD31 (1 $\mu$g/mL) in the presence or the absence of anti-CD31 antibody clone Moon-1 (1 $\mu$g/mL) or antagonistic anti-CD38 antibodies (clone OKT10 or AT13/5, 1 $\mu$g/mL) in the previously described MPP$^+$ *in vitro* model.

We observed that the neuroprotective effect of sCD31 was antagonized in the presence of anti-CD31 antibody clone Moon-1 or of the antagonistic anti-CD38 antibodies clones OKT10 or AT13/5 **(Figure 2).**

The neuroprotective effect of sCD31 is thus indeed mediated through the interaction with CD38.

The neuroprotective effect of sCD31 is reproduced by agonistic anti-CD38 antibody (clone HB7)

Agonistic anti-CD38 antibodies are compounds that reproduce the biological effect of CD31 binding to CD38 (Deaglio et al., 1998. J Immunol. 160(1):395-402). Thus, like sCD31, agonistic anti-CD38 antibodies should also be neuroprotective.

We observed that an agonistic anti-CD38 antibody (clone HB7, 1 $\mu$g/mL) was able to protect DA neurons in the MPP$^+$ *in vitro* model, and that this neuroprotective effect was antagonized in the presence of antagonistic anti-CD38 antibodies (clone OKT10 or AT13/5, 1 $\mu$g/mL) **(Figure 3).**

The neuroprotective effect of sCD31 or of agonistic anti-CD38 antibodies requires tyrosine phosphorylation/activation of tyrosine kinase

As previously mentioned herein, CD38 is a multifunctional molecule including a receptor-mediated function through internalization, a tyrosine phosphorylation-mediated function and an enzyme-mediated function. Previous research demonstrated that agonistic (clone HB7) or antagonistic (clone OKT10 or AT13/5) anti-CD38 antibodies failed to modulate CD38 enzymatic activity *in vitro* (Deckert et al., 2014. Clin Cancer Res. 20(17):4574-83). Consequently, we wanted to determine whether sCD31 or agonistic anti-CD38 antibodies required tyrosine phosphorylation to mediate its neuroprotective effect, by using genistein, an inhibitor of tyrosine kinase.

We observed that, in the MPP$^+$ *in vitro* model, the neuroprotective effect of sCD31 (1 $\mu$g/mL) or of agonistic anti-CD38 antibodies (clone HB7, 1 $\mu$g/mL) was antagonized in the presence of the tyrosine kinase inhibitor genistein (GENI, 10 nM), suggesting that tyrosine phosphorylation is necessary to protect DA neurons **(Figure 4).**

The neuroprotective effect of sCD31 or of agonistic anti-CD38 antibodies is mediated through lysosomal exocytosis

One may assume that sCD31 and agonistic anti-CD38 antibodies could both protect dopaminergic neurons by acting at the CD38 level but through a different mechanism of action. Ruling out this possibility, we observed **(Figure 5)** that in the MPP$^+$ *in vitro* model, the neuroprotective effect of sCD31 (1 $\mu$g/mL) or of agonistic anti-CD38 antibody (clone HB7, 1 $\mu$g/mL) were both antagonized in the presence of the inhibitors of lysosomal maturation and of Ca$^{2+}$-dependent lysosomal exocytosis vacuolin-1 and endosidin2.

Agonistic anti-CD38 antibody (clone HB7) is neuroprotective *in vivo*

To determine whether agonistic anti-CD38 antibody (clone HB7) was also neuroprotective *in vivo,* we intravenously injected a 15 mg/kg dose of this antibody in a mouse model in which dopaminergic neurons of the *substantia nigra pars compacta* degenerate following repeated intraperitoneal injection of MPTP. MPTP has the ability to cross the blood brain barrier and is converted into MPP$^+$ by the astrocytes. MPP$^+$, which is an inhibitor of the mitochondrial complex I, is preferentially transported in dopaminergic neurons through the dopamine transporter, resulting in a specific cell death of dopaminergic population. This model has been extensively used as a test system for assessment of neuroprotective and neurorepair strategies (Dauer & Przedborski, 2003. Neuron. 39(6):889-909).

We observed that intravenous injection of agonistic anti-CD38 antibody (clone HB7) at a 15 mg/kg dose strongly protected dopaminergic neurons of the *substantia nigra pars compacta* from MPTP-induced neurodegeneration **(Figure 6).**

Conclusion

Altogether, the data presented in this example show that sCD31 is neuroprotective *in vitro,* an effect which is mediated through the interaction with CD38. This effect is antagonized in the presence of anti-CD31 antibodies or antagonistic anti-CD38 antibodies.

Additionally, it has been demonstrated that agonistic anti-CD38 antibodies, which mimic the binding of sCD31 to CD38, are also able to induce a neuroprotective effect, said effect being antagonized in the presence of antagonistic anti-CD38 antibodies.

Whether the neuroprotective effect is induced by sCD31 or by agonistic anti-CD38 antibodies, tyrosine phosphorylation/activation of tyrosine kinase has been shown to be necessary to induce said neuroprotective effect. Moreover, the resulting neuroprotective effect has been shown to be due to lysosomal maturation and Ca$^{2+}$-dependent lysosomal exocytosis.

These data therefore suggest a therapeutic role of sCD31 for the treatment of neurodegenerative and neuroinflammatory diseases.

**Example 2: *In vivo* effect of an agonistic anti-CD38 antibody on IL-10 levels**

To test whether the anti-inflammatory properties of agonistic anti-CD38 antibodies were also observe *in vivo,* we intravenously injected anti-CD38 antibody clone HB7 (15 mg/kg) to mice.

Eight days following injection, we observed a 6-fold increase in IL-10 levels **(Figure 7).**

Altogether, the data presented in this example show that agonistic anti-CD38 antibodies induce a strong release of IL-10 *in vivo,* suggesting a role in immunoregulation and against inflammation. Indeed, IL-10 is widely known to downregulate the expression of T$_h$1 cytokines, MHC class II antigens, and co-stimulatory molecules on macrophages; to enhance B cell survival, proliferation, and antibody production; to block NF-$\kappa$B activity; and to regulate the JAK-STAT signaling pathway.

Moreover, IL-10 has also been shown to be effective in treating cancer, in particular by inhibiting tumor metastasis (Sun et al., 2000. J Immunother. 23(2):208-14). Expression of IL-10 from transfected tumor cell lines in IL-10 transgenic mice (Groux et al., 1999. J Immunol. 162(3): 1723-9) or dosing with IL-10 leads to control of primary tumor growth and decreased metastatic burden (Fujii et al., 2001. Blood. 98(7):2143-51; Berman et al., 1996. J Immunol. 157(1):231-8).

Given the consistent findings of Example 1 on the equal effects of sCD31 and agonistic anti-CD38 antibodies, these data therefore suggest a therapeutic role of sCD31 for the treatment of inflammatory diseases and cancer.

**Example 3: Identification of agonistic anti-CD38 antibodies that competes with CD31 by phage display**

To generate new humanized antibodies that reproduce the biological effect of sCD31 on CD38, we used a phage display library of scFvs containing 1.5 billion sequences (Philibert et al., 2007. BMC Biotechnol. 7:81).

A first round of selection was done to identify scFvs that bind human CD38 extracellular domain **(Figure 8)**. Among 95 selected scFvs, 52 were confirmed to bind to CD38+ Jurkat T cells by FACS. These 52 validated binders were sequenced, leading to the identification of 16 non-redundant scFvs that bind CD38. These binders were cloned and the corresponding IgG were produced in CHO cells.

The 16 non-redundant IgGs were then tested (clones A02, B06, B08, B09, C05, C06, C08, D03, D04, D06, D07, D10, E03, E05, E08, F07), first in the MPP+ *in vitro* model (at 0.5 μg/mL for each clone), then by competition assay with the human extracellular domain of CD31 by Biacore on a panel of selected antibodies.

In the MPP+ *in vitro* model, several humanized anti-CD38 antibodies clones were found to be neuroprotective **(Figure 9).** In particular, 4 clones (B08, C05, D06 and D10) shows a stronger neuroprotective effect than that of the reference agonistic anti-CD38 antibody clone HB7 (0.5 μg/mL).

The neuroprotective effect of clones B08, C05, C06, D06 and D07 was antagonized in the presence of the lysosomal exocytosis inhibitors vacuolin-1 (VAC, 10 μM), suggesting that these antibodies reproduce the biological effect of sCD31 **(Figure 10).**

**Example 4: Neuroprotective properties of proprietary agonistic anti-CD38 antibodies in an *in vivo* neurodegenerative model**

We aimed at determining whether B08 or D06 anti-CD38 antibodies were neuroprotective *in vivo* against neurodegeneration induced by oxidative stress. Mouse were administered with 6-hydroxydopamine (6-OHDA) in the right striatum. Since 6-OHDA is an analogue of dopamine, it is transported in dopaminergic neurons through the dopamine transporter, resulting in a specific degeneration and loss of dopaminergic neurons of the right *substantia nigra pars compacta.* It has been extensively used as a test system for novel symptomatic agents and for assessment of neuroprotective and neurorepair strategies (Galindo et al., 2014. In Kostrzewa (Ed.), Handbook of neurotoxicity (1st Ed., pp. 639-651). New York: Springer-Verlag). Since 6-OHDA is injected unilaterally in the right striatum, left striatum remains unaffected and each mouse is its own control.

Mice were intracerebrally injected with 0.1 mg/kg of HB7, B08 and D06 anti-CD38 antibodies. We observed that at a concentration of 0.1 mg/kg injected intracerebrally (icv) concomitantly with 6-OHDA, B08 and D06 anti-CD38 antibodies statistically significantly protected dopaminergic neurons of the right *substantia nigra pars compacta* **(Figure 11).** We also observed that HB7 anti-CD38 antibody did fail to statistically significantly protect dopaminergic neurons.

Of interest, regarding B08 anti-CD38 antibody, this neuroprotective effect was also observed when injected intravenously (iv, 15 mg/kg) one day before 6-OHDA icv injection **(Figure 12).** Noticeably, the anti-CD38 antibody Daratumumab (DARA, 15 mg/kg iv) failed to demonstrate neuroprotective activity.

To understand whether B08 anti-CD38 antibody could also protect neurons when the neurodegenerative process is ongoing, we injected B08 anti-CD38 antibody (at 15 mg/kg iv) two days after 6-OHDA icv injection. We still observed strong neuroprotective activity of the B08 anti-CD38 antibody even after degeneration has started (Figure 13). This demonstrates the powerful neuroprotective effect of B08 anti-CD38 antibody.

As a conclusion, the neuroprotective effect of B08 and D06 anti-CD38 antibodies were demonstrated to be of superior efficacy than that obtained with HB7 anti-CD38 antibody. Noticeably, this neuroprotective effect is still observed when the compound is injected intravenously when the neurodegenerative process is ongoing.

Given the consistent findings of Example 1 on the equal effects of sCD31 and agonistic anti-CD38 antibodies, these data therefore suggest a therapeutic role of sCD31 for the treatment of neurodegenerative diseases.

**Example 5: Anti-inflammatory properties of proprietary agonistic anti-CD38 antibodies *in vitro***

To determine whether agonistic anti-CD38 antibodies (clone B08, C05, D06 and D07) possess anti-inflammatory properties, we assessed their effect on the release of the anti-inflammatory cytokine IL-10 *in vitro* using human peripheral blood mononuclear cells (PBMCs) from 3 healthy donors. We observed that anti-CD38 antibodies (clone B08, C05, D06 and D07) all statistically significantly increased IL-10 release, thus demonstrating anti-inflammatory properties **(Figure 14).** Of importance, HB7 failed to statistically significantly increase IL-10 release in culture human PBMCs in this assay, demonstrating the higher anti-inflammatory effect of the B08, C05, D06 and D07 anti-CD38 antibodies over the HB7 anti-CD38 antibody.

As a conclusion, agonistic anti-CD38 antibodies, as represented by clones B08, C05, D06 and D07, increase IL-10 release from PBMCs *in vitro.* In addition, because IL-10 is involved in the general mechanism of inflammation and has been reported in numerous auto-immune diseases, agonistic anti-CD38 antibodies, as represented by clones B08, C05, D06 and D07, provide a new strategy to treat and/or prevent inflammatory and/or auto-immune diseases.

Given the consistent findings of Example 1 on the equal effects of sCD31 and agonistic anti-CD38 antibodies, these data therefore suggest a therapeutic role of sCD31 for the treatment of inflammatory diseases.

**Example 6: Neuroprotective effect of proprietary agonistic anti-CD38 antibody in an experimental autoimmune encephalomyelitis (EAE) mouse model of multiple sclerosis (MS), an auto-immune, neuro-inflammatory disease**

Multiple sclerosis (MS) is a progressive inflammatory and demyelinating disease of the human central nervous system (CNS) in which an important role for the immune system in the pathogenesis of the disease is suspected (Lassmann, 2008. J Neurol Sci. 274(1-2):45-7). EAE is an induced disease in mice that affects the central nervous system and constitutes a model for human MS. After a subcutaneous injection of an emulsion with a Complete Freund's Adjuvant (CFA) mixed with oligodendrocyte membrane proteins like myelin oligodendrocyte glycoprotein (MOG), an immune response starts in the periphery and, within 2 weeks, causes inflammation in the CNS. In the same time, we can observe, a progressive ascending paralysis (tail to hindlimb to forelimb). To determine whether B08 anti-CD38 antibody could have beneficial effect in this disease, we injected this antibody (15 mg/kg at symptom onset (10 days after MOG injection) and at day 20). We observed that B08 anti-CD38 antibody significantly reduced the clinical score compared with PBS-injected mice **(Figure 15).**

As a conclusion, agonistic anti-CD38 antibodies (clone B08) ameliorate the clinical score in the EAE mouse model of the auto-immune, neuro-inflammatory disease MS when injected intravenously at symptom onset.

Given the consistent findings of Example 1 on the equal effects of sCD31 and agonistic anti-CD38 antibodies, these data therefore suggest a therapeutic role of sCD31 for the treatment of auto-immune, neuro-inflammatory diseases.

**Example 7: Anti-inflammatory properties of a proprietary agonistic anti-CD38 antibody *in vivo,* in a dextran sulfate sodium (DSS)-induced colitis mouse model**

To demonstrate the effect of B08 agonistic anti-CD38 antibody in a purely inflammatory model, we decided to test its effects in the Dextran Sulfate Sodium (DSS)-induced colitis mouse model. Administration of DSS causes human ulcerative colitis-like pathologies due to its toxicity to colonic epithelial cells, which results in compromised mucosal barrier function. Clinical observations similar to human pathologies, such as weight loss, diarrhea and occult blood in stool, are commonly observed in the DSS model. Moreover, diminution of the colon's length is characteristic of this model. Importantly, studies suggest that DSS colitis mostly involves activation of lymphocytes, neutrophils and macrophages (Eichele and Kharbanda, 2017. WorldJ Gastroenterol. 23(33):6016-29).

We observed that administration of B08 anti-DC38 antibody (15 mg/kg, iv) statistically significantly repressed reduction of the diminution of the colon's length **(Figure 16).**

As a conclusion, agonistic anti-CD38 antibody (clone B08) increases colon length in the DSS mouse model of inflammatory bowel disease.

Given the consistent findings of Example 1 on the equal effects of sCD31 and agonistic anti-CD38 antibodies, these data therefore suggest a therapeutic role of sCD31 for the treatment of inflammatory diseases.

**Example 8: Identification of CD31 peptides binding to CD38**

The study was conducted at Pepscan Presto BV (Lelystad, The Netherlands). The aim was to identify epitopes of sCD31 specifically binding to CD38, thereby shunting endogenous CD31 interaction with CD38.

The concept of mapping linear epitopes using libraries of overlapping synthetic peptides is described in Geysen et al., 1984. Proc Natl Acad Sci USA. 81(13):3998-4002. Briefly, a library of linear, overlapping peptides of sCD31 were synthetized on a solid support as described further below. Considering that a majority of biomolecules recognize conformational or discontinuous epitopes, CLIPS (Chemically Linked Peptides on Scaffolds) technology was used to structurally fix peptides of sCD31 into defined 3-D structures, as described in Timmerman et al., 2007. J Mol Recognit. 20(5):283-299. Briefly, the CLIPS reaction takes place between bromo groups of the CLIPS scaffold and thiol sidechains of cysteines introduced into peptide constructs. Using this chemistry, native protein sequences are transformed into CLIPS constructs with a range of different structures including single mP2 loops, stabilized beta sheets, alpha helices, T3 double loops, etc.

Material and methods

*Design of the peptides*

Different sets of sCD31 peptides were synthesized according to the following designs. Note that actual order of peptides on mini-cards in some was randomized.

- **Set #1**
Mimic type: linear
Label: LIN15
Linear peptides of 15 consecutive amino acid residues in length, derived from the target sequence of sCD31 with an offset of 1.5 residues.

- **Set #2**
Mimic type: single loop (mP2 CLIPS)
Label: LOOP7
Constrained peptides of 9 amino acid residues in length. On positions 2-8 are incorporated 7 consecutive amino acid residues derived from the target sequence of sCD31 with an offset of 1.3 residues. Cys residues were inserted on positions 1 and 9 and joined by mP2 CLIPS in order to create a loop mimic. Native Cys residues, if present, were replaced by Ser residues.

- **Set #3**
Mimic type: single loop, (mP2 CLIPS)
Label: LOOP15
Constrained peptides of 17 amino acid residues in length. On positions 2-16 are incorporated 15 consecutive amino acid residues derived from the target sequence of sCD31 with an offset of 1.2 residues. Cys residues were inserted on positions 1 and 17 and joined by mP2 CLIPS in order to create a loop mimic. Native Cys residues, if present, were replaced by Ser residues.

- **Set #4**
Mimic type: β-turn peptide mimics (mP2 CLIPS)
Label: BET
β-turn peptide mimics of 22 amino acid residues in length. On positions 2-21 are incorporated 20 consecutive amino acid residues derived from the target sequence of sCD31 with an offset of 1.3 residues. Residues on positions 11 and 12 are replaced by a Pro-Gly motif in order to induce the β-turn formation. Cys residues were inserted on positions 1 and 22 and joined by mP2 CLIPS in order to stabilize the mimic. Native Cys residues, if present, were replaced by Ser residues.

*Peptide synthesis*

To reconstruct epitopes of sCD31, a library of peptide-based peptide mimics was synthesized using Fmoc-based solid-phase peptide synthesis. An amino functionalized polypropylene support was obtained by grafting with a hydrophilic polymer formulation, followed by reaction with *t*-butyloxycarbonyl-hexamethylenediamine (BocHMDA) using dicyclohexylcarbodiimide (DCC) with *N*-hydroxybenzotriazole (HOBt) and subsequent cleavage of the Boc-groups using trifluoroacetic acid (TFA).

Standard Fmoc-peptide synthesis was used to synthesize peptides on the amino-functionalized solid support by custom modified JANUS liquid handling stations (Perkin Elmer).

Synthesis of structural mimics was done using CLIPS technology. CLIPS technology allows to structure peptides into single loops, double loops, triple loops, sheet-like folds, helix-like folds, and combinations thereof. CLIPS templates are coupled to cysteine residues. The side-chains of multiple cysteines in the peptides are coupled to one or two CLIPS templates. For example, a 0.5 mM solution of the P2 CLIPS (2,6-bis(bromomethyl)pyridine) is dissolved in ammonium bicarbonate (20 mM, pH 7.8)/acetonitrile (1:3, v/v). This solution is added onto the peptide arrays. The CLIPS template binds to side-chains of two cysteines as present in the solid-phase bound peptides of the peptide-arrays (455 wells plate with 3-μL wells). The peptide arrays are gently shaken in the solution for 30 to 60 minutes while completely covered in solution.

Finally, the peptide arrays are washed extensively with excess of $H_2O$ and sonicated in disrupt-buffer containing 1 % SDS / 0.1 % 2,2'-(ethylenedioxy)diethanethiol in PBS (pH 7.2) at 70°C for 30 minutes, followed by sonication in $H_2O$ for another 45 minutes.

The T3 CLIPS carrying peptides were made in a similar way but now with three cysteines.

*ELISA screening*

The binding of the sample (recombinant His-tagged CD38; 9 μg/mL and 20 μg/mL) to each of the synthesized peptides was tested in a pepscan-based ELISA. The peptide arrays were incubated with primary sample solution (overnight at 4°C). After washing, the peptide arrays were incubated with a 1/1000 dilution of an appropriate antibody peroxidase conjugate (rabbit anti-mouse IgG(H+L) HRP conjugate, Southern Biotech, Cat. No 6175-05; anti-His tag HRP, Novagen, Cat. No 70796-3) for 1 hour at 25°C.

After washing, the peroxidase substrate 2,2'-azino-di-3-ethylbenzthiazoline sulfonate (ABTS) and 20 μL/mL of 3 % $H_2O_2$ were added.

After one hour, the color development was measured. The color development was quantified with a charge coupled device (CCD) camera and an image processing system. The values obtained from the CCD camera range from 0 to 3000 mAU, similar to a standard 96-well plate ELISA-reader.

Results

To extract the CD31 epitope(s) responsible for CD38 binding, initial experiments were carried out using recombinant CD38 fused to a mouse IgG2a Fc tail for detection. Unfortunately, results were ambiguous due to considerable overlap with a mouse IgG2a isotype control. While CD38 binding may occur, this was possibly obscured by the Fc binding.

Therefore, current experiments were carried out with a recombinant His-tagged CD38. As no Fc tag was present, it is expected that the main interaction(s) would occur through the CD38 protein. Several binding peaks were found with linear and conformational mapping. In linear mapping (LIN15), we observe one main peak and several smaller peaks **(Figure 17A)**. Similar peaks were observed in the conformational array (LOOP7, **Figure 17B;** LOOP15, **Figure 17C;** BET, **Figure 17D).**

A blank screening without sample but still using anti-His HRP for detection, did not show any binding. This suggests that all binding peaks that were observed are directly related to the His-tagged CD38 sample binding to CD31 peptides.

For determination of the binding site, the sample with the highest signal was used. The sequences with overlapping peptides are listed in **Table 4.**

**Table 4:** list of sCD31 epitope candidates (for each of the four sets LIN15, LOOP7, LOOP15 and BET) found for His-tagged CD38. Peaks were selected if the top peptides are at least 3 times the 20th percentile value of the mimic type (in bold). Core binding sequences (underlined) are based on common sequences in overlapping peptides (overlapping sequences within 30 % of the top peptide intensity in the peak). **: based on recurring peaks in other screenings.

| Mimic type | SEQ ID NO: 1 residues | | Epitope candidate | Value |
| | Start | End | | |
|---|---|---|---|---|
| | 68 | 82 | KPQHQMLFYKDDVLF | 355 |
| | 70 | 84 | QHQMLFYKDDVLFYN | **460** |
| | 71 | 85 | HQMLFYKDDVLFYNI | **441** |
| | 73 | 87 | MLFYKDDVLFYNISS | **451** |
| | 74 | 88 | LFYKDDVLFYNISSM | 383 |
| **LIN15** | 178 | 192 | KREKNSRDQNFVILE | **440** |
| | 179 | 193 | REKNSRDQNFVILEF | **453** |
| | 181 | 195 | KNSRDQNFVILEFPV | **502** |
| | 182 | 196 | NSRDQNFVILEFPVE | **1230** |
| | 184 | 198 | RDQNFVILEFPVEEQ | **1021** |
| | 185 | 199 | DQNFVILEFPVEEQD | **963** |

(continued)

| Mimic type | SEQ ID NO: 1 residues | | Epitope candidate | Value |
| --- | --- | --- | --- | --- |
| | Start | End | | |
| | 187 | 201 | NFVILEFPVEEQDRV | **631** |
| | 308 | 322 | SSRISKVSSIVVNIT | 168 |
| | 310 | 324 | RISKVSSIVVNITEL | **572** |
| | 311 | 325 | ISKVSSIVVNITELF | **642** |
| | 313 | 327 | KVSSIVVNITELFSK | 155 |
| ** | 457 | 471 | NSNDPAVFKDNPTED | 421 |
| | 458 | 472 | SNDPAVFKDNPTEDV | 410 |
| | 460 | 474 | DPAVFKDNPTEDVEY | 402 |
| | 461 | 475 | PAVFKDNPTEDVEYQ | 333 |
| | 463 | 477 | VFKDNPTEDVEYQCV | 303 |
| | 559 | 573 | KQKASKEQEGEYYCT | 337 |
| | 560 | 574 | QKASKEQEGEYYCTA | 316 |
| | 562 | 576 | ASKEQEGEYYCTAFN | 400 |
| | 563 | 577 | SKEQEGEYYCTAFNR | **454** |
| | 565 | 579 | EQEGEYYCTAFNRAN | 340 |

(continued)

| Mimic type | SEQ ID NO: 1 residues | | Epitope candidate | Value |
|---|---|---|---|---|
| | Start | End | | |
| LOOP7 | 52 | 58 | CNLTLQSFC | 370 |
| | 54 | 60 | CTLQSFADC | 427 |
| | 55 | 61 | CLQSFADVC | **561** |
| | 56 | 62 | CQSFADVSC | 413 |
| | 57 | 63 | CSFADVSTC | 390 |
| | 73 | 79 | CMLFYKDDC | **542** |
| | 74 | 80 | CLFYKDDVC | **604** |
| | 76 | 82 | CYKDDVLFC | **585** |
| | 77 | 83 | CKDDVLFYC | **579** |
| | 78 | 84 | CDDVLFYNC | **702** |
| | 80 | 86 | CVLFYNISC | 502 |
| | 89 | 95 | CKSTESYFC | 339 |
| | 90 | 96 | CSTESYFIC | **668** |
| | 91 | 97 | CTESYFIPC | **549** |
| | 93 | 99 | CSYFIPEVC | 400 |
| | 94 | 100 | CY+RC | **523** |
| | 95 | 101 | CFIPEVRIC | 414 |
| | 98 | 104 | CEVRIYDSC | **642** |
| | 99 | 105 | CVRIYDSGC | 479 |
| | 100 | 106 | CRIYDSGTC | 395 |
| | 120 | 126 | CTAEYQVLC | 492 |
| | 121 | 127 | CAEYQVLVC | 508 |
| | 122 | 128 | CEYQVLVEC | 651 |
| | 185 | 191 | CDQNFVILC | **628** |
| | 186 | 192 | CQNFVILEC | **942** |
| | 187 | 193 | CNFVILEFC | **1186** |

(continued)

| Mimic type | SEQ ID NO: 1 residues | | Epitope candidate | Value |
|---|---|---|---|---|
| | **Start** | **End** | | |
| | 189 | 195 | CVILEFPVC | **527** |
| | 190 | 196 | CILEFPVEC | **537** |
| | 263 | 269 | CLAQEFPEC | 412 |
| | 264 | 270 | CAQEFPEIC | 429 |
| | 265 | 271 | CQEFPEIIC | 476 |
| | 267 | 273 | CFPEIIIQC | **596** |
| | 315 | 321 | CSSIVVNIC | 364 |
| | 316 | 322 | CSIVVNITC | 387 |
| | 317 | 323 | CIVVNITEC | **630** |
| | 319 | 325 | CVNITELFC | **625** |
| | 320 | 326 | CNITELFSC | 512 |
| | 412 | 418 | CRISYDAQC | 287 |
| | 414 | 420 | CSYDAQFEC | **570** |
| | 415 | 421 | CYDAQFEVC | **628** |
| | 416 | 422 | CDAQFEVIC | 429 |
| | 467 | 473 | CNPTEDVEC | 402 |
| | 468 | 474 | CPTEDVEYC | 407 |
| | 470 | 476 | CEDVEYQSC | **804** |
| | 471 | 477 | CDVEYQSVC | **556** |
| | 501 | 507 | CDEVQISIC | 378 |
| | 502 | 508 | CEVQISILC | **529** |
| | 503 | 509 | CVQISILSC | 416 |
| | 514 | 520 | CESGEDIVC | 420 |
| | 515 | 521 | CSGEDIVLC | **573** |
| | 516 | 522 | CGEDIVLQC | 442 |
| | 518 | 524 | CDIVLQSAC | 389 |
| | 564 | 570 | CKEQEGEYC | 385 |
| | 566 | 572 | CQEGEYYSC | **619** |
| | 567 | 573 | CEGEYYSTC | **650** |
| | 568 | 574 | CGEYYSTAC | **552** |
| | 570 | 576 | CYYSTAFNC | 477 |

(continued)

| Mimic type | SEQ ID NO: 1 residues | | Epitope candidate | Value |
|---|---|---|---|---|
| | Start | End | | |
| LOOP15 | 66 | 80 | CHVKPQHQMLFYKDDVC | 431 |
| | 67 | 81 | CVKPQHQMLFYKDDVLC | 411 |
| | 68 | 82 | CKPQHQMLFYKDDVLFC | 467 |
| | 70 | 84 | CQHQMLFYKDDVLFYNC | **600** |
| | 71 | 85 | CHQMLFYKDDVLFYNIC | **564** |
| | 72 | 86 | CQMLFYKDDVLFYNISC | **646** |
| | 73 | 87 | CMLFYKDDVLFYNISSC | **630** |
| | 74 | 88 | CLFYKDDVLFYNISSMC | 497 |
| | 80 | 94 | CVLFYNISSMKSTESYC | 371 |
| | 82 | 96 | CFYNISSMKSTESYFIC | **552** |

(continued)

| Mimic type | SEQ ID NO: 1 residues | | Epitope candidate | Value |
|---|---|---|---|---|
| | **Start** | **End** | | |
| | 83 | 97 | CYNISSMKSTESYFIPC | 370 |
| | 84 | 98 | CNISSMKSTESYFIPEC | 478 |
| | 85 | 99 | CISSMKSTESYFIPEVC | 499 |
| | 86 | 100 | CSSMKSTESYFIPEVRC | **343** |
| | 88 | 102 | CMKSTESYFIPEVRIYC | 325 |
| | 89 | 103 | CKSTESYFIPEVRIYDC | 528 |
| | 90 | 104 | CSTESYFIPEVRIYDSC | **660** |
| | 91 | 105 | CTESYFIPEVRIYDSGC | 465 |
| | 92 | 106 | CESYFIPEVRIYDSGTC | 373 |
| | 94 | 108 | CYFIPEVRIYDSGTYKC | 416 |
| | 178 | 192 | CKREKNSRDQNFVILEC | 516 |
| | 179 | 193 | CREKNSRDQNFVILEFC | **1073** |
| | 180 | 194 | CEKNSRDQNFVILEFPC | **566** |
| | 181 | 195 | CKNSRDQNFVILEFPVC | **624** |
| | 182 | 196 | CNSRDQNFVILEFPVEC | **965** |
| | 184 | 198 | CRDQNFVILEFPVEEQC | **1110** |
| | 185 | 199 | CDQNFVILEFPVEEQDC | **634** |
| | 186 | 200 | CQNFVILEFPVEEQDRC | **729** |
| | 187 | 201 | CNFVILEFPVEEQDRVC | 524 |
| | 188 | 202 | CFVILEFPVEEQDRVLC | **550** |
| | 256 | 270 | CSTIQVTHLAQEFPEIC | 320 |
| | 257 | 271 | CTIQVTHLAQEFPEIIC | 504 |
| | 258 | 272 | CIQVTHLAQEFPEIIIC | **797** |
| | 259 | 273 | CQVTHLAQEFPEIIIQC | **617** |
| | 260 | 274 | CVTHLAQEFPEIIIQKC | 343 |
| | 308 | 322 | CSSRISKVSSIVVNITC | 287 |
| | 310 | 324 | CRISKVSSIVVNITELC | **567** |
| | 311 | 325 | CTSKVSSIVVNITELFC | **795** |
| | 312 | 326 | CSKVSSIVVNITELFSC | **593** |
| | 313 | 327 | CKVSSIVVNITELFSKC | 211 |

(continued)

| Mimic type | SEQ ID NO: 1 residues | | Epitope candidate | Value |
|---|---|---|---|---|
| | Start | End | | |
| BET | 63 | 82 | CTTSHVKPQHPGLFYKDDVLFC | **679** |
| | 64 | 83 | CTSHVKPQHQPGFYKDDVLFYC | **725** |
| | 65 | 84 | CSHVKPQHQMPGYKDDVLFYNC | **662** |
| | 67 | 86 | CVKPQHQMLFPGDDVLFYNISC | **792** |
| | 68 | 87 | CKPQHQMLFYPGDVLFYNISSC | **641** |
| | 76 | 95 | CYKDDVLFYNPGSMKSTESYFC | 378 |
| | 77 | 96 | CKDDVLFYNIPGMKSTESYFIC | **695** |
| | 78 | 97 | CDDVLFYNISPGKSTESYFIPC | 524 |
| | 80 | 99 | CVLFYNISSMPGTESYFIPEVC | **632** |
| | 173 | 192 | CKMVKLKREKPGRDQNFVILEC | **897** |
| | 174 | 193 | CMVKLKREKNPGDQNFVILEFC | **1497** |
| | 176 | 195 | CKLKREKNSRPGNFVILEFPVC | **833** |
| | 177 | 196 | CLKREKNSRDPGFVILEFPVEC | **817** |
| | 304 | 323 | CSKVESSRISPGSSIVVNITEC | **839** |
| | 306 | 325 | CVESSRISKVPGIVVNITELFC | **943** |
| | 307 | 326 | CESSRISKVSPGVVNITELFSC | **637** |
| | 353 | 372 | CPPANFTIQKPGTIVSQTQDFC | 739 |
| | 354 | 373 | CPANFTIQKEPGIVSQTQDFTC | 461 |
| | 401 | 420 | CQIVVSEMLSPGRISYDAQFEC | 417 |
| | 402 | 421 | CIVVSEMLSQPGISYDAQFEVC | 414 |
| | 403 | 422 | CVVSEMLSQPPGSYDAQFEVIC | **635** |
| | 488 | 507 | CLSEVLRVKVPGPVDEVQISIC | 601 |
| | 489 | 508 | CSEVLRVKVIPGVDEVQISILC | **649** |
| | 490 | 509 | CEVLRVKVIAPGDEVQISILSC | 447 |

Conclusion

Pepscan analysis identified several CD31 epitopes binding to CD38, listed in **Table 5.** These sCD31 peptides represent promising hits for binding to CD38 and mediating the therapeutic effects observed in Examples 1-7 above.

**Table 5.** List of main CD31 epitopes identified in this study.

| SEQ ID NO: 1 residues | | Epitope candidate |
|---|---|---|
| Start | End | |
| 74 | 82 | LFYKDDVLF |
| 90 | 104 | TESYFIPEVRIYDS |
| 122 | 126 | EYQVL |
| 174 | 196 | MVKLKREKNSRDQNFVILEFPVE |
| 259 | 272 | QVTHLAQEFPEIII |
| 307 | 324 | ESSRISKVSSIVVNITEL |

(continued)

| SEQ ID NO: 1 residues | | Epitope candidate |
|---|---|---|
| Start | End | |
| 403 | 411 | VVSEMLSQP |
| 416 | 420 | DAQFE |
| 463 | 476 | VFKDNPTEDVEYQS |

### Example 9

Cryopreserved human PBMCs (BioIVT) were thawed according to manufacturer's instructions and cultured (100,000 cells per well) using RPMI 1640 supplemented with 10% FBS. One hour after plating, cells were incubated with LPS (500 ng/mL) and interferon-γ 1B (100 ng/mL) in the presence or the absence of peptides of interest (**Table 6**; 50 μg/mL).

After 24 hours, 10 μL of cell culture medium were collected. IL-10 levels in cell culture medium were assayed using a human IL-10 ELISA kit according to manufacturer's instructions.

**Table 6.**

| SEQ ID NO: 1 residues | | Amino acid sequence | Structure | SEQ ID NO: |
|---|---|---|---|---|
| Start | Stop | | | |
| 90 | 96 | STESYFI | Linear | 4 |
| 90 | 96 | CSTESYFIC | Loop | 5 |
| 182 | 196 | NSRDQNFVILEFPVE | Linear | 6 |
| 187 | 193 | NFVILEF | Linear | 7 |
| 187 | 193 | CNFVILEFC | Loop | 8 |
| 184 | 198 | CRDQNFVILEFPVEEQC | Loop | 9 |
| 174 | 193 | CMVKLKREKNPGDQNFVILEFC | β-sheet-like fold | 10 |
| 195 | 201 | CVEEQDRVC | Loop | 11 |
| 317 | 325 | CIVVNITELFC | Loop | 12 |
| 306 | 322 | CVESSRISKVPGIVVNITELFC | β-sheet-like fold | 13 |
| 353 | 372 | CPPANFTIQKPGTIVSQTQDFC | β-sheet-like fold | 14 |
| 470 | 475 | CEDVEYQC | Loop | 15 |
| 489 | 508 | CSEVLRVKVIPGVDEVQISILC | β-sheet-like fold | 16 |
| 582 | 589 | RVILAPWK | Linear | 17 |

**Claims**

1. An isolated peptide which specifically binds to human CD38 having SEQ ID NO: 3 and which comprises at most 25 contiguous amino acid residues from human CD31 having SEQ ID NO: 2 or a variant thereof.

2. The isolated peptide according to claim **1,** wherein said isolated peptide is any one of the peptides of **Table 1.**

3. The isolated peptide according to claim **1** or **2,** wherein said isolated peptide is a 2-D structured peptide, preferably said isolated peptide is cyclized and/or beta-turned.

4. The isolated peptide according to claim **3,** wherein the 2-D structured peptide is any one of the peptides of **Table 1,** wherein:

   a)

      (i) a cysteine residue is inserted at both N-terminal and C-terminal extremities; and
      (ii) any cysteine residue within the isolated peptide sequence is either protected or mutated to another amino acid residue, preferably to serine; and/or

   b) a Pro-Gly or Asp-Gly dipeptide is inserted at a position which is central in said isolated peptide.

5. The isolated peptide according to any one of claims **1** to **4,** selected from the group comprising SEQ ID NOs:4 to 15, preferably SEQ ID NOs: 4 to 13, more preferably SEQ ID NOs: 6 to 10.

6. The isolated peptide according to any one of claims **1** to **5,** fused to a payload being a therapeutic or diagnostic payload and/or a carrier payload.

7. A nucleic acid encoding the isolated peptide according to any one of claims **1** to **6.**

8. An expression vector comprising the nucleic acid according to claim **7.**

9. A composition comprising at least one isolated peptide according to any one of claims **1** to **6.**

10. The isolated peptide according to any one of claims **1** to **6,** for use in preventing and/or treating a disease selected from inflammatory diseases; autoimmune diseases; metabolic and endocrine diseases; neurodegenerative diseases; neuroinflammatory diseases; ocular diseases; age-related diseases; and cancer and metastasis; in a subject in need thereof.

11. The isolated peptide for use according to claim **10,** wherein said disease is selected from multiple sclerosis; rheumatoid arthritis; systemic lupus erythematosus; diabetes; obesity; non-alcoholic steatohepatitis; amyotrophic lateral sclerosis; Parkinson's disease and related disorders; Alzheimer's disease and related disorders; Huntington disease; age-related macular degeneration; and glaucoma.

12. The isolated peptide according to any one of claims **1** to **6,** for use in increasing the level of at least one anti-inflammatory cytokine in a subject in need thereof, in particular in the blood of said subject.

13. The isolated peptide for use according to claim **12,** wherein said anti-inflammatory cytokine is interleukin-10 (IL-10).

14. The isolated peptide according to any of claims **1** to **6,** for use in lowering glucose levels in a subject in need thereof, in particular in the blood of said subject.

FIG. 1

FIG. 2

**FIG. 3**

**FIG. 4**

FIG. 5

FIG. 6

FIG. 7

FIG. 8

FIG. 9

FIG. 10

FIG. 11

FIG. 12

FIG. 13

FIG. 14

D0      D2      D10      D20      D28

MOG and    2nd Pertussis    PBS, B8 (15 mg/kg)    PBS, B8 (15 mg/kg)    Sacrifice
1st Pertussis    Toxin injection    iv injection    iv injection
Toxin injection

**FIG. 15A**

**FIG. 15B**

**FIG. 16A**

**FIG. 16B**

FIG. 17A

FIG. 17B

FIG. 17C

**FIG. 17D**

Europäisches
Patentamt

European
Patent Office

Office européen
des brevets

**EUROPEAN SEARCH REPORT**

Application Number

EP 20 30 5843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WONG M-X ET AL: "Proteolytic cleavage of platelet endothelial cell adhesion molecule-1 (PECAM-1/CD31) is regulated by a calmodulin-binding motif", FEBS LETTERS, ELSEVIER, AMSTERDAM, NL, vol. 568, no. 1-3, 18 June 2004 (2004-06-18), pages 70-78, XP004516410, ISSN: 0014-5793, DOI: 10.1016/J.FEBSLET.2004.04.094 * abstract * * figure 4 * | 1-14 | INV. C07K14/435 A61K38/17 |
| X | WO 2019/020643 A1 (ENCEFA [FR]) 31 January 2019 (2019-01-31) | 1,9 | |
| A | * page 8, line 15 - line 19 * * examples 1,4 * | 2-8, 10-14 | |
| Y | DEAGLIO S ET AL: "Human CD38 (ADP-ribosyl cyclase) is a counter-receptor of CD31, an Ig superfamily member.", JOURNAL OF IMMUNOLOGY (BALTIMORE, MD. : 1950) 01 JAN 1998, vol. 160, no. 1, 1 January 1998 (1998-01-01), pages 395-402, XP55757728, ISSN: 0022-1767 * the whole document * | 1-14 | |

-/--

TECHNICAL FIELDS SEARCHED (IPC)

C07K
A61K

The present search report has been drawn up for all claims

| Place of search | Date of completion of the search | Examiner |
|---|---|---|
| Munich | 8 December 2020 | Voigt-Ritzer, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 30 5843

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | TIBOR KRISTIAN ET AL: "Mitochondrial dysfunction and nicotinamide dinucleotide catabolism as mechanisms of cell death and promising targets for neuroprotection", JOURNAL OF NEUROSCIENCE RESEARCH., vol. 89, no. 12, 1 December 2011 (2011-12-01), pages 1946-1955, XP055757769, US ISSN: 0360-4012, DOI: 10.1002/jnr.22626 * last sentence * ----- | 1-14 | |

TECHNICAL FIELDS
SEARCHED (IPC)

The present search report has been drawn up for all claims

| Place of search | Date of completion of the search | Examiner |
|---|---|---|
| Munich | 8 December 2020 | Voigt-Ritzer, Heike |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 20 30 5843

This annex lists the patent family members relating to the patent documents cited in the above-mentioned European search report.
The members are as contained in the European Patent Office EDP file on
The European Patent Office is in no way liable for these particulars which are merely given for the purpose of information.

08-12-2020

| Patent document cited in search report | | Publication date | Patent family member(s) | | Publication date |
|---|---|---|---|---|---|
| WO 2019020643 | A1 | 31-01-2019 | AU | 2018308649 A1 | 13-02-2020 |
| | | | CA | 3070756 A1 | 31-01-2019 |
| | | | CN | 111108127 A | 05-05-2020 |
| | | | EP | 3434692 A1 | 30-01-2019 |
| | | | EP | 3658586 A1 | 03-06-2020 |
| | | | JP | 2020528447 A | 24-09-2020 |
| | | | WO | 2019020643 A1 | 31-01-2019 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017100305 A

### Non-patent literature cited in the description

- **MALAVASI et al.** *Physiol Rev.,* 2008, vol. 88 (3), 841-86
- **CHINI.** *Curr Pharm Des.,* 2009, vol. 15 (1), 57-63
- **VERDIN.** *Science,* 2015, vol. 350 (6265), 1208-13
- **FUNARO et al.** *J Immunol.,* 1990, vol. 145 (8), 2390-6
- **DEAGLIO et al.** *J Immunol.,* 1998, vol. 160 (1), 395-402
- **NEWMAN ; NEWMAN.** *Arterioscler Thromb Vasc Biol.,* 2003, vol. 23, 953-964
- **NEWMAN.** *J Clin Invest.,* 1997, vol. 99 (1), 3-8
- **KALINOWSKA ; LOSY.** *Eur J Neurol.,* 2006, vol. 13 (12), 1284-90
- **WANG et al.** *Am J Physiol Heart Circ Physiol.,* 2003, vol. 284 (3), H1008-17
- **ILAN ; MADRI.** *Curr Opin Cell Biol.,* 2003, vol. 15 (5), 515-24
- **ILAN et al.** *FASEB J.,* 2001, vol. 15 (2), 362-72
- **FENG et al.** *Eur Rev Med Pharmacol Sci.,* 2016, vol. 20 (19), 4082-4088
- **KJAERGAARD et al.** *APMIS,* 2016, vol. 124 (10), 846-55
- **MULLER et al.** *J Exp Med.,* 1993, vol. 178 (2), 449-60
- **HORENSTEIN et al.** *Biochem J.,* 1998, vol. 330 (3), 1129-35
- **GOLDBERGER et al.** *J Biol Chem.,* 1994, vol. 269 (25), 17183-17191
- **FORNASA et al.** *J Immunol.,* 2010, vol. 184 (10), 5485-5492
- **MALAVASI et al.** *Physiological Review,* 2008, vol. 88, 841-886
- **STROHL.** *BioDrugs.,* 2015, vol. 29 (4), 215-239
- **NEEDLEMAN ; WUNSCH.** *J Mol Biol.,* 1970, vol. 48 (3), 443-53
- **SMITH ; WATERMAN.** *Adv Appl Math.,* 1981, vol. 2 (4), 482-9
- **GRIBSKOV ; BURGESS.** *Nucleic Acids Res.,* 1986, vol. 14 (16), 6745-63

- Matrices for detecting distant relationships. **SCHWARTZ ; DAYHOFF.** Atlas of protein sequences. National Biomedical Research Foundation, 1979, vol. 5, 353-358
- **HUANG ; MILLER.** *Adv Appl Math.,* 1991, vol. 12 (3), 337-57
- **CROOKE et al.** *Cell Metab.,* 2018, vol. 27 (4), 714-739
- **STROHL.** *BioDrugs,* 2015, vol. 29 (4), 215-239
- **RESPAUD et al.** *MAbs.,* 2014, vol. 6 (5), 1347-55
- **GUILLEMINAULT et al.** *J Control Release.,* 2014, vol. 196, 344-54
- **RESPAUD et al.** *Expert Opin Drug Deliv.,* 2015, vol. 12 (6), 1027-39
- **JACKISCH et al.** *Geburtshilfe Frauenheilkd.,* 2014, vol. 74 (4), 343-349
- **SOLAL-CELIGNY.** *Expert Rev Hematol.,* 2015, vol. 8 (2), 147-53
- **MICHEL et al.** *J Neurochem.,* 1997, vol. 69 (4), 1499-507
- **GUERREIRO et al.** *Mol Pharmacol.,* 2008, vol. 74 (4), 980-9
- **TRAVER et al.** *Mol Pharmacol.,* 2006, vol. 70 (1), 30-40
- **TOULORGE et al.** *Faseb J.,* 2011, vol. 25 (8), 2563-73
- **DOUHOU et al.** *J Neurochem.,* 2001, vol. 78 (1), 163-74
- **HARA-YOKOYAMA et al.** *Int Immunopharmacol.,* 2008, vol. 8 (1), 59-70
- **WILLIAMS et al.** *J Neurosci Res.,* 1996, vol. 45 (6), 747-57
- **DEAGLIO et al.** *J Immunol.,* 1996, vol. 156 (2), 727-34
- **DECKERT et al.** *Clin Cancer Res.,* 2014, vol. 20 (17), 4574-83
- **DAUER ; PRZEDBORSKI.** *Neuron.,* 2003, vol. 39 (6), 889-909
- **SUN et al.** *J Immunother.,* 2000, vol. 23 (2), 208-14
- **GROUX et al.** *J Immunol.,* 1999, vol. 162 (3), 1723-9

- **FUJII et al.** *Blood,* 2001, vol. 98 (7), 2143-51
- **BERMAN et al.** *J Immunol.,* 1996, vol. 157 (1), 231-8
- **PHILIBERT et al.** *BMC Biotechnol.,* 2007, vol. 7, 81
- **GALINDO et al.** Handbook of neurotoxicity. Springer-Verlag, 2014, 639-651
- **LASSMANN.** *J Neurol Sci.,* 2008, vol. 274 (1-2), 45-7
- **EICHELE ; KHARBANDA.** *WorldJ Gastroenterol.,* 2017, vol. 23 (33), 6016-29
- **GEYSEN et al.** *Proc Natl Acad Sci USA.,* 1984, vol. 81 (13), 3998-4002
- **TIMMERMAN et al.** *J Mol Recognit.,* 2007, vol. 20 (5), 283-299